(19) Europäisches Patentamt
European Patent Office
Office européen des brevets

(11) EP 2 397 139 B1

# (12) EUROPEAN PATENT SPECIFICATION

(45) Date of publication and mention of the grant of the patent:
**17.09.2014 Bulletin 2014/38**

(21) Application number: **10735774.1**

(22) Date of filing: **25.01.2010**

(51) Int Cl.:
*A61K 31/4709* (2006.01) *A61K 31/352* (2006.01)
*A61K 31/381* (2006.01) *A61K 31/403* (2006.01)
*A61K 31/404* (2006.01) *A61K 31/4045* (2006.01)
*A61K 31/4152* (2006.01) *A61K 31/4155* (2006.01)
*A61K 31/4184* (2006.01) *A61K 31/421* (2006.01)
*A61K 31/422* (2006.01) *A61K 31/423* (2006.01)
*A61K 31/426* (2006.01) *A61K 31/427* (2006.01)
*A61K 31/428* (2006.01) *A61K 31/4439* (2006.01)
*A61P 25/28* (2006.01)

(86) International application number:
**PCT/JP2010/050903**

(87) International publication number:
**WO 2010/087306 (05.08.2010 Gazette 2010/31)**

## (54) ANTI-NEURODEGENERATIVE DISEASE AGENT

MITTEL GEGEN NEURODEGENERATIVE ERKRANKUNGEN

AGENT ANTI-MALADIE NEURODÉGÉNÉRATIVE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.01.2009 JP 2009017406**
**13.02.2009 JP 2009032068**
**06.09.2009 JP 2009205390**

(43) Date of publication of application:
**21.12.2011 Bulletin 2011/51**

(73) Proprietor: **Hayashibara Co., Ltd.**
**Okayama-shi, Okayama (JP)**

(72) Inventors:
- **OHTA Hitomi**
  **Okayama-shi**
  **Okayama 700-0907 (JP)**
- **AKITA Kenji**
  **Okayama-shi**
  **Okayama 700-0907 (JP)**
- **OHTA Tsunetaka**
  **Okayama-shi**
  **Okayama 700-0907 (JP)**
- **KAWATA Toshio**
  **Okayama-shi**
  **Okayama 700-0907 (JP)**
- **FUKUDA Shigeharu**
  **Okayama-shi**
  **Okayama 700-0907 (JP)**

(74) Representative: **Daniels, Jeffrey Nicholas**
**Page White & Farrer**
**Bedford House**
**John Street**
**London WC1N 2BF (GB)**

(56) References cited:
**WO-A1-02/090314** **WO-A1-03/106439**
**WO-A1-2004/050088** **WO-A1-2009/003147**
**WO-A2-02/07781** **WO-A2-2007/041358**
**JP-A- 2000 355 579** **JP-A- 2002 121 416**
**JP-A- 2003 137 784** **JP-A- 2004 250 407**
**JP-T- 2002 518 380** **JP-T- 2003 534 341**
**JP-T- 2005 505 519** **JP-T- 2005 514 368**
**US-A1- 2003 013 656** **US-A1- 2006 018 825**

- **J.BIOLOGICAL CHEMISTRY vol. 280, no. 9, 2005, pages 7614 - 7623, XP003014102**
- **BIOCHEMISTRY vol. 45, no. 19, 2006, pages 6085 - 6094, XP002436919**
- **EUROPEAN J.PHARMACOLOGY vol. 559, no. 2-3, 2007, pages 124 - 131, XP005907818**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- M. Ishihara ET AL: "Kinetic Radical-scavenging Activity of Platonin, a Cyanine Photosensitizing Dye", In vivo, vol. 20, 1 January 2006 (2006-01-01), pages 845-848, XP055050418,
- K. Kogure ET AL: "Inhibitory Effects of Pentamethine Trinuclear Cyanine Dyes on ADP/Fe2+-Induced Lipid Peroxidation in Rat Liver Mitochondria: Changes in the Mode of Action with the Hydrophobic Nature of the Dyes", Biol. Pharm. Bull., vol. 21, 1 January 1998 (1998-01-01), pages 180-183, XP055050420,
- OHTSUKA K ET AL: "Immunological effects of lumin-A, a fluorescent antioxidant drug, on T cell subsets in various immune organs of mice", ACTA MEDICA BIOLOGICA, NIIGATA DAIGAKU IGAKUBU, NIIGATA, JP, vol. 43, no. 4, 1 January 1995 (1995-01-01), pages 205-209, XP008086887, ISSN: 0567-7734
- B. Wolozin ET AL: "Mechanisms of Neurodegenerative Disorders. Part 1: Protein Aggregates", Arch. Neurol., vol. 57, 1 January 2000 (2000-01-01), pages 793-796, XP055050361,
- B. Wolozin ET AL: "Mechanisms of Neurodegenerative Disorders. Part 2: Control of Cell Death", Arch. Neurol., vol. 57, 1 January 2000 (2000-01-01), pages 801-804, XP055050369,

**Description**

Technical Field

The present invention relates to a compound represented by General formula 2 or 3 for use in preventing or treating a neuro-degenerative disease, wherein the prevention or treatment of the neuro-degenerative disease comprises promoting Neurocyte-growth or Neurite-outgrowth:

General formula 2:

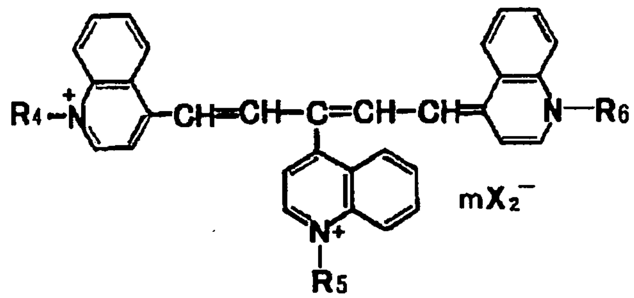

wherein in General formula 2, $R_4$ through $R_6$ each independently represent the same alkyl group having a carbon number of 2 to 8; $X_2^-$ represents a counter anion selected from the group consisting of fluorine ion, chlorine ion, bromine ion, iodine ion, prechloric acid ion, periodic acid ion, hexafluorophosphoric ion, hexafluoroantimonate ion, hexafluorostanate ion, phosphoric acid ion, fluoroboric ion, tetrafluoroborate ion, thiocyanic acid ion, benzene sulfonic acid ion, naphthalenesulfonic acid ion, naphthalenedisulfonic aciod ion, p-toluenesulfonic acid ion, alkyl sulfonic acid, ion, benzenecarboxylic acid ion, alkylcarboxylic acid ion, trihaloalkylcarboxylic acid ion, alkylsulfonate ion, trihaloalkylsulfonate ion, nicotinic acid ion and aspartic acid ion; and m represents a integer of 1 or 2 that forms an electric charge for balancing with the electric charge of a cationic part;

General formula 3:

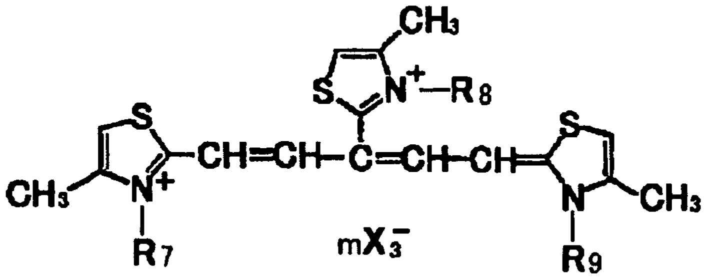

wherein in General formula 3, $R_7$ through $R_9$ each independently represent the same alkyl group having a carbon number of 3 to 12 x3- represents a counter anion selected from the grop consisting of fluorine ion, chlorine ion, bromine ion, iodine ion, prechloric acid ion, periodic acid ion, hexafluorophosphoric ion, hexafluoroantimonate ion, hexafluorostanate ion, phosphoric acid ion, floroboric ion, tetrafluoroborate ion, thiocyanic acid ion, benzene sulfonic acid ion, naphthalenesulfonic acid ion, naphthalenedisulfonic acid ion, p-toluenesulfonic acid ion, alkyl sulfonic acid ion, benezenecarboxylic acid ion, alkylcarboxylic acid ion, trihaloalkylcarboxylic acid ion, alkylsulfonate ion, trihaloalkylsulfonate ion, nicotinic acid ion and aspartic acid ion; and m represents an integer of 1 or 2 that forms an electric charge for balancing with the electric charge of a cationic part.

Background Art

Neurodegenerative diseases are those which are induced by the collapse of nervous circuit neural network based on the systematic degeneration and deciduation of neurocytes, and they have been known as various intractable diseases such as Alzheimer's disease, Parkinson's disease, parkinsonism, vascular dementia, frontotemporal labor degeneration, amyotrophic lateral sclerosis, progressive supranuclear palsy, Huntington disease, and spinocerebellar degeneration.

It can be speculated that numerous molecular groups complicatedly relate to the mechanism of neurodegenerative death as a causative of neurodegenerative diseases and they may cause disorders in their expressions and functions. However, almost no molecular pathogenesis for neurodegenerative diseases has been revealed and any effective method for inhibiting neurodegeneration has not been established. In addition to the treatment for removing causatives of such diseases, more important is to reconstruct neural network. For example, there have been said that, in Alzheimer's disease where cytotoxicity of amyloid β peptide has been recognized as its causative, both the atrophia of neurites (axis cylinder and dendrite) and the reduction of synapse trigger off the deterioration of neurofunction, and in reverse, even after such triggering, neurocytes which are not fully denatured or survived without degeneration can be recovered if they can possibly be activated to extend neurites for recovering synapse. It is said that axis cylinder of damaged peripheral nervous system may be recovered; however, axis cylinder in central neurosystem could not occur without any treatment such as transplantation of peripheral nervous system.

It has been known that proteins included in the group of neurotrophic factors such as a nerve growth factor (may be abbreviated as "NGF", hereinafter) relate to the differentiation and survival of neurocytes and the regulation of synapse, however, due to their defects of scarcely passing through the blood brain barrier, the therapeutic effects of such proteins on neurodegenerative diseases, which are inherent to the degeneration of central nerve through systemic administrations such as subcutaneous and intravascular administrations, are not so expected. Surgical treatment is inevitably required to administer such proteins intracerebrally with an expectation of desired effect, resulting in a physical and spiritual heavy-load on patients.

Clinical symptoms of neurodegenerative diseases are varied from light ones to severe ones, depending on respective diseases; tremor, rigidity, akinesia, hypokinesia, bradykinesia, attitude reflex failure, dysautonomia, pulsion, gait disturbance, depression, dysmnesia, amyotrophia, muscle loss, disorder of shoulder girdle, articulation disorder, dysphagia, respiration disorder, numbness, and paralysis, which are major hurdles in performing daily activities.

Neurodegenerative diseases represented by Alzheimer's disease, Parkinson's disease, etc., are severe diseases which cause degeneration of neurocytes. To improve these diseases and their accompanying symptoms and neurofunctional disorders, therapeutic agents containing some compounds as effective ingredients have been proposed (see, for example, International Patent Publication No. WO97/030703, Japanese Patent Kokai Nos. 228417/1999, 143708/2006, and 321737/2006) ; and a neurite outgrowth accelerator has been also proposed (see, for example, Japanese Patent Kokai No. 234841/2002). However, any effective therapeutic method has not yet been found. Commercialized therapeutics for neurodegenerative diseases may have some problems in terms of side effects, etc., on a relatively-long-term-successive use.

In medical fields, desired is the exploitation of a novel anti-neurodegenerative disease agent which cures pathema and clinical symptoms accompanied by neurodegeneration through the inhibition of neurodegeneration in such a manner of acting on neurocytes in central nerve system, activating the neurocytes, and inhibiting the atrophy of neurite or accelerating the outgrowth of neurites, through the systematic administration of agents, such as subcutaneous and intravascular administrations thereof, with only a lesser physical and psychic load on patients.

Disclosure of Invention

The present invention has an object to provide a novel anti-neurodegenerative disease agent.

To solve the above object, the present inventors diligently studied and screened and found that the compounds represented by General formula 1 have an advantageous action of both activating neurocytes and accelerating neurite outgrowth. They also found that these compounds have an inhibitory action on neurite death induced by a cytotoxic factor, activate neurocytes in central nerve system and inhibit neurodegeneration even when administered systematically, and delay or improve the symptom and the onset of pathema induced by neurodegeneration. The description provides an anti-neurodegenerative disease agent containing any of the following compounds represented by General formula 1:

General formula 1:

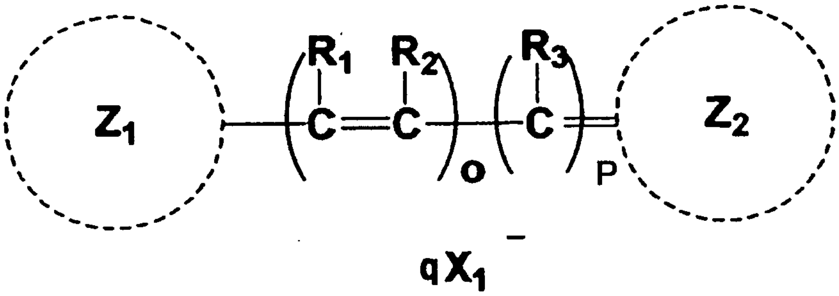

wherein in General formula 1, $R_1$ through $R_3$ independently represent a hydrogen atom or an appropriate substituent; $Z_1$ represents a heterocyclic ring and $Z_2$ represents the same or different heterocyclic or aromatic ring as in $Z_1$, wherein the heterocyclic and aromatic rings optionally have a substituent; o represents an integer of 0, 1 or 2; p represents an integer of 0 or 1, with the proviso that p is 1 when o is 0 or 2, and p is 0 when o is 1; $R_1$ and $R_2$ do not exist when o is 0, while, when p is 0, $R_3$ does not exist and the binding between the carbon atom to which $R_2$ binds and $Z_2$ is a single bond; $X_1^-$ represents an appropriate counter anion and q represents an integer of 1 or 2.

The present invention specifically provides a compound represented by General formula 2 or 3 for use in preventing or treating a neurodegenerative disease, wherein the prevention or treatment of the neuro-degenerative disease comprises promoting Neurocyte-growth or Neurite-outgrowth:

General formula 2:

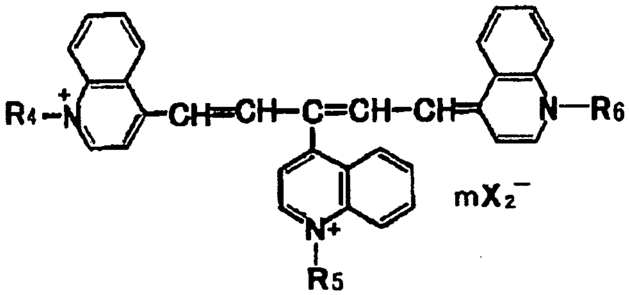

wherein in General formula 2, $R_4$ through $R_6$ each independently represent the same alkyl group having a carbon number of 2 to 8; $X_2^-$ represents a counteranion selected from the group consisting of fluorine ion, chlorine ion, bromine ion, iodine ion, prechloric acid ion, periodic acid ion, hexafluorophosphoric ion, hexafluoroantimonate ion, hexafluorostanate ion, phosphoric acid ion, fluoroboric ion, tetrafluoroborate ion, thiocyanic acid ion, benzene sulfonic acid ion, naphthalenesulfonic acid ion, naphthalenedisulfonic aciod ion, p-toluenesulfonic acid ion, alkyl sulfonic acid, ion, benzenecarboxylic acid ion, alkylcarboxylic acid ion, trihaloalkylcarboxylic acid ion, alkylsulfonate ion, trihaloalkylsulfonate ion, nicotinic acid ion and aspartic acid ion; and m represents a integer of 1 or 2 that forms an electric charge for balancing with the electric charge of a cationic part;

General formula 3:

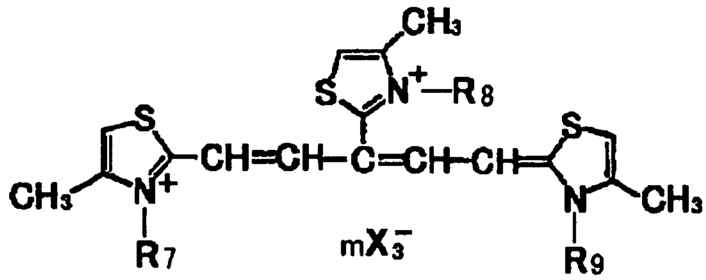

wherein in General formula 3, $R_7$ through $R_9$ each independently represent the same alkyl group having a carbon number of 3 to 12 x3- represents a counter anion selected from the grop consisting of fluorine ion, chlorine ion, bromine ion, iodine ion, prechloric acid ion, periodic acid ion, hexafluorophosphoric ion, hexafluoroantimonate ion, hexafluorostanate ion, phosphoric acid ion, floroboric ion, tetrafluoroborate ion, thiocyanic acid ion, benzene sulfonic acid ion, naphthalenesulfonic acid ion, naphthalenedisulfonic acid ion, p-toluenesulfonic acid ion, alkyl sulfonic acid ion, benezenecarboxylic acid ion, alkylcarboxylic acid ion, trihaloalkylcarboxylic acid ion, alkylsulfonate ion, trihaloalkylsulfonate ion, nicotinic acid ion and aspartic acid ion; and m represents an integer of 1 or 2 that forms an electric charge for balancing with the electric charge of a cationic part.

When administered parenterally, the anti-neurodegenerative disease agent for use according to the present invention accelerates the growth of neurocytes and the outgrowth of neurites, and protects cells from nutrition/oxygen hunger or cytotoxic factors such as amyloid β peptide to inhibit neurodegeneration induced by these cytotoxic factors, resulting in improving tremor, rigidity, akinesia, hypokinesia, bradykinesia, attitude reflex failure, dysautonomia, pulsion, gait disturbance, depression, dysmnesia, amyotrophia, muscle loss, disorder of upper and lower limbs, articulation disorder, dysphagia, respiration disorder, numbness, paralysis, etc. Further, the compounds for use according to the present invention are significantly high in safeness.

Best Mode for Carrying Out the Invention

The term "neurite (s) " as referred to as in the present inventionmeans axis cylinder ordendrite that extends fromneurosome. The term "neurite outgrowth accelerating action" means an action of activating neurosome to elongate axis cylinder and/or dendrite, as well as actions of inhibiting atrophy or reduction of neurite, accelerating synapse formation between neurosomes, and inhibiting synapse reduction.

The term "neurodegeneration" as referred to as in the present invention means functional reduction, death or reduction (defluxion) of neurosome in the central nerve system, and includes atrophy or reduction of neurite, reduction of synapse, functional reduction, death or lowering of glia, and death or degeneration of retinal cells.

The description provides anti-neurodegenerative disease agents containing any of the compounds represented by the following General formula 1:

[Chem. 3]

General formula 1:

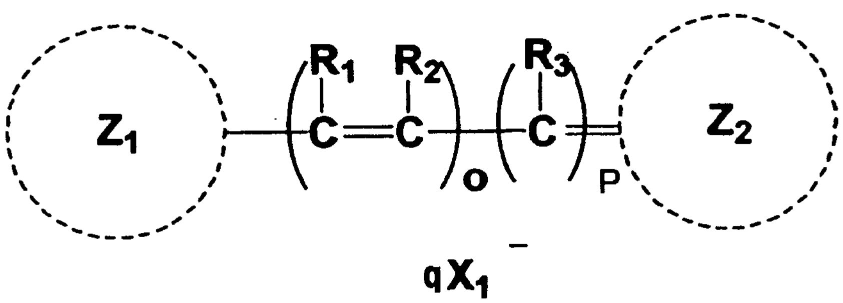

wherein in General formula 1, $R_1$ through $R_3$ independently represent a hydrogen atom or an appropriate substituent;

$Z_1$ represents a heterocyclic ring and $Z_2$ represents the same or different heterocyclic or aromatic ring as in $Z_1$, wherein the heterocyclic and aromatic rings optionally have a substituent; o represents an integer of 0, 1 or 2; p represents an integer of 0 or 1, with the proviso that p is 1 when o is 0 or 2, and p is 0 when o is 1; $R_1$ and $R_2$ do not exist when o is 0, while, when p is 0, $R_3$ does not exist and the binding between the carbon atom to which $R_2$ binds and $Z_2$ is a single bond; $X_1^-$ represents an appropriate counter anion and q represents an integer of 1 or 2.

In General formula 1, $X_1^-$ represents an appropriate counter anion and generally includes, for example, inorganic anions such as fluorine ion, chlorine ion, bromine ion, iodine ion, perchloric acid ion, periodic acid ion, hexafluorophosphoric ion, hexafluoroantimonate ion, hexafluorostanate ion, phosphoric acid ion, fluoroboric ion, and tetrafluoroborate ion; and organic acid ions such as thiocyanic acid ion, benzene sulfonic acid ion, naphthalenesulfonic acid ion, naphthalenedisulfonic acid ion, p-toluenesulfonic acid ion, alkyl sulfonic acid ion, benzenecarboxylic acid ion, alkylcarboxylic acid ion, trihaloalkylcarboxylic acid ion, alkylsulfonate ion, trihaloalkyl sulfonate ion, nicotinic acid ion, and aspartic acid ion.

The present invention specifically provides compounds represented by General formulae 2 and 3, for use in preventing or treating a neuro-degenerative disease, wherein the prevention or treatment of the neuro-degenerative disease comprises promoting neurocyte-growth or neurite-outgrowth;

[Chem. 4]

General formula 2:

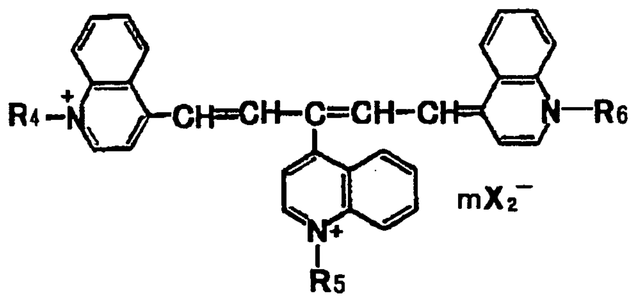

wherein in General formula 2, $R_4$ through $R_6$ each independently represent the same or different alkyl group having a carbon number of 2 to 8; $X_2^-$ represents a counter anion selected from the group consisting of fluorine ion, chlorine ion, bromine ion, iodine ion, prechloric acid ion, periodic acid ion, hexafluorophosphoric ion, hexafluoroantimonate ion, hexafluorostanate ion, phosphoric acid ion, fluoroboric ion, tetrafluoroborate ion, thiocyanic acid ion, benzene sulfonic acid ion, naphthalenesulfonic acid ion, naphthalenedisulfonic acid ion, p-toluenesulfonic acid ion, alkyl sulfonic acid ion, benzenecarboxylic acidion,alkylcarboxylic acid ion, trihaloalkylcarboxylic acid ion, alkylsulfonate ion, trihaloalkylsulfonate ion, nicotinic acid ion and aspartic acid ion; and m represents an integer of 1 or 2 that forms an electric charge for balancing with the electric charge of a cationic part.

[Chem. 5]

General formula 3:

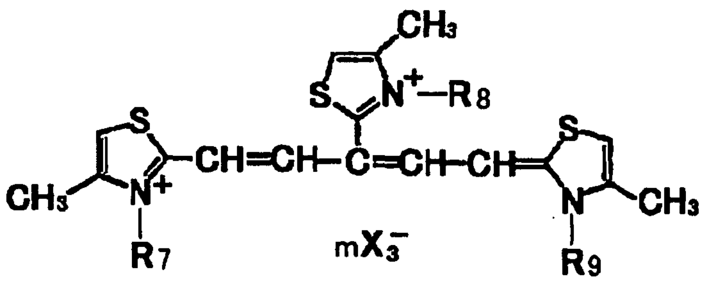

wherein in General formula 3, $R_7$ through $R_9$ each independently represent the same alkyl group having a carbon number of 3 to 12; $X_3^-$ represents a counter anion selected from the group consisting of fluorine ion, chlorine ion, bromine ion, iodine ion, prechloric acid ion, periodic acid ion, hexafluorophosphoric ion, hexafluoroantimonate ion, hexafluorostanate ion, phosphoric acid ion, fluoroboric ion, tetrafluoroborate ion, thiocyanic acid ion, benzene sulfonic acid ion, naphthalenesulfonic acid ion, naphthalenedisulfonic acid ion, p-toluenesulfonic acid ion, alkyl sulfonic acid ion, benzenecarboxylic

acid ion, alkylcarboxylic acid ion, trihaloalkylcarboxylic acid ion, alkylsulfonate ion, trihaloalkylsulfonate ion, nicotinic acid ion and aspartic acid ion; and m represents an integer of 1 or 2 that forms an electric charge for balancing with the electric charge of a cationic part.

The description further provides an anti-neurodegenerative disease agent containing compounds represented by General Formulae 4 and 5.

[Chem. 6]

General formula 4:

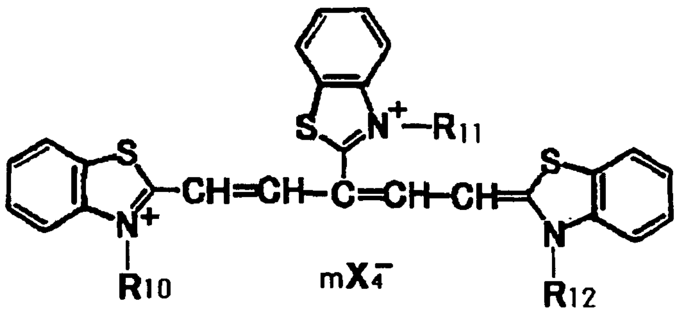

wherein in General formula 4, $R_{10}$ through $R_{12}$ each independently represent the same or different aliphatic hydrocarbon group; $X_9^-$ represents an appropriate counter anion; and m represents an integer of 1 or 2 that forms an electric charge for balancing with the electric charge of a cationic part.

[Chem. 7]

General formula 5:

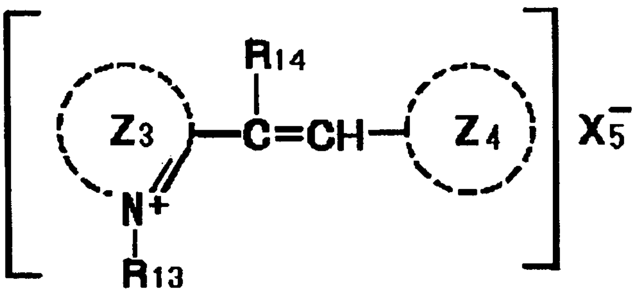

wherein in General formula 5, $Z_3$ represents a heteroaromatic ring which optionally has a substituent; $Z_4$ represents an aromatic or heteroaromatic ring which optionally has a substituent; $R_{13}$ represents an aliphatic hydrocarbon group which optionally has a substituent; $R_{14}$ represents a hydrogen atom or an appropriate substituent; and $X_5^-$ represents an appropriate counter ion.

In preferred examples of the description, the aliphatic hydrocarbons represented by $R_4$ through $R_{13}$ in General formulae 2 to 5 can be selected from those with a carbon atom number of 1 to 12, preferably, those with a carbon atom number of 2 to 10, and more preferably, those with a carbon atom number of 2 to 9. Among which, due to their strong neurodegenerative-inhibitory-actions, most desirable are the compounds represented by General formula 2 for use according to the present invention, wherein the carbon atom number of the aliphatic hydrocarbons, $R_4$ through $R_6$, is 2 to 12; or the compounds represented by General formula 3, wherein the carbon atom number of the aliphatic hydrocarbons, $R_7$ through $R_9$, is 4 to 10. Respective examples of such are methyl, ethyl, propyl, isopropyl, butyl, isobutyl, sec-butyl, tert-butyl, pentyl, isopentyl, tert-pentyl, 1-methylpentyl, 2-methylpentyl, hexyl, isohexyl, 5-methylhexyl, pentyl, octyl, nonyl, decyl, undecyl, and dodecyl groups. In General formulae 2 to 5, appropriate counter anions represented by $X_2^-$ through $X_5^-$ are usually selected from inorganic acid anions such as fluorine ion, chlorine ion, bromine ion, iodine ion, perchloric acid ion, periodic acid ion, hexafluorophosphoric ion, hexafluoroantimonate ion, hexafluorostannate ion, phosphoric acid ion, fluoroboric ion, and tetrafluoroborate ion; and organic acid ions such as thiocyanic acid ion, benzene

sulfonic acid ion, naphthalenesulfonic acid ion, naphthalenedisulfonic acid ion, p-toluenesulfonic acid ion, alkyl sulfonic acid ion, benzenecarboxylic acid ion, alkylcarboxylic acid ion, trihaloalkylcarboxylic acid ion, alkylsulfonate ion, trihaloalkyl sulfonate ion, nicotinic acid ion, and aspartic acid ion.

Concrete examples of the compounds represented by General formula 2 include those which are represented by Chemical formula 1 (may be called "NK-26", hereinafter), Chemical formula 2 (may be called "NK-4", hereinafter), and General formula 2 wherein the carbon atom number of the side chain of alkyl groups of $R_4$ through $R_6$ is 3 (may be called "NK-234", hereinafter). For example, "Kankoso-Hyo" (Table of Photosensitive Dyes), published by Kankoshikiso-Kenkyu-Sho, Okayama, Japan, 1969, and "CHEMICAL ABSTRACT Index Guide (N-Z), pp. 1531G-1536G, 1994, describe the structures of the compounds corresponding to NK-numbers described in the specification.

[Chem. 8]

Chemical formula 1:

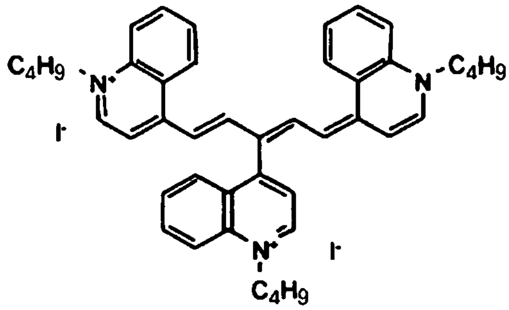

[Chem. 9]

Chemical formula 2:

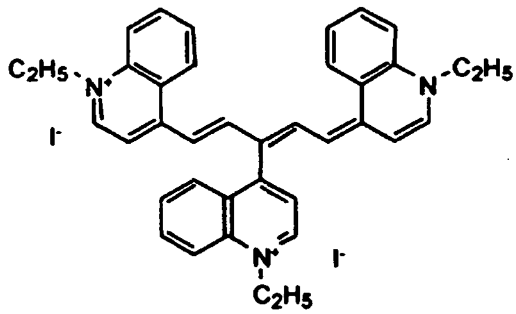

Concrete examples of the compounds represented by General formula 3 include those which are represented by Chemical formula 3 (may be called "NK-150", hereinafter) and Chemical formula 4 (may be called "NK-19", hereinafter). The compound represented by General formula 5 (may be called "NK-53", hereinafter), corresponding to NK-19 wherein the counter anion ($I^-$) is replaced with $Cl^-$ can be advantageously used similarly as NK-19.

[Chem. 10]

Chemical formula 3:

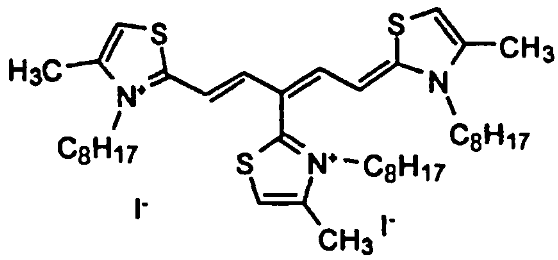

[Chem. 11]

Chemical formula 4:

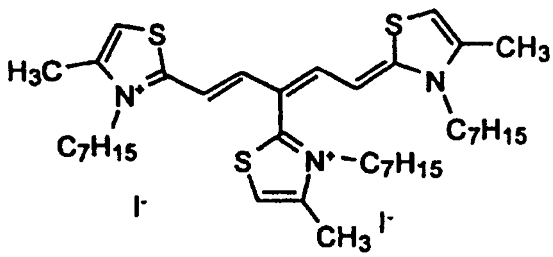

[Chem. 12]

Chemical formula 5:

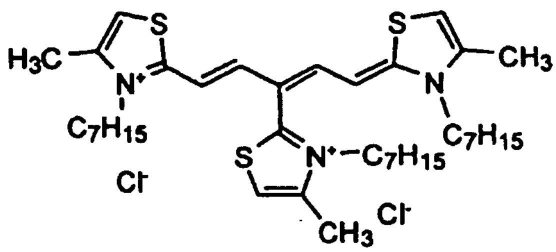

The description further provides concrete examples of the compounds represented by General formula 4 which include the compound represented by chemical formula 6 (may be called "NK-100", hereinafter).

[Chem. 13]

Chemical formula 6:

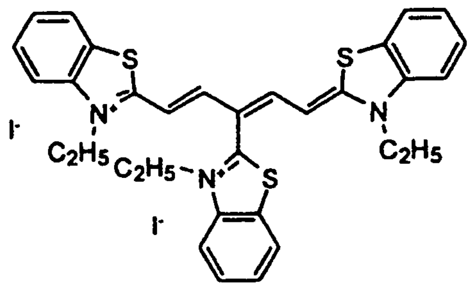

The description further provides examples of the compounds represented by General formula 5 which include any of the compounds represented by chemical formulae 7 to 9 (may be called "NK-528", "NK-557" and "NK-1516", respectively).

[Chem. 14]

Chemical formula 7:

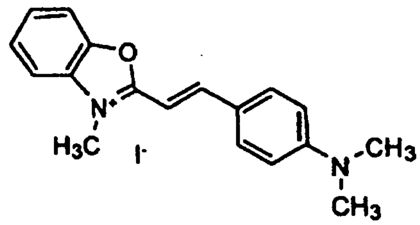

[Chem. 15]

Chemical formula 8:

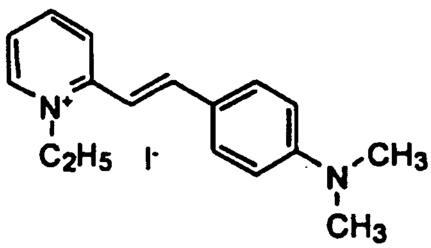

[Chem. 16]

Chemical formula 9:

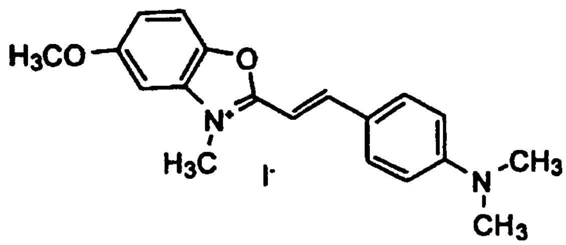

All the compounds represented by General formulae 1 to 9 have a neurocyte activating action and a neurite outgrowth accelerating action, as well as having an action of inhibiting cell death and neurite atrophy through protecting neurocyte from cytotoxic factors such as starvation, radical, amyloid β peptide, etc. Therefore, the above-identified compounds are more preferable as effective ingredients for the anti-neurodegenerative disease agent of the present invention. From a strong effective and functional standpoint, most preferable are NK-26 (a compound represented by Chemical formula 1), NK-4 (a compound represented by Chemical formula 2), NK-23 (a compound represented by General formula 2 wherein the carbon atom number of the side chain is 3), and NK-150 (a compound represented by Chemical formula 3). NK-4 and NK-234 are preferable; however, the former is most preferable because of their strong inhibitory action on acetylcholinesterase activity, intracephalic transportability, and formulation feasibility.

The compounds, represented by General formulae 2 and 3 used as an effective ingredient according to the present invention, should not be restricted to specific origins and preparation methods.

In addition to the compounds represented by General formulae 2 and 3 as an effective ingredient, one or more ingredients which are acceptable for pharmaceutical engineering and in the fields of food products, cosmetics, pharmaceuticals, and quasi-drugs are optionally incorporated into the anti-neurodegenerative disease agent of the present invention before being formulated in a preparation form.

Examples of the ingredients acceptable for pharmaceutical engineering include additives, excipients, disintegrants, glosses, stabilizers, surfactants, antiseptics (antimicrobials), flavors, viscosity-imparting agents, antioxidants, chelates, vitamins, amino acids, aqueous media, saccharides, water-soluble high molecules, pH controllers, blisters, additives forpharmaceuticals/quasi-drugs/cosmetics/foodproducts, effective ingredients for pharmaceuticals/quasi-drugs, etc., one or more of which can be appropriately incorporated in combination to prepare the agent of the present invention depending on its desired preparation form.

The anti-neurodegenerative disease agent for use according to the present invention can be advantageously used in combination with neurite outgrowth accelerators other than the compounds represented by General formula 1 and medicaments for neurodegenerative diseases and their inducing pathema and neurofunctional disorders. Concrete examples of such are medications for cerebrovascular diseases (e.g., cerebral embolism), cerebral infarction (e.g., cerebral thrombosis, cerebral embolism, etc.), transient ischemic attack, reperfusion injury, encephalorrhagia (e.g., hypertensive intracerebral hemorrhage, subarachnoid hemorrhagia, etc.), brain tumor (e.g., astrocytoma, pyencephalus, etc.), hypovolemic shock, traumatic shock, head injury and/or traumatic cerebrospinal (e.g., cerebral confusion/penetration/shearing/compression/ laceration, birth trauma, whiplash shaken infant syndrome, etc.); those for neurodegenerative diseases (e.g., Parkinson's disease, parkinsonism, striatonigral degeneration, Huntington's disease, choreaathetosis, progressive supranuclear palsy, diffuse Lewy body disease, corticobasal degeneration, Alzheimer's disease, senile dementia, Pick disease, front temporal lobar degeneration, familial dementia, spinocerebellar degeneration (e.g., olivopontocerebellar atrophy, late cerebellar atrophy, familial spinocerebellar ataxia (e.g., Machado-Joseph disease, etc.), dentatorubral-pallidoluysian atrophy, familial spastic paraplegia, and Friedreich disease, etc.); those for motor neuropathy (e.g., amyotrophic lateral sclerosis, familial amyotrophic lateral sclerosis, etc.); those for demyelinating disease (e.g., multiple sclerosis, lateral sclerosis, acute disseminated encephalomyelitis, acute inflammation of the cerebellum, transverse myelitis, Guillain-Barré syndrome, etc.); those for encephalomyelopathy accompanied by infectious diseases (e.g., meningitis, influenza-associated encephalopathy, Creutzfeldt-Jakob disease, agnosia induced by AIDS encephalopathy, etc.); those for neurofunctional disorders induced by toxins (e.g., arsenic, cadmium, organomercury, sarin, soman, tabun, VX gas, etc.) and radiations; those for mental diseases (e.g., neurosis, psychosomatic disease, anxiety, schizophrenia, manic depression, etc.); those for epilepsy, Meige's syndrome, dystonia, and Down's syndrome; those for sleep disturbance (e.g., hypersomnia, narcolepsy, sleep apnea syndrome, etc.); those for diabetic, diabeticcomplication, and hyperlipidemia; dopamine agonist (dopamine receptor stimulant), dopamine release stimulant (dopamine secretion or release accelerator), dopamine-uptake inhibitor, dopamine agonist, centrally acting anticholinergic, aromatic L-amino acid decarboxylase inhibitor (DCI), monoamine oxidase type B (MAO-B) inhibitor, catechol-O-methyltransferase (COMT) inhibitor, norepinephrine (noradrenaline) replenisher, acetylcholinesterase inhibitor, NMDA (N-methyl-D-aspartate) receptor antagonist, AMPA (2-amino-3-(methyl-3-hydroxyisooxazole-4-yl) propanic acid/kainate receptor antagonist, GABA receptor modulator, adenosine A2A receptor blocker, nicotinic receptor modulator, neuronal nitric oxide synthase (n-NOS) inhibitor, inhibitor for production/secretion/accumulation/aggregation/ deposition of β-amyloid protein (e.g., β-secretase inhibitor, γ-secretase inhibitor, amyloid β protein aggregation inhibitor, amyloid β-protein degrading enzyme, amyloid β-vaccine, etc.), apoptosis inhibitor, neuronal differentiation/regeneration promoting drug, neurotrophic factor (e.g., neurotrophin, TGF-β super family, neurokine family, growth factor such as NGF, etc.), other brain activating factor (e.g., cerebrometabolic stimulant, cerebral circulation improving agent, etc.), Rho-kinase inhibitor, diuretic (e.g., benzothiazide diuretic, loop diuretic, potassium-sparing diuretic, etc.), β-receptor blocker, calcium channel blocker (calcium antagonist), angiotensin converting enzyme (ACE) inhibitor, angiotensin II receptor inhibitor, sodium channel blocker, potassium channel blocker, antiplatelet drug, anticoagulant, thrombolytic drug, thromboxane $A_2$ synthase inhibitor, matrix metalloproteinase (MMP) inhibitor, cyclooxygenase (COX)-2 inhibitor, non steroidal anti-inflammatory agent, steroid, antioxidant, vitamins, disease-modifying antirheumatic drug, cytokine, anti-cytokine drug (e.g., TNF inhibitor, etc.), MAP kinase inhibitor, sex hormone or derivatives thereof (e.g., progesterone, estradiol, estradiol benzoate, etc.), parathyroid hormone (e.g., PTH, etc.), calcium antagonist, etc. These medicaments can be administered respectively or in a mixture formulation with the effective ingredient (s) of the present invention.

Preferred medicaments for neurodegenerative diseases and their inducing pathema and neurofunctional disorders, which are used in combination with the anti-neurodegenerative disease agent of the present invention, include, for example, those for cerebrovascular diseases (e.g., cerebral embolism), cerebral infarction (e.g., cerebral thrombosis, cerebral embolism, etc.), transient ischemic attack, encephalorrhagia (e.g., hypertensive intracerebral hemorrhage, subarachnoid hemorrhagia, etc.), brain tumor, neurofunctional disorders accompanied by traumatic brain/spinal cord injury (e.g., contusio cerebri, etc.), neurodegenerative diseases (e.g., Parkinson's disease, parkinsonism, Huntington's disease, Alzheimer's disease, senile dementia, spinocerebellar degeneration, etc.), motor neuropathy (e.g., amyotrophic lateral sclerosis, etc.), demyelinating disease (e.g., multiple sclerosis, etc.), cerebrospinal diseases accompanied by

infectious diseases (e.g., meningitis, influenza-associated encephalopathy, Creutzfeldt-Jakob disease, agnosia induced by AIDS encephalopathy, etc.), neuropathy, psychosomatic disease, anxiety, schizophrenia, psychosis, etc.), epilepsy, dystonia, diabetic, diabetic complication and/or hyperlipidemia; and other dopamine receptor agonist, dopamine release stimulant, dopamine uptake inhibitor, dopamine agonist, centrally acting anticholinergic, aromatic L-amino acid decarboxylase inhibitor (DCI), monoamine oxidase type B (MAO-B) inhibitor, catechol-O-methyltransferase (COMT) inhibitor, norepinephrine (noradrenaline) replenisher, acetylcholinesterase inhibitor, NMDA (N-methyl-D-aspartate) receptor antagonist, AMPA (2-amino-3-(methyl-3-hydroxyisooxazole-4-yl) propanic acid/kainic acid receptor antagonist, $GABA_A$ receptor modulator (e.g., $GABA_A$ receptor agonist), $GABA_B$ receptor modulator, adenosine A2A receptor blocker, β-secretase inhibitor, amyloid β protein aggregation inhibitor, apoptosis inhibitor, neuronal differentiation/regeneration promoting drug, neurotrophic factor (e.g., neurotrophin, TGF-β super family, neurokine family, growth factor, etc.), other brain activating factor (e.g., cerebrometabolic stimulant, cerebral circulation improving agent, etc.), Rho-kinase inhibitor, diuretic (e.g., benzothiazide diuretic, loop diuretic, potassium-sparing diuretic, etc.), β-receptor blocker, calcium channel blocker (calcium antagonist), angiotensin converting enzyme (ACE) inhibitor, angiotensin II receptor inhibitor, sodium channel blocker, potassium channel blocker, antiplatelet drug, anticoagulant, thrombolytic drug, cyclooxygenase (COX)-2 inhibitor, non steroidal anti-inflammatory agent, steroid, antioxidant, and vitamins. More preferably, medicaments for cerebrovascular diseases (e.g., cerebral apoplexy, cerebral infarction, etc.), neurofunctional disorders accompanied by cerebral contusion, neurodegenerative diseases (e.g., Parkinson's disease, parkinsonism, Huntington's disease, Alzheimer's disease, senile dementia, etc.), amyotrophic lateral sclerosis, multiple sclerosis, psychosomatic disease (e.g., neurosis, psychosomatic disease, anxiety, schizophrenia, psychosis, etc.), epilepsy and/or dystonia, diabetic, diabetic complication and/or hyperlipidemia, dopamine receptor agonist, dopamine release stimulant, dopamine uptake inhibitor, dopamine agonist, centrally acting anticholinergic, aromatic L-amino acid decarboxylase inhibitor (DCI), monoamine oxidase type B (MAO-B) inhibitor, catechol-O-methyltransferase (COMT) inhibitor, norepinephrine (noradrenaline) replenisher, acetylcholinesterase inhibitor, NMDA (N-methyl-D-aspartate) receptor antagonist, β-secretase inhibitor, amyloid β protein aggregation inhibitor, apoptosis inhibitor, neuronal differentiation/regeneration promoting drug, neurotrophic factor (e.g., NGF such as neurotrophin, TGF-β super family, neurokine family, growth factor, etc.), and other brain activating factor (e,g., cerebrometabolic stimulant, cerebral circulation improving agent, etc.), β-receptor blocker, calcium channel blocker (calcium antagonist), angiotensin converting enzyme (ACE) inhibitor, angiotensin II receptor inhibitor, antiplatelet drug, anticoagulant, thrombolytic drug, and more particularly, medicaments for cerebrovascular diseases (e.g. cerebral apoplexy, cerebral infarction, etc.), neurofunctional disorders accompanied by cerebral contusion(e.g.,contusio cerebri,etc.),neurodegenerative diseases (e.g., Parkinson's disease, parkinsonism, Huntington's disease, Alzheimer's disease, etc.), amyotrophic lateral sclerosis, multiple sclerosis, and epilepsy and/or diabetic complication; as well as dopamine receptor agonist, dopamine release stimulant, dopamine uptake inhibitor, dopamine agonist, centrally acting anticholinergic, aromatic L-amino acid decarboxylase inhibitor (DCI), monoamine oxidase type B (MAO-B) inhibitor, catechol-O-methyltransferase (COMT) inhibitor, norepinephrine (noradrenaline) replenisher, acetylcholinesterase inhibitor, neurotrophic factor, and other brain activating factor. Among the above-identified medicaments, NGF is most preferable because it effectively enhances the physiological functions including the neurocyte activating action, neurite outgrowth accelerating action, and neurocyte protecting action inherent to the compounds represented by General formula 1 used in the present invention.

The anti-neurodegenerative disease agent for use according to the present invention can be provided in the form of a medicament for parenteral injection. The compounds represented by General formulae 2 and 3 as the effective ingredients of the agent can be incorporated thereunto in any step from their material stage and completion of their final products, considering the composition or the use of an objective parenterally administrable medicament such as a medicament for injection. Examples of the methods thereof can be appropriately selected from one or more of mixing kneading, dissolving, melting, dispersing, suspending, emulsifying, reverse micellisation, penetrating, crystallizing, spreading, applying, spraying, coating, injecting, soaking, solidifying, supporting, etc.

In the case of parenteral medicaments such as injection medicaments, they can be in the form of a dried or liquid medicament because, depending on diseases or symptoms to be applied, they are usually dissolved in pyrogen-free aqueous systems before being injected intradermally, subcutaneously, intramuscularly, trans-endocelially (intrapleurally, intraperitoneally, etc.), intravascularly, or intracerebrally including intraspinally. In the case of dried medicaments, they can be used after being dissolved in aqueous solvents such as refined water for injection, physiological saline, and glucose solution. In the case of liquid medicaments, they can be administered intact or used after being added to parenteral fluid, perfusion solution, peritoneal dialysate, etc. When the effective ingredients have any troublesome in solubility in solvents or aqueous media or they are prepared into gradually degradable medicaments, they can be arbitrarily increased their solubility with amphophilic solvents, oily bases, or emulsifiers. The effective ingredients can be administered after being encapsulated into liposome, etc.

The term "aqueous solvents" as referred to as in the present invention mean aqueous solvents in general that are incorporated with one or more hydrophilic organic solvents selected from the group consisting of: alcohols such as ethanol, propanol, and isopropanol; ketones such as acetone; ethers such as diethyl ether; and sulfur-atom-containing

dimethyl sulfoxide (may be abbreviated as "DMSO", hereinafter). Preferred examples of the aqueous solvents in the liquid agents according to the present invention are either those which consist of refined water for injection, physiological saline, Ringer solution, etc., or mixture solutions of refined water with physiologically acceptable hydrophilic organic solvents such as ethanol, propanol, isopropanol, diethyl ether, DMSO, etc. Compounds to be formulated can be adjusted to their highest possible pHs to meet their highest dissolution levels by adding pH controllers such as lactic acid, hydrochloric acid, sodium hydroxide, potassium hydroxide, sodium bicarbonate, phosphate buffer solution, etc.

In the case of such liquid agents, depending on the compounds represented by General formulae 2 and 3 to be used, they may become unstable due to dissolved oxygen. In such a case, for example, the dissolved oxygen level of such compound solutions should be lowered. These liquid compositions can be generally prepared through the steps of dissolving the compounds in aqueous solvents, and allowing the aqueous solvents to decrease their oxygen levels at normal temperature and pressure under atmospheric conditions. To dissolve these compounds in aqueous solvents, for example, prescribed amounts of the compounds are added to appropriate amounts of aqueous solvents, optionally, allowed to dissolve by heating/stirring, and if necessary further admixed with aqueous solvents to give a prescribed concentration level.

To adjust the dissolved oxygen levels of the aqueous solvents to their levels at normal temperature and pressure under atmospheric conditions, for example, preferably employed methods are as follows: The compounds represented by General formula 1 should be prepared into their liquid forms under a reduced pressure and then stored, allowed to replace the dissolved oxygen with other gasses, or allowed to contact with deoxidizers. To replace the dissolving oxygen in liquid compositions with relatively inactive gasses such as nitrogen gas, they can be bubbled with rare gas such as neon, argon, krypton, or xenon gas. To lower the oxygen levels in the liquid compositions with deoxidizers, the liquid compositions can be admixed with adequate amounts of L-ascorbic acid, L-ascorbic acid stearate, sodium sulfite, sodium hydrogensulfite, alpha-thioglycerine, sodium edetate, cysteine chloride, citric acid, soybean lecithin, sodium thioglycolate, sodium thiomalate, sodium pyrosulfite, or butylhydroxyanisole, etc. The above-identified methods can be applied to either solutions of the compounds or aqueous solvents before dissolving the compounds. The oxygen concentration dissolved in such aqueous solvents can be usually adjusted to 0.4 ppm or lower, preferably, 0.1 ppm or lower. The following ingredients can be advantageously added in an appropriate amount to stabilize the compounds used as the effective ingredients of the present invention before they are formulated into desired medicaments: Ingredients with an activity of eliminating singlet oxygen such as tocopherol, carotin, histidine, tryptophane, tyrosine, methionine, cysteine, dopa, rutin, rutin derivatives, thiotaurine, hypotaurine, bilirubin, cholesterol, quinoline, quercetin, catechin, anthocyanin, and thiamine; viscosity-imparting agents such as alkyl cellulose and carboxy vinyl polymers; and surfactants such as triton X, polysorbate, deoxycholic acid or its salts, and cholic acid or its salts.

The solutions with the compounds represented by General formulae 2 and 3 thus obtained can be stored in a sealed condition of being enclosed in an oxygen-shielded appropriate container, depending on use. Any materials can be used for such a container without particular restriction as long as they can theoretically retain the above liquid compositions and substantially shield oxygen; preferably, light-shielding containers such as brown bottles and vials are desirable. Varying depending on use, the liquid compositions can be sterilized, for example, by filtration sterilization before distributing them into containers such as glass amples and vials or with high-pressure sterilization or filtration sterilization after distributing the compositions into containers and then sealing them.

The anti-regenerative disease agent for use according to the present invention can be used in the form of a cataplasm or aspiration nebula for lung absorption, etc., or in the form of a gradually degradable agent embedded subcutaneously, as well as in the form of an injection. The use of the present invention may arbitrarily comprise treating animals, excluding humans, including pet animals suffering fromneurodegenerative diseases. The compounds defined herein may further be used as a prophylactic or therapeutic agent for neurofunctional disorders and diseases accompanied by neurodegenerative diseases.

The anti-neurodegenerative disease agent for use according to the present invention thus prepared is a safe medicament without causing serious side effects even when used successively for a relatively long period of time.

Depending on pathological conditions or symptoms, the anti-neurodegenerative disease agent for use according to the present invention can be administered with a prescribed amount of dose every day or at an interval of one or more days and at a dose frequency of once or several times a day. The daily dose should not specifically be restricted as long as it attains a desired function and effect; usually, in the case of intravenous administration including instillation, as well as subcutaneous and intraperitoneal administrations, the compounds represented by General formula 1 can be administered in total at a dose of 0.01 mg/kg body weight/day or more, preferably, 0.1 to 20 mg/kg body weight/day, and most preferably, 0.5 to 5 mg/kg body weight/day. Even when administered at a dose of 50 mg/kg body weight/day or more, a desired enhancement effect could not promisingly be observed to meet its expected dose effect. When administered with a hope of functioning as a radical scavenger, the anti-neurodegenerative disease agent as defined herein can be arbitrarily administered at a higher dose than the above-identified doses. The dose period of the agent can be controlled depending on the objective diseases, pathological conditions, and symptoms; and it can be administered over a period until symptoms are improved or diminished when applied to acute symptoms. Desirably, such dose should be continued

in chronic patients with dementia, even when the symptoms are improved or diminished.

The anti-neurodegenerative disease agent defined herein is useful for treating neurodegenerative diseases, particularly; diseases induced by central nervous denaturation because it can protect the brain and neurocytes from necrosis to inhibit their denaturation, activate neurocytes, promote neurite outgrowth or inhibit neurite atrophy, and prolong neurocyte survival or inhibits neurocyte denaturation. The term "neurodegenerative diseases" as referred to as in the present invention includes all diseases which accompany denaturation of neurocytes (central nerve such as cranial nerve and spinal nerve), and/or peripheral nerves (autonomic nerve such as sympathetic nerve and parasympathetic nerve, motorius system, and sensory nerve system), and it should not be restricted by its causatives. Concrete examples of such are those which are recognized as neurodegenerative diseases in general, for example, Parkinson's disease, parkinsonism, striatonigral degeneration, Huntington's disease, chorea-athetosis, progressive supranuclear palsy, diffuse Lewy body disease, corticobasal degeneration, Alzheimer's disease, senile dementia, Pick disease, frontotemporal lobe dementia, familial dementia, spinocerebellar degeneration (e.g., olivopontocerebellar atrophy, late cerebellar atrophy, familial spinocerebellar ataxia (e.g., Machado-Joseph disease, etc.), dentatorubral-pallidoluysian atrophy, familial spastic paraplegia, and Friedreich disease, etc.); those for motor neuropathy (e.g., amyotrophic lateral sclerosis, familial amyotrophic lateral sclerosis, etc.); those for demyelinating disease (e.g., multiple sclerosis, lateral sclerosis, acute disseminated encephalomyelitis, acute inflammation of the cerebellum, transverse myelitis, Guillain-Barré syndrome, etc.), metabolic brain disease, congenital and hereditary diseases (nervous system lysosomal storage disease); other neurofunctional disorders accompanied by cerebrovascular disease (e.g., cerebrovascular diseases (e.g., stroke, cerebral infarction (e.g., cerebral thrombosis, cerebral embolism, etc.), cerebrovascular disease occurred during treatment such as cryotherapy, hypoxic ischemic brain damage, transient ischemic attack, re-circulating injury, cerebral hemorrhage (e.g., hypertensive intracerebral hemorrhage, subarachnoid cerebral hemorrhage, etc.), brain tumor (e.g., astrocytoma, brain abscess, etc.)/hypovolemic shock/traumatic shock/head injury and/or traumatic cerebrospinal (e.g., cerebral confusion/penetration/shearing/compression/ laceration, birth trauma, whiplash shaken infant syndrome, etc.); and others such as cerebrospinal diseases accompanied by infectious diseases (e.g., meningitis, influenza-associated encephalopathy, Creutzfeldt-Jakob disease, agnosia induced by AIDS encephalopathy, etc.), neurofunctional disorders induced by those for neurofunctional disorders induced by toxins (e.g., arsenic, cadmium, organomercury, sarin, soman, tabun, VX gas, etc.) and radiations; those for mental diseases (e.g., neurosis, psychosomatic disease, anxiety, schizophrenia, manic depression, etc.); those for epilepsy, Meige's syndrome, dystonia, and Down's syndrome; and sleep disturbance (e.g., hypersomnia, narcolepsy, sleep apnea syndrome, etc.).

Preferred examples of neurodegenerative diseases, to which the agent of anti-neurodegenerative diseases of the present invention is applicable, include Parkinson's disease, parkinsonism, Huntington's disease, Alzheimer's disease, senile dementia, spinocerebral... degeneration, demyelinating disease (e.g., multiple sclerosis, etc.), cerebrovascular disease (e.g., stroke, cerebral infarction (e.g., cerebral thrombosis, cerebral embolism, etc.), transient cerebral ischemic attack, neurologic dysfunction accompanied by cerebral hemorrhage (e.g., hypertensive intracerebral hemorrhage, subarachnoid hemorrhage, etc.), brain tumor/traumatic shock/head injury and/or traumatic brain/spinal cord injury (e.g., contusio cerebri, etc.), encephalomyelopathy accompanied by infectious diseases (e.g., meningitis, influenza encephalitis/encephalopathy, Creutzfeldt-Jakob diseases, agnosia induced by AIDS encephalopathy, etc.), diseases originated from neurodegeneration in the central nerve system such as epilepsy; more preferably, for example, Parkinson's disease, parkinsonism, Alzheimer's disease, amyotrophic lateral sclerosis associated neurologic dysfunction, encephalomyelopathy accompanied by infectious diseases (e.g., meningitis, influenza encephalopathy, Creutzfeldt-Jakob diseases, agnosia induced by AIDS encephalopathy, etc.), epilepsy; and most preferably, neurologic dysfunction accompanied by Parkinson's disease and Alzheimer's disease.

The anti-neurodegenerative disease agent as defined herein is also useful for treating neurologic dysfunction by activating neurocyte, elongating neurite, promoting the formation of synapse, etc. The neurologic dysfunction to be treated includes any of disorders in neurologic function and includes, for example, cognitive dysfunction, confusion, bilateral paralysis, the other side single paralysis, alternate hemiplegia, facial paralysis, sensory disturbance, transient blindness (e.g., amaurosis fugax, etc.), homonymous hemianopia, vertigo, nystagmus, double vision, aphasia, tinnitus, coma, etc. Most preferably applicable ones are, for example, neurologic dysfunctions accompanied by the above-identified neurodegenerative diseases. The above-mentioned neurologic dysfunctions are mainly observed, although the neurologic dysfunctions accompanied by the above-mentioned neurodegenerative diseases are varied depending on vascular occlusion sites or the symptoms are varied depending on the disturbed levels. The judgment of neurologic dysfunction in cerebral infarction can be diagnosed on various conventional diagnostic tests used in the art to detect neurologic dysfunctions. Concrete examples of such usable in the above are the following conventional methods; cognitive function score used in evaluating memory and cognitive dysfunction induced by Alzheimer's disease, etc. (Alzheimer's disease assessment scale-cognitive part; ADAS-cog); score of clinical symptom improvement (Alzheimer's disease cooperative study-clinical global impression of change; ADCS-CGIC); mini-mental state examination (MMSE); Hasegawa evaluation; glasgow outcome scale (GOS); glasgow coma scale (GCS); rankin scale (RS); modified rankin scale (mRS); disability rating scale (DRS) ; NIH stroke scale (NIHSS), etc. These diagnostic tests to detect neurologic

dysfunctions can be conducted in combination with a testing method for detecting physical brain abnormity, for example, computed tomographic scan, measurement of intracranial pressure, etc.

Accordingly, the anti-neurodegenerative disease agent as defined herein can be advantageously used as a neurite protective agent, neurite activator, neurite-outgrowth-promoting agent, neurite atrophy inhibitor, inhibitor for Purkinje's cell degeneration/dropout, and therapeutic agent for pathema accompanied by neurodegenerative diseases or for neurologic dysfunction. The term "treatment of neurodegenerative diseases and neurologic dysfunction" as referred to as in the present invention means progression inhibition to prevent progression of pathema accompanied by neurodegeneration, and prevention of the onset of diseases, as well as so called therapy to allow pathema or functional dysfunction inherent to neurodegeneration to direct to curing.

Since the anti-neurodegenerative disease agent as defined herein reduces free radicals including hydroxyl radicals, it can be advantageously used as a prophylactic or therapeutic agent for diseases and pathema inherent to free radicals or lipoperoxide generated by recirculation after ischemia in vessels and organs other than brain, as well as tissues; inflammatory diseases including immunopathy, allergy, and tumors; infectious diseases; drugs; radiations; and physical stimulations. More concretely, in addition to a prophylactic/therapeutic agent for the above-identified neurodegenerative diseases, the agent can be advantageously used as, for example, a brain protective agent, oxidative dysfunction inhibitory agent for the brain (neurocyte, hemangioendothelial cells), ischemic brain injury, inhibitor for cerebral infarction development, cerebral edema, delayed neuronal death inhibitor, agent for normalizing brain failure, oxidative stress inhibitor, antiseptic ulcer agent, hyperglycemia inhibitor, and prophylactic and therapeutic agents for eye diseases such as cataract and corneal injury; organ transplant preservative; necrosis inhibitor for transplanted tissues (including the skin)/organs; prophylactic and therapeutic agents for organ disorders such as nephropathy induced by acute renal failure/chemicals, skin tissue damage, lung injury, liver fibrosis, functional disorder of skin tissue induced by chemical substances, endotoxin and burn/scald, liver damage induced by ischemia, spinal cord injury, vessel wall defect such as artery, muscle problem such as myocardia, tubulointerstitial disorder; inhibitors for sensory cells, sensory nerves, and sensory organs such as visual disorder, optic nerve disorder, retinal disorder, acoustic cell disorder, acoustic nerve disorder, etc.; prophylactic and therapeutic agents for medicinal poisoning induced by agricultural chemicals and organic media; Na-Ca exchange system inhibitor, prophylactic and therapeutic agents for pain and pruritus; protein kinase stimulant; prophylactic and therapeutic agents for mitochondrial encephalomyopathy; prophylactic and therapeutic agents for arterial occlusion/stenosis; blood-brain-barrier rhexis inhibitor; therapeutic agent for drug dependent diseases; apoptosis inhibitor; formation inhibitor for lipid peroxide; and radical scavengers such as hydroxyl-, peroxyl- and NO-radicals; as well as prophylactic and therapeutic agents for functional disorders and clinical symptoms accompanied by the above-identified disorders. The anti-neurodegenerative disease agent of the present invention can be also arbitrarily used as an amyloid β peptide aggregation inhibitor, inhibitor for amyloid β peptide disorder, acetylcholinesterase inhibitor, serine/threonine kinase (Akt) activator, phosphatidylinositol (3,4,5) 3-phosphokinase (PI3K)-serine/threonine kinase (Akt) cascade activator, accelerator for increasing cyclic AMP concentration, SAPK/JNK phosphorylation inhibitor, etc.

The following Experiments explain the present invention in more detail.

Experiment 1: Effect of pentamethine cyanine dye on injury of neurocyte

Neurocytes are known to be quite susceptive to injury induced by nutrient starvation and active oxygen. Such characteristic feature of neurocytes has been recognized as a causative for inducing neurocyte death as found in neurodegenerative diseases including Alzheimer's disease and Huntington's disease. The following experiment was conducted to examine the influence of pentamethine cyanine dye on injury of neurocyte induced by cytotoxic factors.

<Test specimen>

In this experiment, the compound represented by Chemical formula 2 ("NK-4" produced by Hayashibara Biochemical Laboratories, Inc., Okayama, Japan) was used as a test specimen. Since NK-4 is scarcely dissolvable in water, it was dissolved in "D8418", a product number of DMSO, commercialized by SIGMA Corporation, Tokyo, Japan, to give a concentration of 5 mg/ml, followed by filtering the resulting solution with a membrane filter ("MILLEX-LG SLLG025SS", a product name of Millipore Corporation MA, USA, prepared with a DMSO-resistant-membrane), and diluting the filtrate with Dulbecco's Medium (abbreviated as "D-MEM medium", commercialized by Nissui Pharmaceutical Co., Ltd., Tokyo, Japan) for use in this experiment. Prior to conduct the following tests, it was confirmed that there exists no influence of DMSO concentrations in the solutions, prepared by diluting the test specimen dissolved in DMSO with D-MEM medium to the concentrations for use in such tests, on any testing system. The cyanine dyes used in the following tests were all synthesized by Hayashibara Biochemical Laboratories Inc., Okayama, Japan.

<Assay for measuring cytotoxicity inhibitory action>

<Effect of NK-4 on cytotoxicity induced by nutrition starvation>

Nerve growth factor (NGF) high sensitive strain of PC-12 cells derived from rat pheochromocytoma (called "PC12-HS cells", hereinafter), obtained from Human Science Research Resources Bank, Osaka, Japan, which has been used as a suitable model for research on human neurodegenerative diseases, was used. The cells were cultured with a serum-free medium as a nutrition-starvation-condition. PC12-HS cells were subjected to tests after stock cultured cells thereof were thawed and cultured with a D-MEM medium supplemented with 10% by volume of fetal bovine serum (FBS). The cells used for the tests were in usual manner detached from the surface of cell culture vessels with 0.25% by weight of trypsin solution, diluted with a D-MEM medium supplemented with 10% by volume of FBS, and inoculated to "MICROTEST PALTE", a product name of collagen-coated 96-well plate with round bottoms for cell culture, commercialized by Becton, Dickinson and Company (Falcon), NJ, USA, to give a cell density of 5 x $10^3$ cells/100 μl/well. After 24 hours, the culture supernatant was removed from each well of the plate, and the resulting cells in each well were received any one of NK-4, which had been diluted with a FBS-free D-MEM medium and adjusted to give a concentration of two-fold higher than respective final concentrations as shown in Table 1, in a volume of 100 μl/well, and cultured for three days. On three days of the culture, the culture supernatant was removed from each well, and the cells were received with ALAMAR BLUE, commercialized by Trek Diagnostic Systems Inc., Ohio, USA, which had been diluted with a D-MEM medium supplemented with 10% by volume of FBS to give a concentration of 10% w/v, cultured for six hours, and measured for fluorescent intensity at a wavelength of 544 to 590 nm by "SpectraMax Gemini HY", a product name of a fluorescent plate reader commercialized by Japan Molecular Devices Corporation, Tokyo, Japan. As a control, cells were cultured with a NK-4 free D-MEM medium and, similarly as above, cultured after the addition of an ALAMAR BLUE solution commercialized by Trek Diagnostic Systems Inc., Ohio, USA, and measured for fluorescent intensity for each well. Relative values for cells in each well were determined when the fluorescent intensity for control was regarded as 100%, and the data are also shown in Table 1 as survival percentages (%) of cells in each well. In this experiment and the following experiments, PC12-HS cells were cultured in an incubator controlled at 37°C under 5% by volume $CO_2$ conditions.

<Effect of NK-4 on cytotoxicity by hydrogen peroxide>

PC12-HS cells, which hadbeen cultured similarly as above, were diluted with D-MEM MEDIUM supplemented with 10% by volume of FBS, inoculated to 96-well plates coated with collagen at a cell density of 2 x $10^4$ cells/100 μl/well, and cultured for 24 hours. Thereafter, to the cells were simultaneously added an 800 μM aqueous hydrogen peroxide solution (commercialized by Wako Pure Chemicals, Tokyo, Japan) in a volume of 50 μl/well (at a final concentration of 200 μM); and NK-4, which had been diluted with D-MEM medium supplemented with 10% by volume of FBS to give a 4-fold higher concentration to respective final concentrations as shown in Table 1, in a volume of 50 μl/well (at a final concentration of 5 to 50, 000 ng/ml of NK-4). The resulting cells were cultured for two hours in an incubator and fixed by the addition of 20 μl/well of 25% by volume of glutaraldehyde, commercialized by Wako Pure Chemicals, Tokyo, Japan (at a final concentration of 20% by volume). The absorbance for each well was measured in a usual manner by dye-uptake method after the addition of 100 μl/well of 0.05% by weight of methylene blue, commercialized by Wako Pure Chemicals, Tokyo, Japan. Asacontrol, cellswerecultured similarly as above except for not adding hydrogen peroxide and NK-4, and measured for their absorbances after the addition of methylene blue. Viability (%) of cells in each well was determined and shown in Table 1 by calculating a relative value when the viability (absorbance) of the control cells was regarded as 100 (%).

<Effect of NK-4 on cytotoxicity by amyloid β peptide>

A plurality of peptide fragments (called "AMYLOID β FRAGMENT" commercialized by AnaSpec, Inc., CA, USA, eachpeptidehaving the amino acid sequence of SEQ ID NO:1), which have an amino acid sequence positioning from 25$^{th}$ to 35$^{th}$ from the N-terminus of amyloid β peptide with human origin and have been recognized as a main factor of neurocyte death in Alzheimer's disease, were diluted with phosphate buffered saline (PBS) to give a concentration of 2 mM, and used after being aged at 37°C for six hours to coagulate them to increase their cytotoxicity before use. PC12-HS cells, which had been cultured similarly as above, were diluted with D-MEM medium supplemented with 10% by volume of FBS, and inoculated to 96-well plates coated with collagen at a cell density of 5 x $10^3$ cells/100 μl/well, followed by removing the supernatant in each well after 24-hours incubation, and further cultured for three days after the addition of an amyloid β fragment solution, which had been diluted with D-MEM medium supplemented with 10% by volume of FBS, in a volume of 50 μl/well (at a final concentration of 50 μM of amyloid μ fragment) and an NK-4 solution in a volume of 50 μl/well (at a final concentration of 40 to 5,000 ng/ml). On three days of the culture, the supernatant in each well

was removed, and the resulting cells were received with a solution of ALAMAR BLUE (commercialized by Trek Diagnostic Systems Inc., Ohio, USA), which had been diluted with D-MEMmedium supplemented with 10% by volume of FBS to give a concentration of 10% by weight in a volume of 200 μl/well, cultured for six hours, and measured for fluorescent intensity at a wavelength of 544 to 590 nm on a fluorescent plate reader. As a control, cells were cultured with only D-MEM medium supplemented with 10% by volume of FBS (being free of both amyloid β fragment and NK-4) and similarly measured for fluorescent intensity after the addition of ALAMAR BLUE solution. Viability (%) of cells in each well was determined and shown in Table 1 by calculating a relative value when the fluorescent intensity of the control was regarded as 100%. While, the supernatant of cell culture, which had been cultured under the same conditions as above, was removed, and the resulting cells were fixed by the addition of 1% by volume of glutaraldehyde, which had been prepared by diluting with PBS, in a volume of 100 μl/well for 30 min. Thereafter, the cells were stained with 1 mMHoechst 33258 dye commercialized by SIGMA Corporation, Tokyo, Japan, for five min, observed at a magnitude corresponding to a visual filed containing about 100 cells under a phase contrast microscope and a fluorescent microscope, and counted. The occupied percentage (%) of cells with apoptosis against the total cells in one visual field was calculated and the data was also shown in Table 1. The judgment of cells with apoptosis was made with an index of fragmentation of cell nuclei and chromatin aggregation within the nuclei.

Table 1

| NK-4 | Survibal percentage of cells when admixed with cytotoxic factor (%) | | | Percentage of appoptosis-induced cells (%) |
|---|---|---|---|---|
| Concentration (ng/ml) | Nutrition starvation | Hydrogen peroxide | Amyloid β fragment | Amyloid β fragment |
| 0 | 45 | 52 | 39 | 72 |
| 5 | - | 51 | - | - |
| 40 | - | - | 89** | - |
| 50 | 47 | 50 | - | - |
| 200 | - | - | 129** | 13** |
| 500 | 78** | 50 | - | - |
| 1000 | - | - | 97** | - |
| 5000 | 64** | 70** | 97** | - |
| 50000 | - | 90** | - | - |
| 0<br>(Free of cytotoxic factor: Control) | 100 | 100 | 100 | 5 |
| The symbols "**" means that there exists a significant difference (P<0.01) compared to survibal percentage with an NK-4 concentration of 0 ng/ml in the presence of cytotoxic factor.<br>The symbol "-" means "not done". | | | | |

As evident from Table 1, it was revealed that NK-4 has a protecting action on neurocyte, though the compound has different effective concentrations on the following respective cytotoxic factors; nutrition starvation, hydrogen peroxide injury, and injury through amyloid β fragment against PC12-HS cells. Comparing the concentrations of NK-4 effective for exerting protection action against the three cytotoxic factors shown in Table 1, it was revealed that the protection action against injury through amyloid β fragment exerts at a minimum concentration of 40 ng/ml, while 500 ng/ml for nutrition starvation and 5,000 ng/ml for hydrogen peroxide injury are respectively required. Considering the induction rate for injury, hydrogen peroxide injury is the fastest and the injury through amyloid β fragment is the latest, indicating that the cell protection action by NK-4 against the cytotoxic factors is as follows; the higher the induction rate, the higher concentration is needed. Although concrete data is not shown, it was judged as follows: Since any NK-4 solutions with concentrations of 50,000 ng/ml or lower have no ability of eliminating hydrogen peroxide injury, and NK-4 has an ability of eliminating free radicals such as peroxyl radical and hydroxyl radical, however, the cell protection action against cytotoxic injury induced by hydrogen peroxide in this experiment is not a direct action of its removing hydrogen peroxide but its action on cells to inhibit apoptosis. Referring to the occupied percentages of cells with apoptosis, calculated based

on the Hoechst dye image, the cells with the addition of amyloid β fragment (NK-4 concentration of 0 ng/ml) marked an occupied percentage of 72% and were promoted their apoptosis, and the cells with 200 ng/ml of NK-4 marked an occupied percentage of 13% close to that (5%) of the control. These data revealed that NK-4 inhibits the apoptosis induced by amyloid β fragment. Although concrete data is not shown, cell coagulation and apoptosis were found by the addition of amyloid β fragment even under a phase contrast microscopic observation, while cell coagulation and apoptosis are inhibited by the addition of NK-4. It was also revealed that the addition of NK-4 inhibits chromatin aggregation and nucleus fragmentation, which are induced by apoptosis observed by the addition of amyloid β fragment, even with the Hoechst dye image. These data show that NK-4 has a neurotrophic factor activity due to its neurocyte protecting action against nutrient starvation damage, meaning that the compound can be used as a neurocyte protective agent or neurotrophic factor. Since NK-4 protects cells from cytotoxic factors and inhibits apoptosis and has a neurodegenerative inhibitory activity, it can be used as an effective therapeutic agent for human neurodegenerative diseases represented by Alzheimer's disease induced by cytotoxic factors including amyloid β peptide. NK-4 can be also used as an apoptosis inhibitory agent.

Although concrete data is omitted, considering a report that shows the action of nerve growth factor (NGF) against PC12-HS cells is inhibited by K252a as a protein kinase inhibitor highly specific to TrkA (see Kase H. et al., "Biochemical and Biophysical Research Communications", Vol. 144, pp. 35-40, 1987), the present inventors examined whether NK-4 acts on PC12-HS cells via the same pathway as in NGF and found that K252a did not inhibit the actions of NK-4 on PC12-HS cell growthpromotion and the later described neurite outgrowth. It was suggested that these actions of NK-4 are due to the activation of PI3K-Akt cascade because the action of NK-4 is inhibited by LY294002 which inhibits phosphatidylinositol (3,4,5) 3-phosphokinase (PI3K), suppresses the formation of phosphatidylinositol (3,4,5) 3-phosphoric acid, and finally inhibits the activation of serine/threonine kinase (Akt) that mainly acts on cell survival and growth (see Vlahos C., et al., "Journal of Biological Chemistry", Vol. 269, No. 5241-5248, 1994), and there is found phosphorylated Akt positioning at the downstream of PI3K.

Further, NK-4 has an action of inducing the increment of intracellular cyclic adenosine monophosphate (AMP).

As for the action on the induction of phosphorylation of SAPK (Stress Activated Protein Kinase)/JNK (c-Jun N-terminal Kinase) (see Renae L., et al., "Journal of Biological Chemistry", Vol. 274, p. 35499, 1999; Wanli W. et al., "Journal of Biological Chemistry", Vol. 277, pp. 17649-17656, 2002), NK-4 inhibits the phosphorylation of SAPK/JNK by hydrogen-peroxide-induced-cell-injury and thus it was judged that signaling pathway via SAPK/JNK also relates to the neurite outgrowth action of NK-4.

Experiment 2: Effect of NK-4 administration on behavior and brain tissue of hamster with cerebellar ataxia

Since NK-4 was confirmed to have a neurodegenerative inhibitory action in Experiment 1, hamsters with cerebellar ataxia (cerebellar ataxia is called "cerebellar A" and hamsters with cerebellar ataxia are called "hamsters with cerebellar A", hereinafter) as a suitable model for human neurodegenerative diseases (e.g., spinal cerebellar degeneration) were subjected to examine the influence of NK-4 administration on their behaviors and brain tissues. Twenty-five hamsters with natural mutation (Nna1 inhibition), which are known to lose Purkinje's cells after three-weeks of age and then induce a spontaneous onset of kinetic motor ataxia after seven-weeks of age (see Akita K. et al., "J. Neurogenetics", Vol. 21, pp. 19-29, 2007), had been fed at Hayashibara Biochemical Laboratories., Inc., Okayama, Japan, were randomly allocated to the test groups 1 to 5, five heads in each group, as shown in Table 2. Prior to the onset of cerebellar A (at three-weeks of age), PBS was started to administer to the hamsters in the test group 1 at a dose of 10 ml/kg/day. Prior to the onset of cerebellar A (at three-weeks of age), NK-4 was started to be administered to the hamsters in the test groups 2 to 4 at a daily dose of 20 μg/kg, 100 μg/kg or 500 μg/kg. "IGF-1, human, a product name of insulin-like growth factor, commercialized by Assaypro LLC, MO, USA. These administered ingredients were intraperitoneally administered daily to hamsters once a day up to 10-weeks of age. The degree of symptom of cerebellar A and the improvement effect on the symptom by NK-4 were evaluated with an index of improvement in motor coordination of hamsters by conducting once a week the later described rotarod test and the slant tolerance test. At the age of 10-weeks, the hamsters were subjected to counting the falling down frequency and extracted their brains, which were then histologically evaluated. In parallel, the concentration of glutamic acid in the bloods and cerebrospinal fluids (CSF) of the hamsters was assayed. As a test group 6, five normal hamsters, with the same age as the hamsters with cerebellar A used in the test groups 1 to 5, were provided and tested similarly as the hamsters with cerebellar A in such a manner of intraperitoneally administering PBS to them at a dose of 10 ml/kg/day once a day up to 10-weeks of age.

<Rotarod test>

As an index for coordinated movement, it was used a duration time of hamster's movement to staying on a rotarod by locomotion in synchronization with the turn of the rotarod; placing a hamster on an apparatus with a rotarod (having a diameter of 60 mm, prepared by Hayashibara Biochemical Laboratories, Inc., Okayama, Japan), turning at a

constant rate of six rpm, and measuring the time until the hamster falls down from the rotarod (see Fernandez et al., "Proc. Natl. Acad. Sci. USA", Vol. 95, pp. 1253-1258, 1998). The test was repeated six times for each hamster, where five-times of tests starting from the initiation of the test were made as apreliminary examination, and the time until each hamster fell down from the rotarod (called "falling time", hereinafter) was measured, followed by averaging the falling times for each group consisting of five heads. The results are in Table 2. The falling time was measured until 180 seconds. Based on the results, the relationship between the age and the falling time for each hamster with cerebellar A (test group 1) administered with PBS was graphed. When administered with NK-4 (test groups 2 to 4) or IGF-1 (test group 5), the hamsters with cerebellar A at the age of five-weeks or more (two-weeks or more after the administration of NK-4 or IGF-1) were determined from the above graph whether their falling times correspond to that of what age of the hamsters in the test group 1, and the number of days where cerebellar A of the hamsters in the test groups 2 to 5 was calculated {=(any one of the test groups 2 to 5) x 7 (days) - (the age of hamsters in the test group 1 that exhibits the same falling time as the hamsters in the test groups 2 to 5) x 7 (days)}. The results are in Table 3. As shown in Table 3, since there was found no normal hamster at any tested weeks of age, who fell down from the rotarod within 180 seconds, hamsters who fell down from the rotarod within 180 seconds were judged to have developed cerebellar A and lowered in coordinated movement.

<Slant tolerance test>

Hamsters were placed on a slope-angle-variable-board while keeping their heads upward, followed by determining the angle for allowing the hamsters to keep staying thereupon for five seconds for use as an endurable-slant-slope-angle test (see Rivlin et al., "J. Neurosurg. ", Vol. 47, pp. 577-581, 1997) . The initial slope angle was set to 25 degrees and increased by 5 degrees step by step. When a tested hamster in a static condition fell down within five seconds, the slope angle was decreased by one degree step by step, followed by judging the angle that allows the hamster to keep static staying for five seconds, measuring the tolerable slant-slope-angle, and averaging the data for five heads in each group. The results are in Table 4.

<Hamster's falling down frequency>

Hamsters, 10-weeks of age, were respectively housed in a breeding cage. Their falling down frequencies within one minute were counted under a macroscopic observation and the data from five heads in each group were averaged. The results are in Table 5. For reference, the hamsters with cerebellar A used in this experiment are known to increase their falling down frequencies after nine weeks of age.

<Histological evaluation>

Hamsters, 10-weeks of age, after completion of test on coordinated movement such as a confirmation of falling down frequency were intraperitoneally received 50 mg/kg pentobarbital, bled from their postcavas to let them die, and extracted their cerebra and cerebella, followed by fixing them with a 10% by volume of formalin solution. The resulting cerebra and cerebella were photographed with a digital camera apart from a prescribed high position and measured diameters in sagittal and horizontal directions for each photograph. The volumes of cerebra and cerebella were respectively determined by calculating with the formula, (length of sagittal direction)$^2$ x (length of horizontal direction) x 0.5; and the data from five heads in each group were averaged. The results are in Table 5. The hamsters with cerebella A used in this experiment are known to reduce cell density of Purkinje's cells and granular cells. Cerebellar slices, which had been cut out in sagittal direction, were stained with hematoxylin-eosin stain, and microscopically observed to count the total number of Purkinje's cells within a Purkinje's cell layer (flocculi I to X) and the number of granular cells per unit area, and to confirm subjects in which demyelination was observed within their cerebellar white matters. The results are in Table 5 inparallel. Since no difference was observed in the cerebral volumes of the subjects in each test group, only calculated data for cerebella's volumes are shown in Table 5.

<Concentration of glutamic acid in blood and cerebrospinal fluids (CSF)>

The content of glutamic acid, which is used for synthesizing gamma-amino butyric acid (GABA) as a main excitatory neurotransmitter and inhibitory neurotransmitter that regulate higher-order functions such as memory/learning in mammalian central nervous system, was assayed. In harvesting the brains from the above hamsters, they were blooded from their postcavas, followed by collecting CSF for use in the later described assay for glutamic acid concentration. "Amplex™ Red Glutamic Acid/Glutamate Oxidase Assay Kit", a product name of glutamic acid assay kit, commercialized by Invitrogen Corporation, CA, USA, was used for assaying glutamic acid in the blood and CSF. The results are in Table 5 in parallel. Since no difference was observed in glutamic acid content between the test groups, Table 5 only shows the data for CSF.

Table 2

| Test group | Administered ingredient | Dose | Hamster's condition | For each hamster with different age, time (sec) endured until falling from rotarod apparatus | | | | | | |
|---|---|---|---|---|---|---|---|---|---|---|
| | | | | 4-weeks | 5-Weeks | 6-Weeks | 7-weeks | 8-Weeks | 9-Weeks | 10-Weeks |
| Test group 1 | PBS | 10 ml/kg body weight | Cerebellar ataxia | 108±10 | 54±5 | 19±2 | 19±2 | 6±2 | 4±2 | 0±0 |
| Test group 2 | NK-4 | 20 μg/kg body weight | Cerebellar ataxia | 152±4 | 53±5 | 89±9** | 74±10* | 36±2** | 48±3** | 40±4** |
| Test group 3 | | 100 μg/kg body weight | Cerebellar ataxia | 136±5 | 145±8** | 139±10** | 122±7** | 79±12* | 66±6** | 66±6** |
| Test group 4 | | 500 μg/kg body weight | Cerebellar ataxia | 156±5* | 95±8* | 119±4** | 145±7** | 85±8** | 60±7** | 52±5** |
| Test group 5 | IGF-1 | 25 μg/kg body weight | Cerebellar ataxia | 126±11 | 63±13 | 43±11 | 21±5 | 9±2 | 6±1 | 6±1* |
| Test group 6 | PBS | 10 ml/kg body weight | Normal | 180 or more | 180 or more | 180 or more | 180 or more | 180 or more | 180 or more | 180 or more |
| *, ** : There exists a significant difference compared with Test group 1 (* : P<0.05, ** : P<0.01). Data measured : Average ± SEM | | | | | | | | | | |

Table 3

| Test group | Administered ingredient | Dose | Hamster's condition | Delayed days until the onset of cerebellar ataxia in hamster | | | | | |
|---|---|---|---|---|---|---|---|---|---|
| | | | | 5-Weeks | 6-Weeks | 7-Weeks | 8-Weeks | 9-Weeks | 10-Weeks |
| Test group 1 | PBS | 10 ml/kg body weight | Cerebellar ataxia | 0 | 0 | 0 | 0 | 0 | 0 |
| Test group 2 | NK-4 | 20 µg/kg body weight | Cerebellar ataxia | 0 | 11 | 17 | 17 | 27 | 32 |
| Test group 3 | | 1.00 µg/kg body weight | Cerebellar ataxia | 7 | 14 | 21 | 21 | 29 | 36 |
| Test group 4 | | 500 µg/kg body weight | Cerebellar ataxia | 6 | 14 | 21 | 21 | 28 | 35 |
| Test group 5 | IGF-1 | 25 µg/kg body weight | Cerebellar ataxia | 1 | 8 | 1 | 1 | 0 | 0 |

Table 4

| Test group | Administered ingredient | Dose | Hamster's condition | For each hamster with different age, endurable-slant-slope-angle (degree) | | | | | | |
|---|---|---|---|---|---|---|---|---|---|---|
| | | | | 4-Weeks | 5-Weeks | 6-Weeks | 7-Weeks | 8-Weeks | 9-Weeks | 10-Weeks |
| Test group 1 | PBS | 10 ml/kg body weight | Cerebellar ataxia | 40.6±0.3 | 41.0±0.5 | 42.0±0.4 | 42.2±0.3 | 38.0±0.5 | 36.9±2 | 35.8±1.0 |
| Test group 2 | NK-4 | 20 μg/kg body weight | Cerebellar ataxia | 42.6±0.5 | 47.2±0.4** | 44.6±0.5 | 47.0±0.3** | 42.8±0.5** | 44.4±0.5** | 45.8±0.6** |
| Test group 3 | | 100 μg/kg body weight | Cerebellar ataxia | 41.6±0.4 | 45.6±0.2** | 44.4±0.3* | 46.2±0.4** | 47.6±0.4** | 46.8±0.3* | 51.6±0.6** |
| Test group 4 | | 500 μg/kg body weight | Cerebellar ataxia | 43.8±0.4* | 46.4±0.2** | 44.0±0.6 | 44.6±0.5* | 48.0±0.5** | 47.5±0.4** | 51.8±0.4** |
| Test group 5 | IGF-1 | 25 μg/kg body weight | Cerebellar ataxia | 44.4±0.2** | 41.8±0.6 | 40.2±0.7 | 41.6±0.8 | 35.6±0.2* | 35.2±0.6 | 33.0±0.9** |
| Test group 6 | PBS | 10 ml/kg body weight | Normal | 49.8±0.5 | 48.5±0.2 | 48.0±0.5 | 51.7±0.7 | 46.7±0.3 | 47.3±0.5 | 50.8±0.5 |

*, ** : There exits a significant difference compared with Test group 1 (* : P<0.05, ** * P<0.01).
Data measured : Average ± SEM

Table 5

| Test group | Administered ingredient | Dose | Hamster's condition | Falling down frequency (frequency/ min) | Cerebellar volume ($mm^3$) | Number of Purkinje cells (cells) | Number of granular cells (cells/20000 $\mu m^2$) | Number of subject (head) with apparent demyelination in cerebellar white matter | Concentration of glutamic acid in cerebrospinal fluid ($\mu$M) |
|---|---|---|---|---|---|---|---|---|---|
| Test group 1 | PBS | 10 ml/kg body weight | Cerebellar ataxia | 12.8±0.5 | 64.6±9.4 | 67±20 | 380±4 | 5 | 6.4±1.1 |
| Test group 2 | NK-4 | 20 μg/kg body weight | Cerebellar ataxia | 4.0±1.0** | 76.0±8.2* | 187±37** | 408±8 | 4 | 7.2±0.8 |
| Test group 3 | | 100 μg/kg body weight | Cerebellar ataxia | 1.6±0.9** | 77.0±2.8* | 252±29** | 419±6* | 1 | 8.7±2.6 |
| Test group 4 | | 500 μg/kg body weight | Cerebellar ataxia | 1.2±0.8** | 80.5±10.8* | 231±41** | 436±7** | 0 | 9.5±2.9* |
| Test group 5 | IGF-1 | 25 μg/kg body weight | Cerebellar ataxia | 11.4±0.4 | 77.6±6.1* | 71±16 | 371±11 | 3 | 7.9±2.0 |
| Test group 6 | PBS | 10 ml/kg body weight | Normal | 0 | 94.7±11.4* | 698±97 | 480±6 | 0 | 7.7±3.0 |

The symbols "*" and "**" mean "significant value ($P<0.05$) against Test group 1 " and "significant value ($P<0.01$) against Test group 1", respectively.
Data measured : Average ± SEM

As evident from Table 2, the falling time of normal mice with 4-weeks of age (test group 6) was 180 seconds or more, while those with cerebellar A, 4-weeks of age, of the test group 1 (administered with PBS) was 108±10 seconds, indicating that there was a significant shortening of falling time compared to that of the normal hamsters and the falling time was more shortened with hamsters' age. At 10-weeks of age, those with cerebellar A could not stay on the rotarod and instantly fell down (0 second) . On the contrary, the hamsters administered with NK-4 showed inhibition of shortening of falling time in a dose dependent manner from one week after initiating the administration (4-weeks of age). For the test group 3 (100 μg/kg body weight) and test group 4 (500 μg/kg body weight), inhibitory effects of significant shortening of falling time were observed compared to the test group 1. Such inhibitory effects were continued until 10-weeks of age (seven weeks after administration). Also the test group 2 (20 μg/kg body weight) showed inhibitory effect of shortening the falling time shorter than that of the test group 1, on and after 2-weeks after initiating the administration of NK-4 (5-weeks of age), however, the inhibitory effect was low compared to the test groups 3 and 4. When administered with IGF-1 known to have therapeutic effect on motorius degenerative diseases, the mice (test group 5) showed almost no inhibitory effect on shortening of falling time. Similarly, as evident from Table 3 which shows delayed days for shortening the falling time, even at 10-weeks of age (NK-4 administration period of 49 days), the test groups 2 to 4 showed delays for shortening the falling time of 32, 36 and 35 days, respectively. Throughout the experiment, the test groups 3 and 4 showed the highest inhibitory effect on shortening the falling time and exhibited delaying effect on the onset of cerebellar A. Test group 5 administered with IGF-1, however, showed almost no delaying effect on the onset thereof, and showed no difference of falling time between the test groups 1 and 5 at 10-weeks of age. The data indicates that NK-4 can be used as a therapeutic agent for human neurodegenerative diseases and their pathema and symptom.

As evident from the results in Table 4, the endurable-slant-slope-angle for the test group 6 (normal hamsters) was within 46 to 52 degrees throughout the experiment, however, the test group 1 (administered with PBS) using hamsters with cerebellar A gave a significantly low degree of 40.6±0.3. The degree more lowered with age, particularly, it was further lowered on and after eight-weeks of age and finally lowered to 35.8±1.0 degrees at 10-weeks of age. Since the test group 5 (administered with IGF-1) gave a degree of 44.4±0.2 at four-weeks of age, there was found a significant reduction inhibitory effect on endurable-slant-slope- angle compared to the degree of 40.6±0.3 as in the test group 1, however, the test group 5 gave a lowered endurable- slant-slope-angle with age similarly as in the test group 1, and gave no significant improvement on and after five-weeks of age. On the contrary, the test groups 2 to 4 (administered with NK-4) gave no reduction of endurable-slant-slope-angle throughout the experiment at any administration dose used in the experiment and showed a relatively high inhibitory effect on reduction of endurable-slope-slant-angle. The endurable-slant-slope-angles of the test groups 2 to 4 (administered with NK-4) at 10-weeks of age were respectively 45.8±0.6, 51.6±0.6, and 51.8±0.4 degrees that were significantly high compared to those of the test group 1 (administered with PBS) and test group 5 (administered with IGF-1).

As evident from the results in Table 5, no falling was found in normal hamsters with 10-weeks of age (test group 6), while the test group 1 (administered with PBS) fell down at a frequency of 12.8±0.5 times/min. While, the test group 5 (administered with IGF-1) tended to fall down at a frequency of 11.4±0.4/min slightly lower than the test group 1, however, it gave no significant reduction effect on falling frequency. On the contrary, all the test groups 2 to 4 (administered with NK-4) gave significantly reduced falling down frequencies of 4.0±1.0, 1.6±0.9, and 1.2±0.8 times/min, respectively. A significant inhibitory effect on cerebellar atrophy in the cerebellar volumes of 64.6±9.4 $mm^3$ in the hamsters, 10-weeks of age, in the test group 1 (administered with PBS), was found compared with 94.7±11.4 $mm^3$ for the normal hamsters with the same age (test group 6). The test group 5 (administered with IGF-1) gave 77.6±6.1 $mm^3$, a significant inhibitory effect on cerebellar atrophy compared to the test group 1. The test groups administered with NK-4 exhibited inhibitory effect on cerebellar atrophy in a dose dependent manner, and in the case of being administered with 20, 100 and 500 μg/kg, they gave cerebellar volumes of 76.0±8.2, 77.0±2.8 and 80.5±10.8 $mm^3$ (they all have a significant value of $p<0.05$), respectively. In all the test groups, no significant dif ferent was f ound between their cerebral volumes (data not shown). These data revealed that NK-4 improves coordinated movement in hamsters with cerebellar A and inhibits cerebellar atrophy, and the effects are superior to those of IGF-1.

As evident from the results in Table 5, the normal hamsters with 10-weeks of age (test group 6) had a granular cell density of 480±6 cells/20,000 $\mu m^2$ in granular cell layer of cerebellar cortex, while the granular cell density of the hamsters with cerebellar A (test group 1) was significantly lowered to 380±4 cells/20, 000 $\mu m^2$. The granular cell density of the hamsters in the test group 5 (administered with IGF-1) was 371±11 cells/20, 000 $\mu m^2$ being not different from that in the test group 1**.** The test groups 2 to 4 (administered with NK-4) gave granular cell densities of 408±8, 419±6 and 436±7 cells/20,000 $\mu m^2$, respectively, revealing that NK-4 has a reduction inhibitory effect on granular cell density in a dose dependent manner. Not showing any concrete data, microscopic observation of cerebellar parenchymal tissue confirmed that a remarkable atrophy and denaturation in granular cells were found in the test groups 1 and 5, but such alterations were inhibited in the test groups 2 to 4. In the case of hamsters with cerebellar A administered with PBS (test group 1), demyelination of cerebellar white matter was found in all the subjects (five out of five), but only found four out of five, one out of five, and zero out of five in respective the test groups 2 to 4 (administered with NK-4), where inhibition of atrophy of Purkinje's cell-bearing dendrite was also observed. These data show that NK-4 inhibits demyelination of

cerebellar white matter, meaning that the compound is useful as an inhibitory agent for denaturation and demyelination of Purkinje's cells and it can be used as an inhibitor or outgrowth accelerator of cell process of neurocyte including Purkinje's cells.

As evident from the results in Table 5, the concentration of glutamic acid in CSF (spinal fluid) recovered, depending on the dose of NK-4 (test groups 2 to 4); and in the test group 4 (administered with 500 μg/kg of NK-4), the concentration was significantly increased compared to the test group 1 (administered with PBS).

As described above, the hamsters with cerebellar A, administered with NK-4, were improved in their symptoms in all test items on rotarod test, slant tolerance test, and falling down frequency. The effects were dependent on the NK-4 dose and they were particularly high at a dose of 100 and 500 μg/kg body weight/day. The results indicate that NK-4 acted on crania neurocytes and inhibited cerebellar A. Since the administration of NK-4 lowered the level of glutamic acid concentration of CSF in hamsters with cerebellar A, the result shows that NK-4 inhibits the function reduction of neurocyte accompanied by neurodegeneration though the activation of neurocyte and inhibits the reduction of motion and learning abilities. These results indicate that NK-4 is useful as a systematically-administered therapeutic agent for human neurodegenerative diseases and their accompanying pathema and clinical symptoms. It was judged that NK-4 is relatively highly safe even when administered for a relatively long period of time because no significant difference was found between the groups administered with PBS, NK-4 and IGF-1 at any weeks of age when the hamsters with cerebellar A were successively weighed up to completion of the test (10-weeks of age) once every week and the data in each test group were averaged.

Experiment 3: Action of dye compounds other than NK-4

In Experiments 1 and 2, since NK-4 was revealed to have protection action on cytotoxic factor, neurodegenerative inhibitory action, reduction inhibitory action on Purkinje's cells capable of inducing cerebellar A, neurocyte reduction inhibitory action, etc., it was examined whether dye compounds other than NK-4 (may be simply called "Compounds", hereinafter) have a similar action. In addition to the compounds represented by Chemical formulae 2 and 4 to 9 in Table 6, 232 types of compounds (239 specimens in total) represented by the following Chemical formulae 10 to 241 were examined for activity of cell proliferation and outgrowth promoting action on nerve process against PC12-HS cells based on cell proliferation accelerating activity (EvaluationmethodA) and nerve process outgrowth action (Evaluation method B). The data were in Table 6. In the case that an effect lesser than each standard criterion was merely obtained in the following test, it was represented by a blank column in Table 6. Throughout the specification, the compounds represented by Chemical formulae 2 and 4 to 241 may be declared with the symbols (NK series numbers) in Table 6.

[Chem. 17]

Chemical formula 10

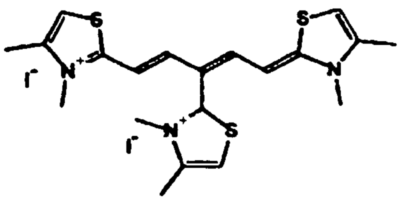

Chemical formula 11

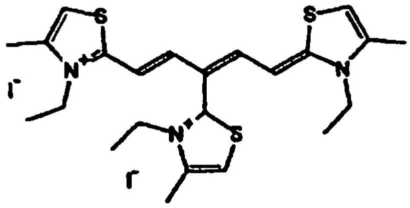

Chemical formula 12

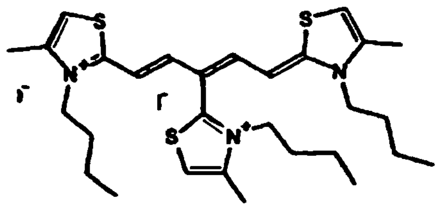

Chemical formula 13

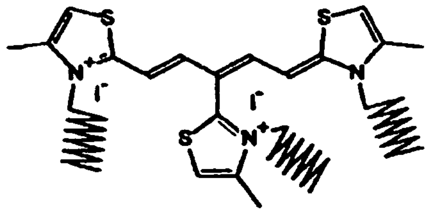

Chemical formula 14

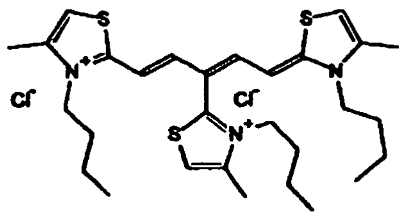

Chemical formula 15

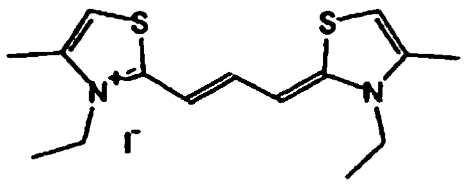

Chemical formula 16

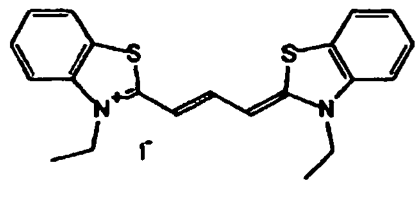

Chemical formula 17

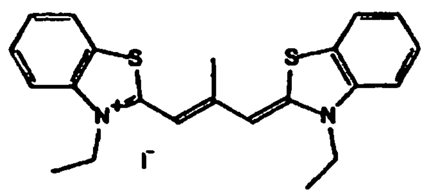

Chemical formula 18

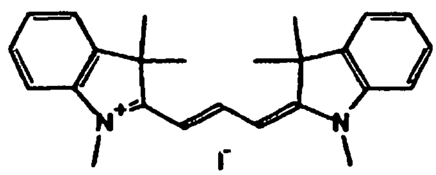

Chemical formula 19

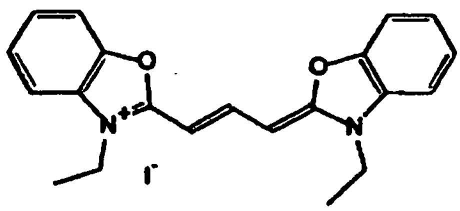

[Chem. 18]

Chemical formula 20

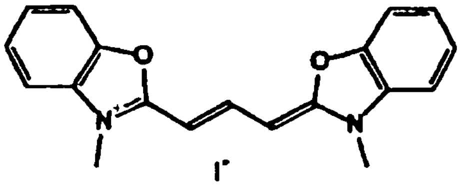

Chemical formula 21

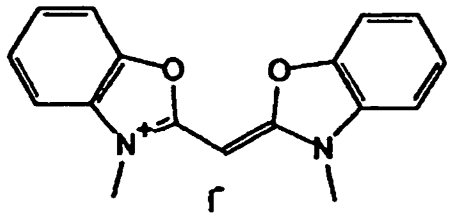

Chemical formula 22

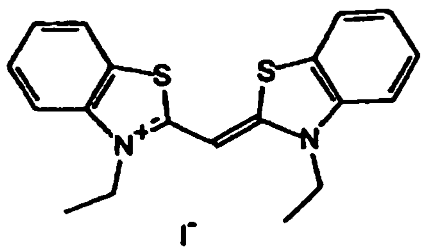

Chemical formula 23

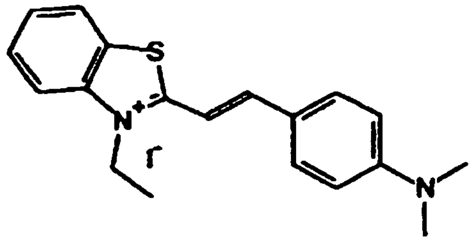

Chemical formula 24

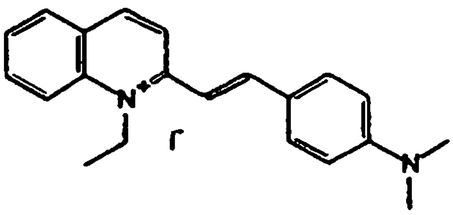

Chemical formula 25

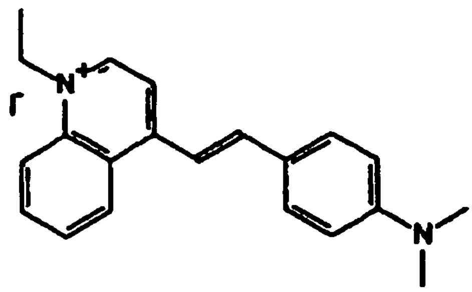

Chemical formula 26

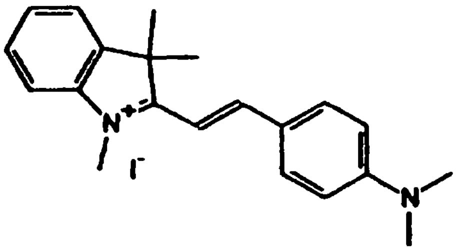

Chemical formula 27

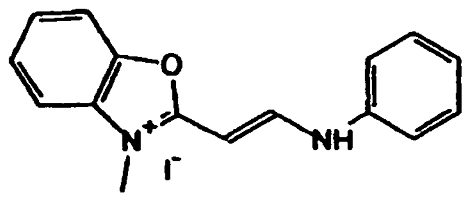

Chemical formula 28

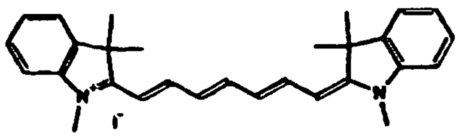

Chemical formula 29

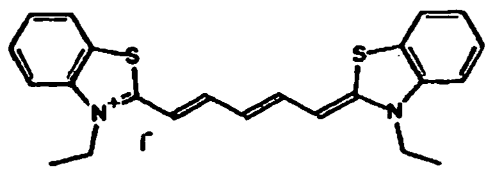

[Chem. 19]

Chemical formula 30

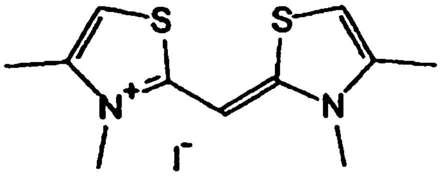

Chemical formula 31

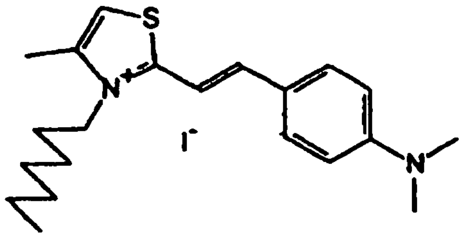

Chemical formula 32

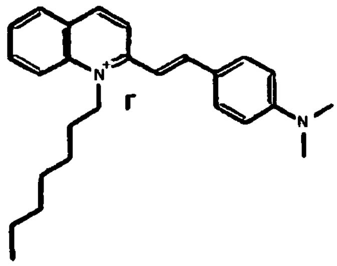

Chemical formula 33

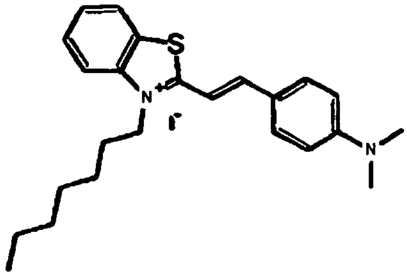

Chemical formula 34

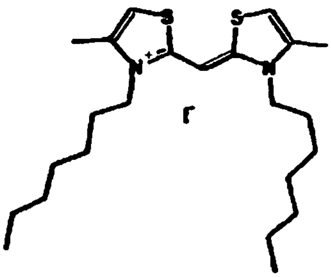

Chemical formula 35

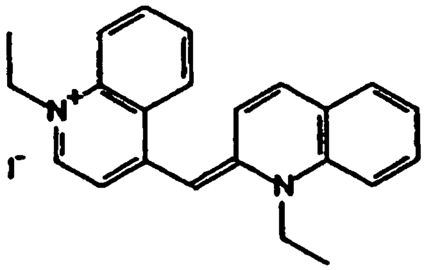

Chemical formula 36

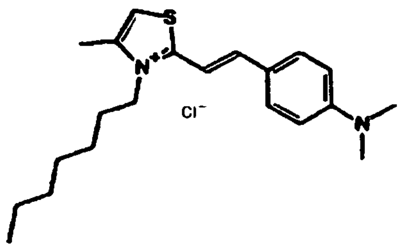

Chemical formula 37

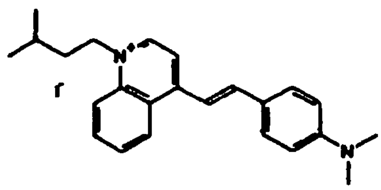

Chemical formula 38

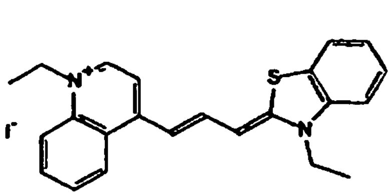

Chemical formula 39

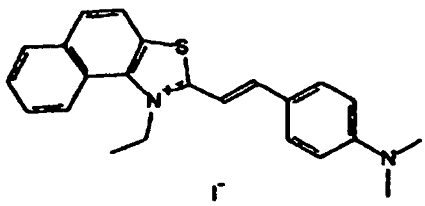

[Chem. 20]

**Chemical formula 40**

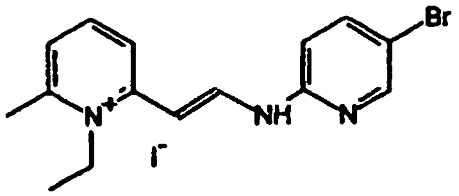

**Chemical formula 41**

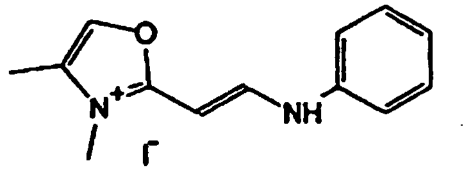

**Chemical formula 42**

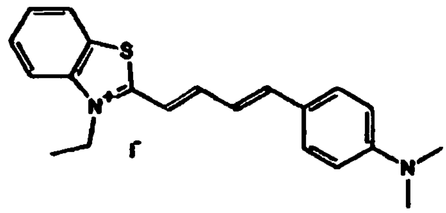

**Chemical formula 43**

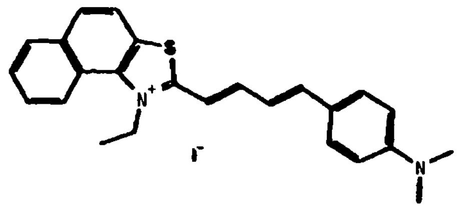

**Chemical formula 44**

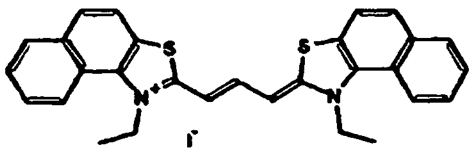

**Chemical formula 45**

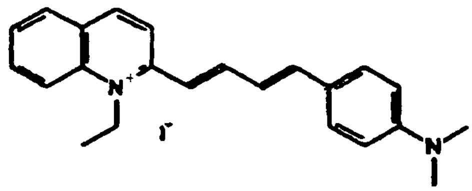

**Chemical formula 46**

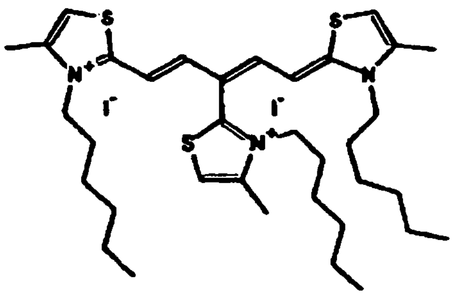

**Chemical formula 47**

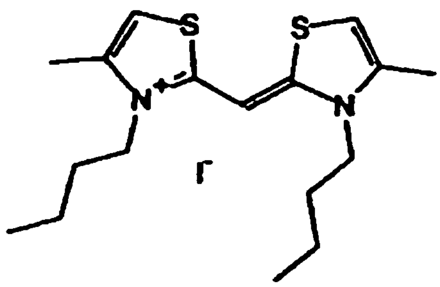

**Chemical formula 48**

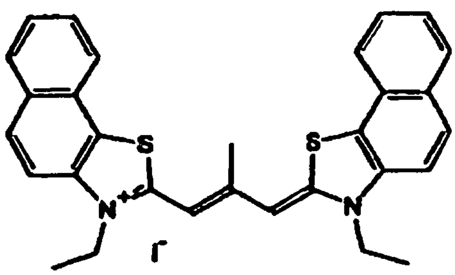

**Chemical formula 49**

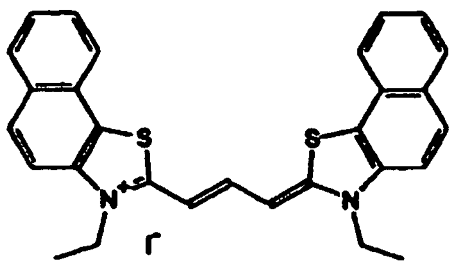

[Chem. 21]

Chemical formula 50

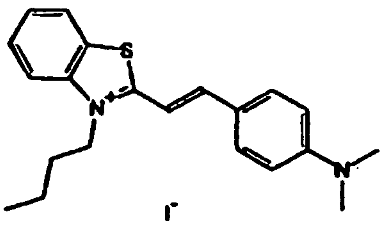

Chemical formula 51

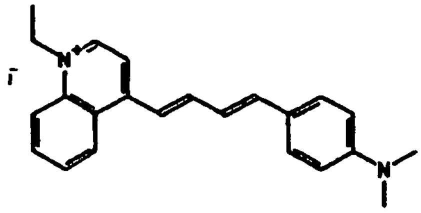

Chemical formula 52

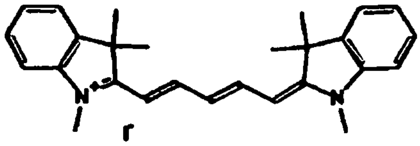

Chemical formula 53

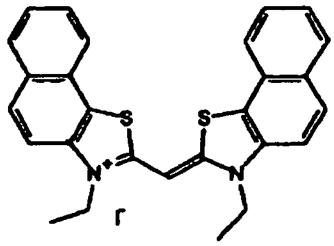

Chemical formula 54

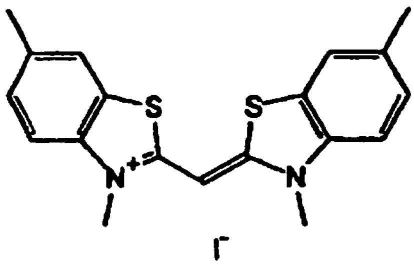

Chemical formula 55

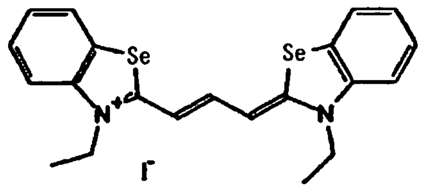

Chemical formula 56

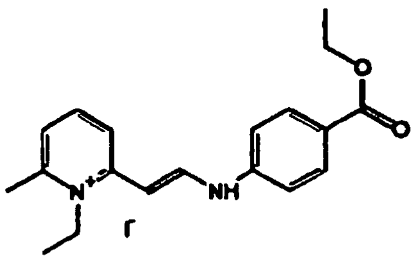

Chemical formula 57

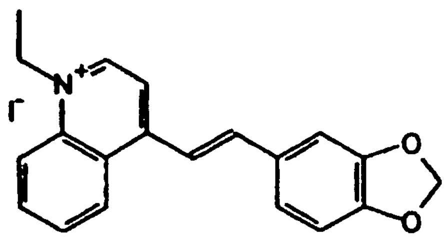

Chemical formula 58

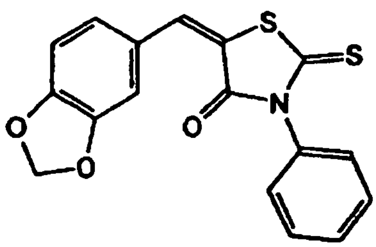

Chemical formula 59

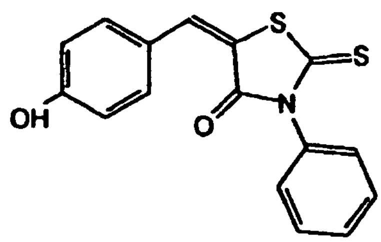

[Chem. 22]

Chemical formula 60

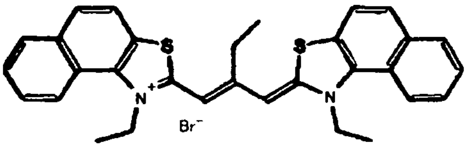

Chemical formula 61

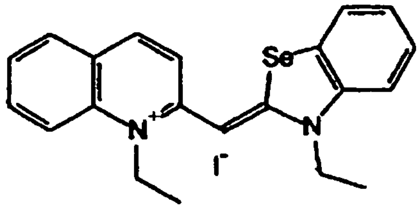

Chemical formula 62

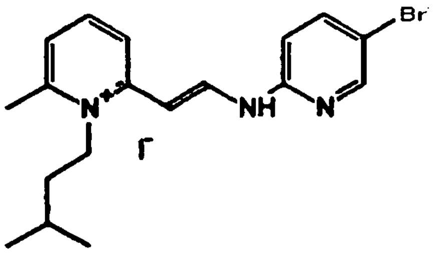

Chemical formula 63

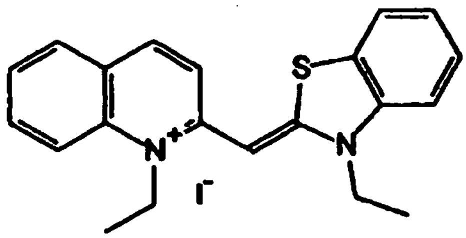

Chemical formula 64

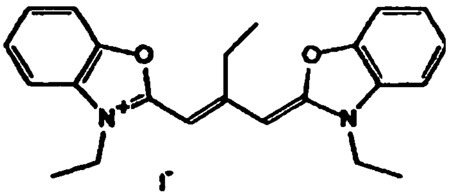

Chemical formula 65

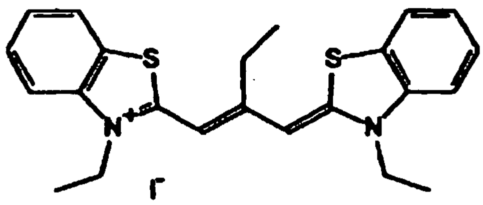

Chemical formula 66

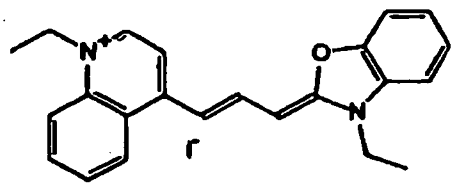

Chemical formula 67

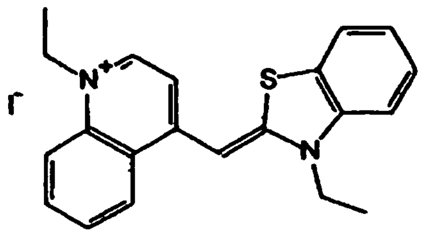

Chemical formula 68

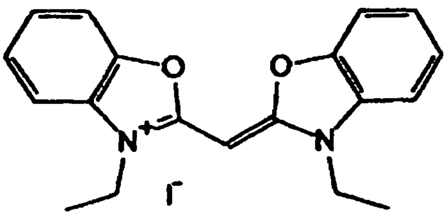

Chemical formula 69

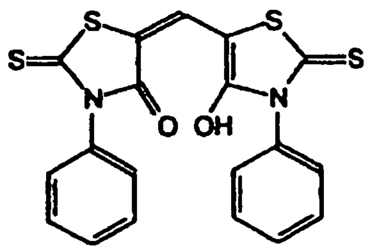

[Chem. 23]

Chemical formula 70

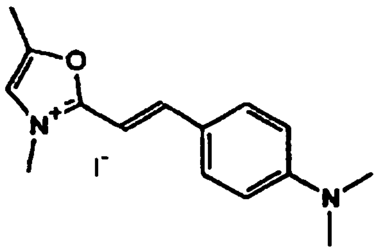

Chemical formula 71

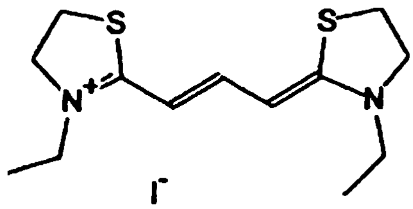

Chemical formula 72

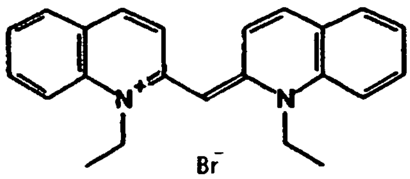

Chemical formula 73

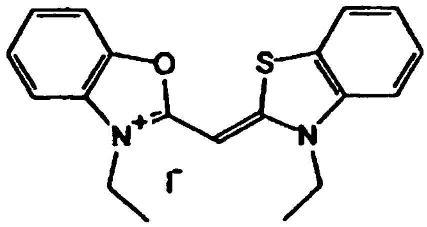

Chemical formula 74

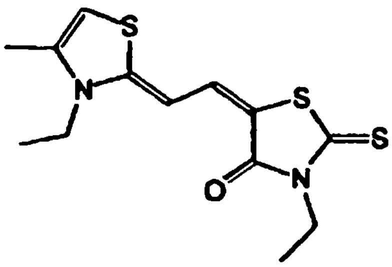

Chemical formula 75

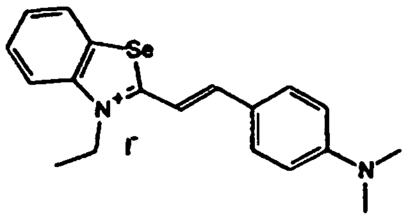

Chemical formula 76

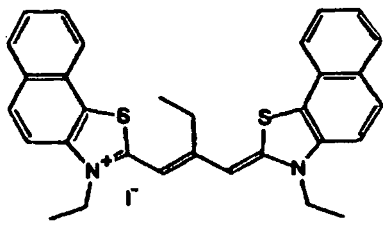

Chemical formula 77

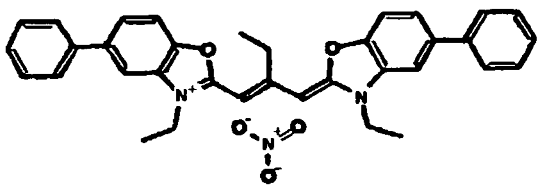

Chemical formula 78

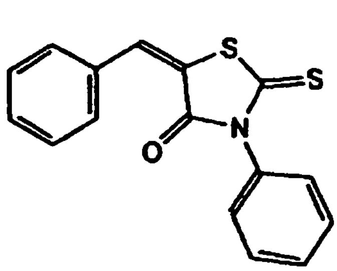

Chemical formula 79

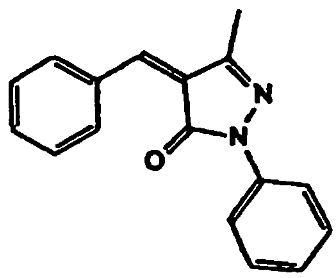

[Chem.24]

Chemical formula 80

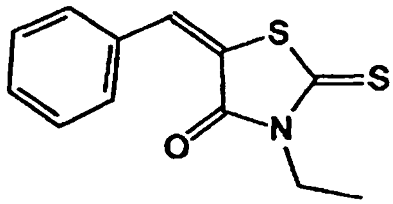

Chemical formula 81

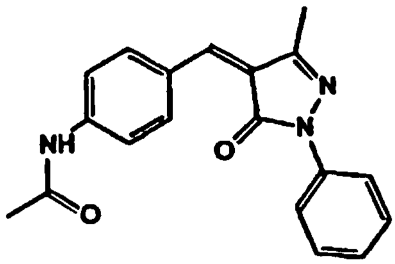

Chemical formula 82

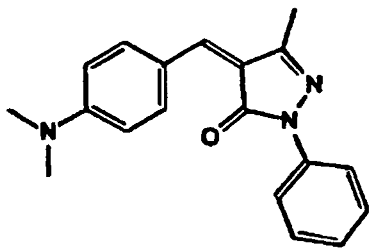

Chemical formula 83

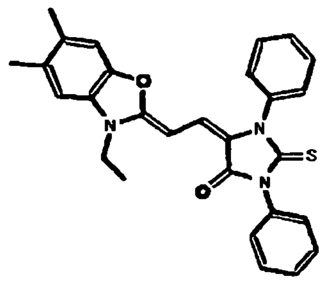

Chemical formula 84

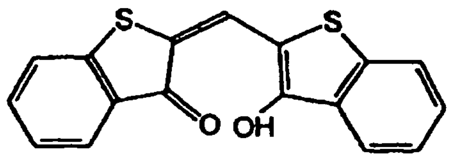

Chemical formula 85

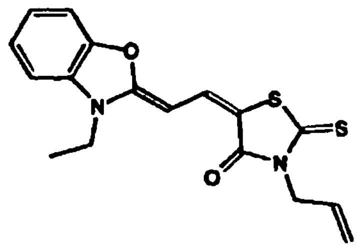

Chemical formula 86

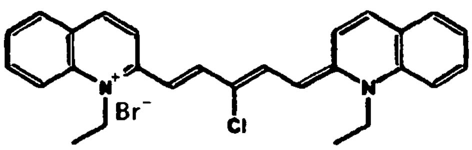

Chemical formula 87

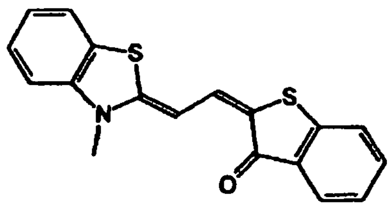

Chemical formula 88

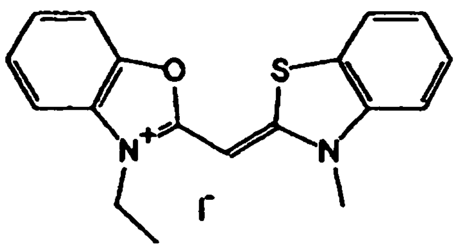

Chemical formula 89

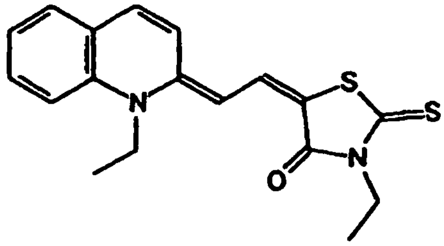

[Chem. 25]

Chemical formula 90

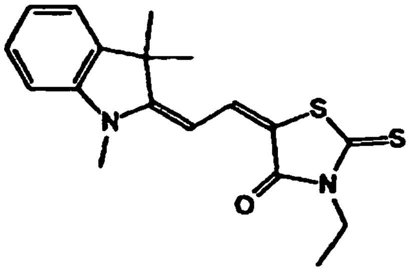

Chemical formula 91

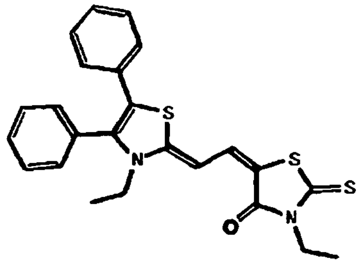

Chemical formula 92

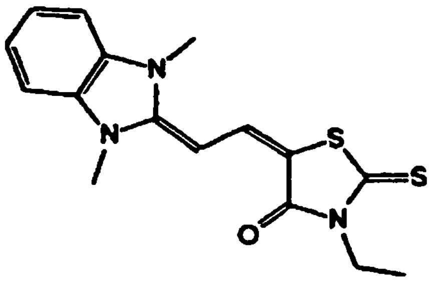

Chemical formula 93

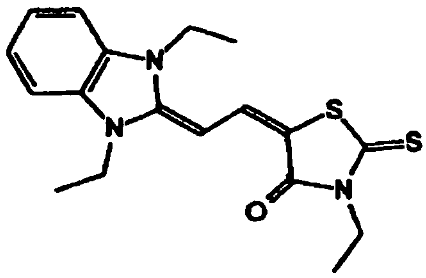

Chemical formula 94

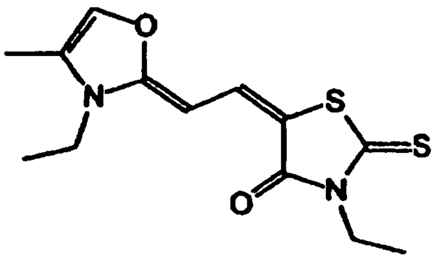

Chemical formula 95

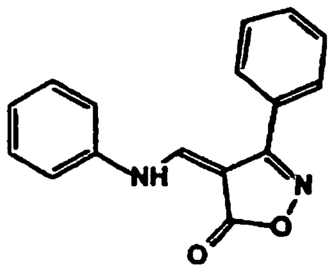

Chemical formula 96

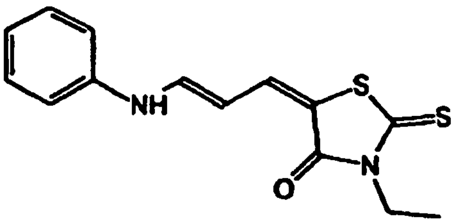

Chemical formula 97

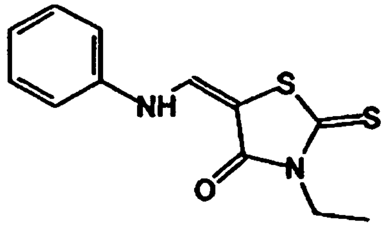

Chemical formula 98

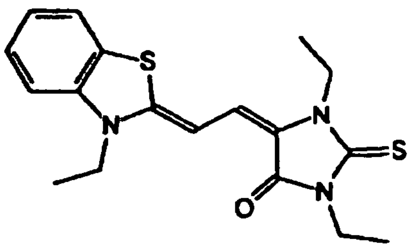

Chemical formula 99

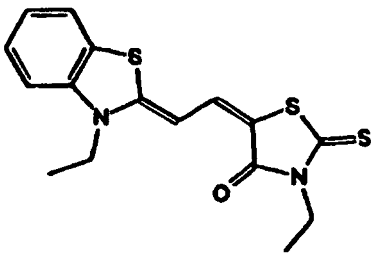

[Chem. 26]

Chemical formula 100

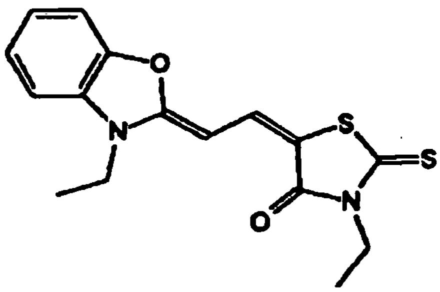

Chemical formula 101

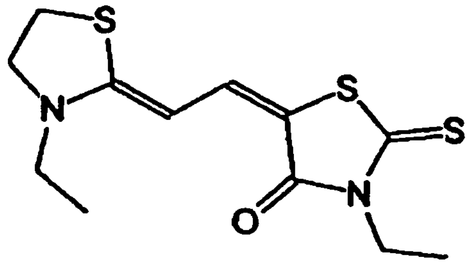

Chemical formula 102

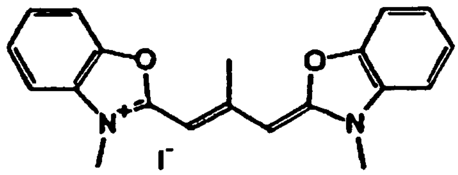

Chemical formula 103

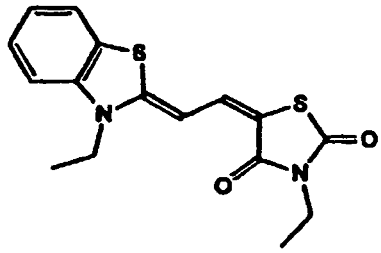

Chemical formula 104

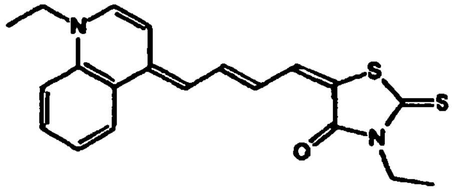

Chemical formula 105

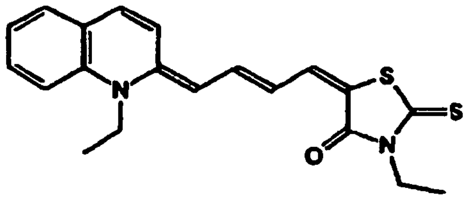

Chemical formula 106

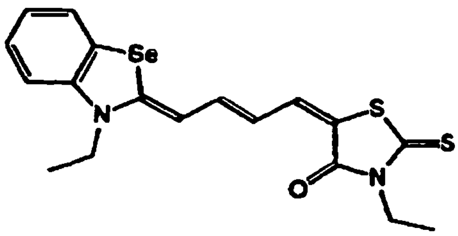

Chemical formula 107

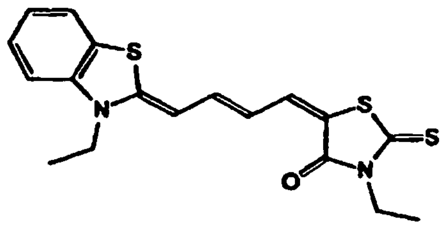

Chemical formula 108

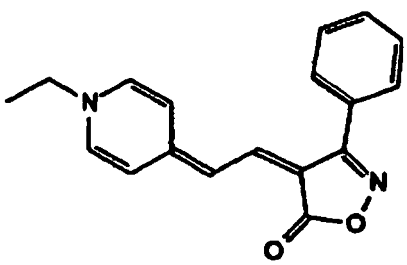

Chemical formula 109

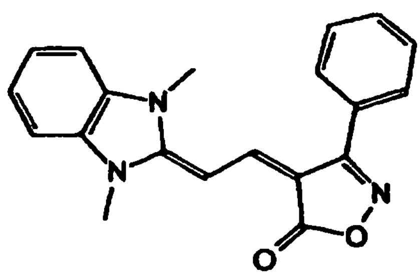

[Chem. 27]

Chemical formula 110

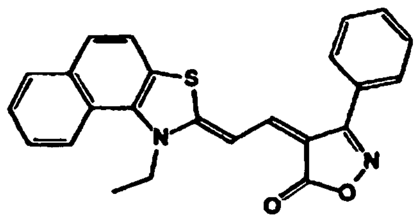

Chemical formula 111

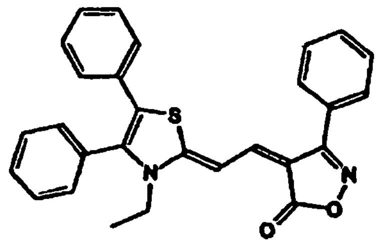

Chemical formula 112

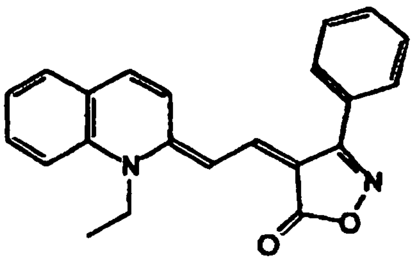

Chemical formula 113

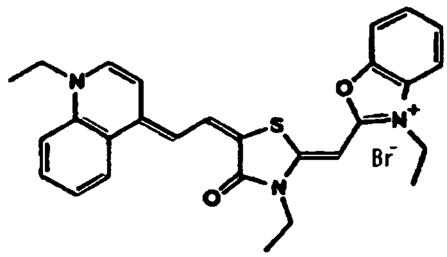

Chemical formula 114

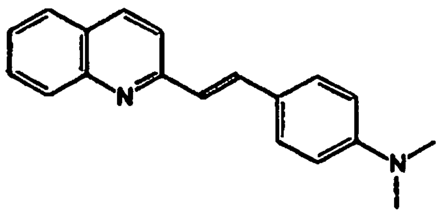

Chemical formula 115

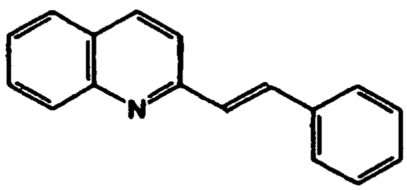

Chemical formula 116

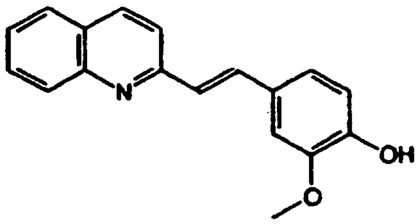

Chemical formula 117

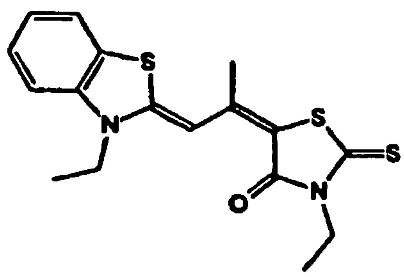

Chmical formula 118

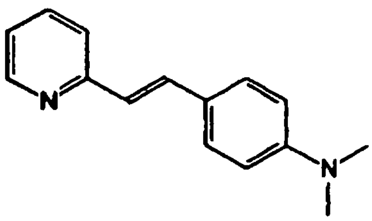

Chemical formula 119

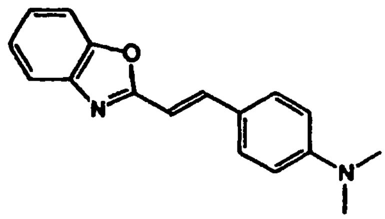

[Chem. 28]

Chemical formula 120

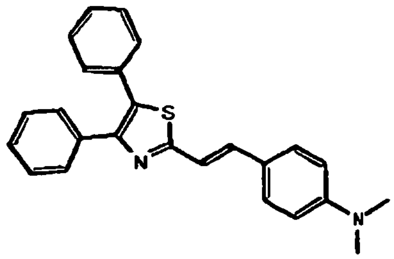

Chemical formula 121

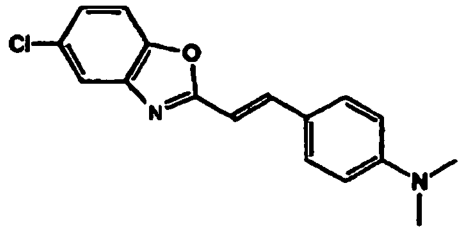

Chemical formula 122

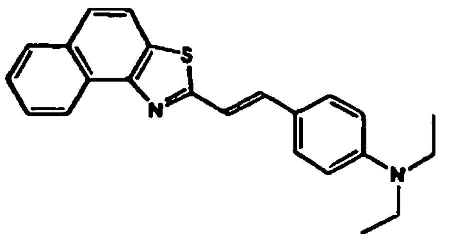

Chemical formula 123

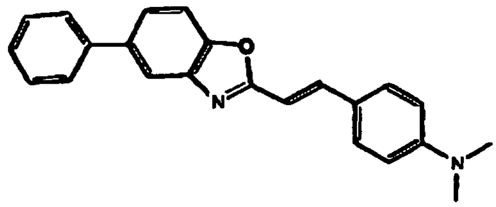

Chemical formula 124

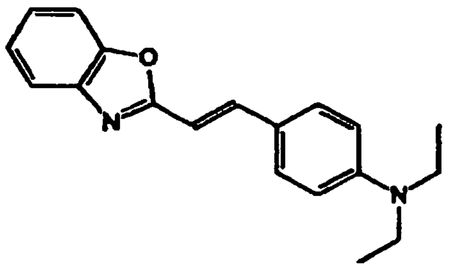

Chemical formula 125

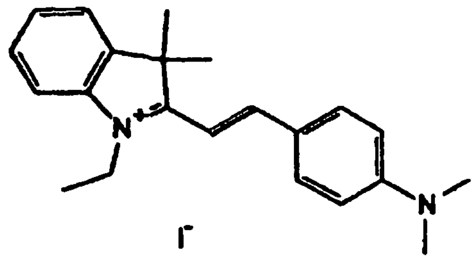

Chemical formula 126

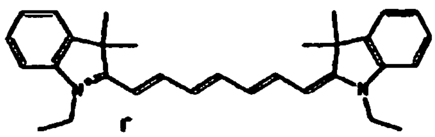

Chemical formula 127

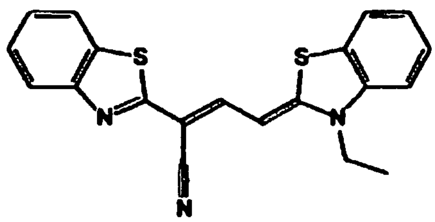

Chemical formula 128

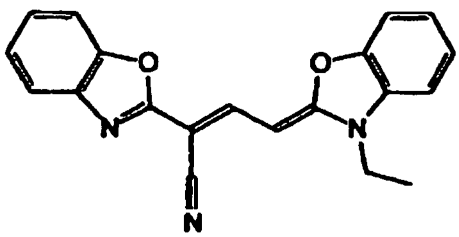

Chemical formula 129

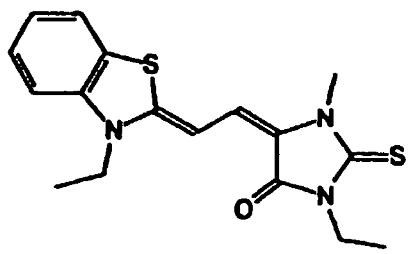

[Chem. 29]

Chemical formula 130

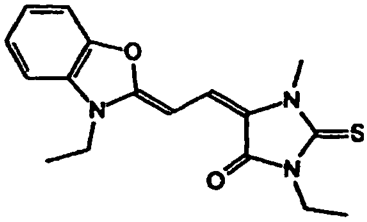

Chemical formula 131

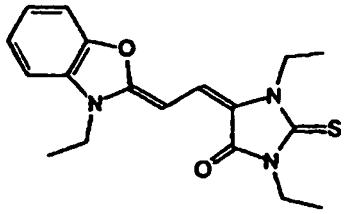

Chemical formula 132

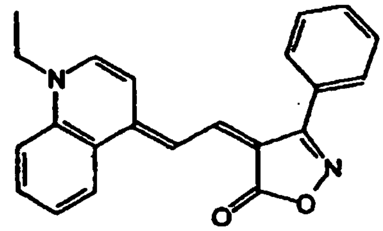

Chemical formula 133

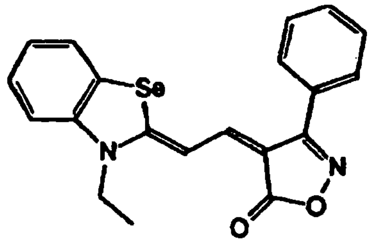

Chemical formula 134

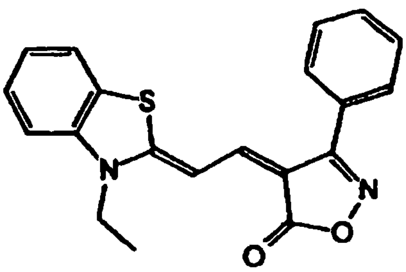

Chemical formula 135

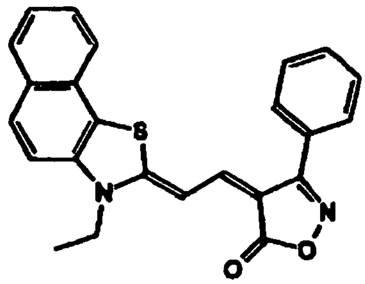

Chemical formula 136

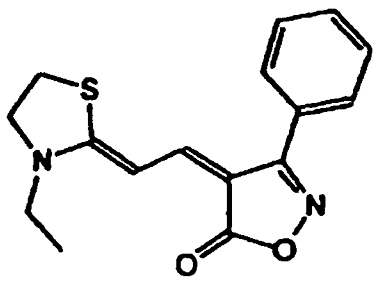

Chemical formula 137

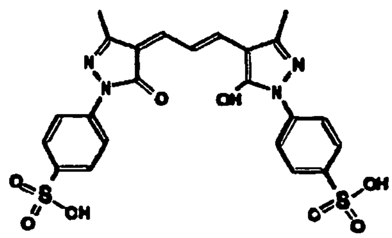

Chemical formula 138

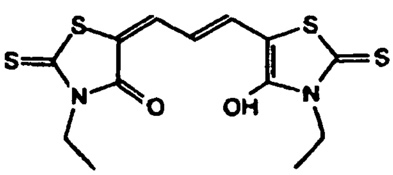

Chemical formula 139

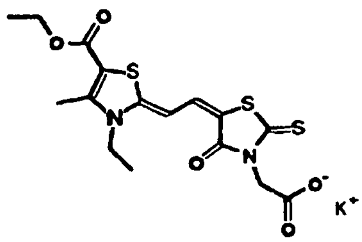

[Chem. 30]

Chemical formula 140

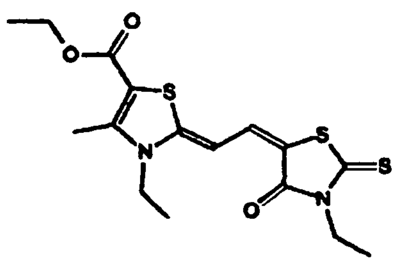

Chemical formula 141

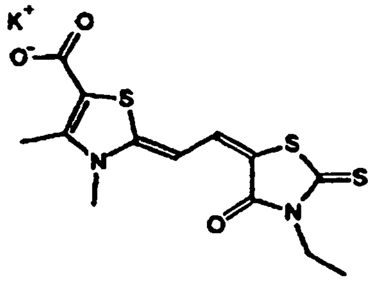

Chemical formula 142

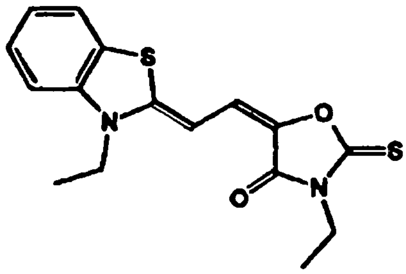

Chemical formula 143

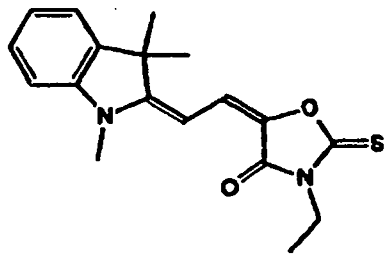

Chemical formula 144

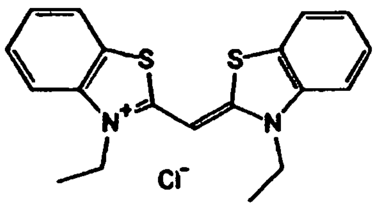

Chemical formula 145

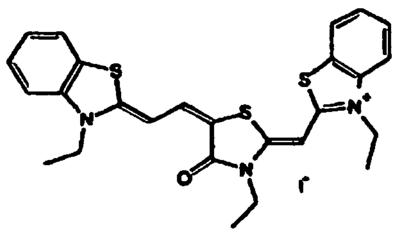

Chemical formula 146

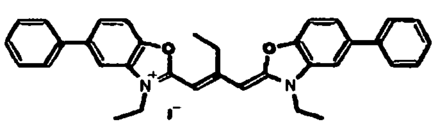

Chemical formula 147

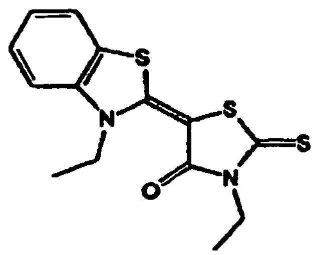

Chemical formula 148

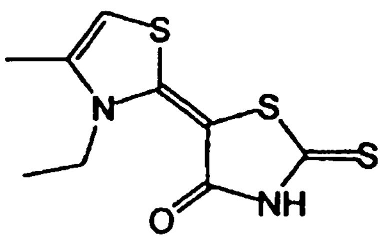

Chemical formula 149

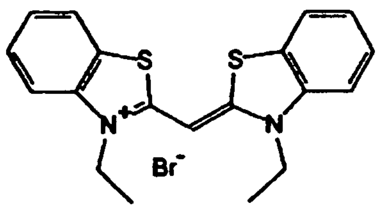

[Chem. 31]

Chemical formula 150

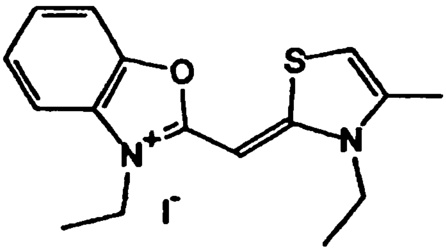

Chemical formula 151

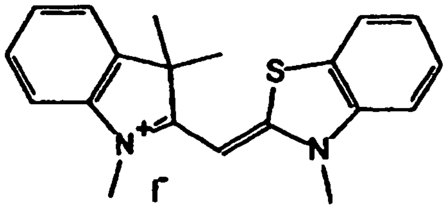

Chemical formula 152

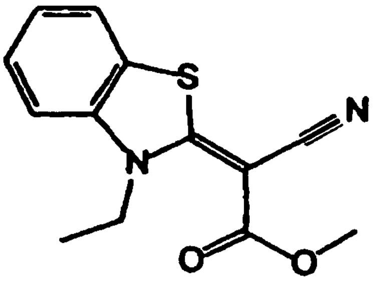

Chemical formula 153

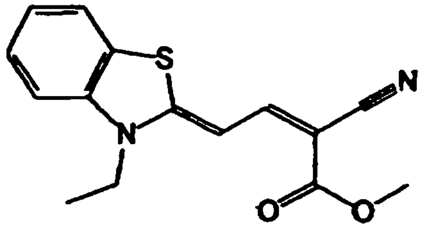

Chemical formula 154

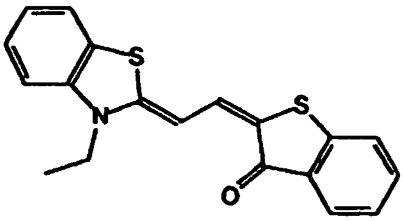

Chemical formula 155

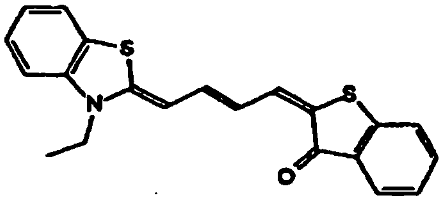

Chemical formula 156

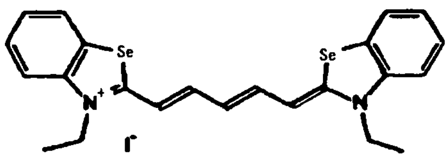

Chemical formula 157

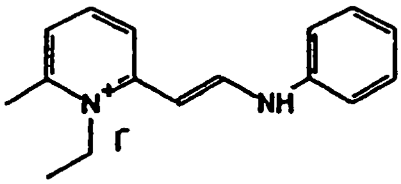

Chemical formula 158

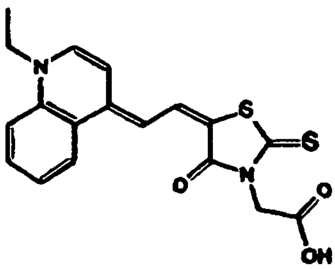

Chemical formula 159

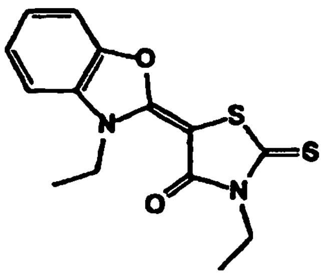

[Chem. 32]

Chemical formula 160

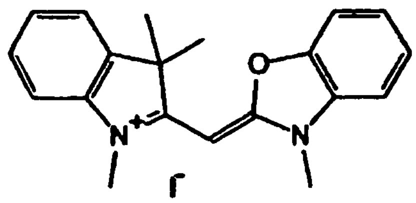

Chemical formula 161

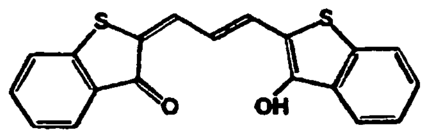

Chemical formula 162

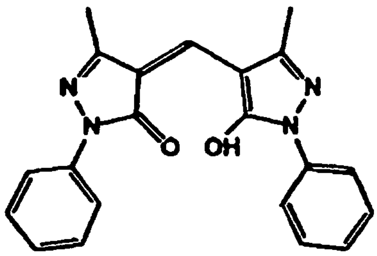

Chemical formula 163

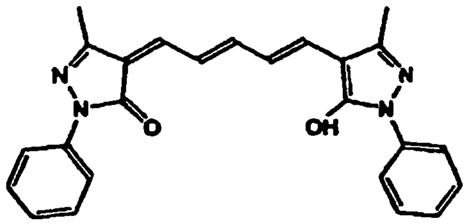

Chemical formula 164

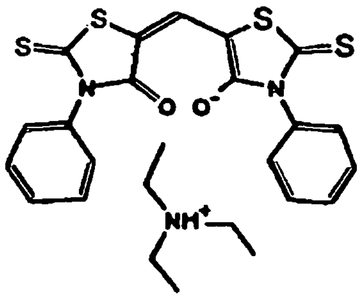

Chemical formula 165

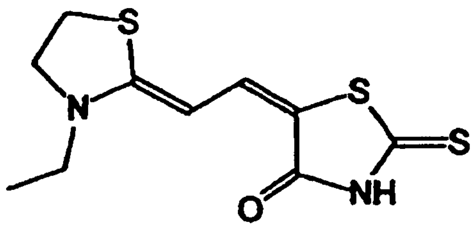

Chemical formula 166

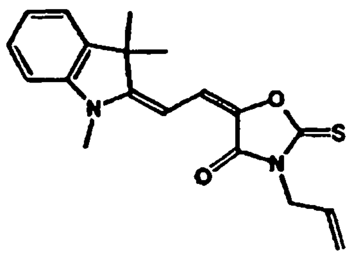

Chemical formula 167

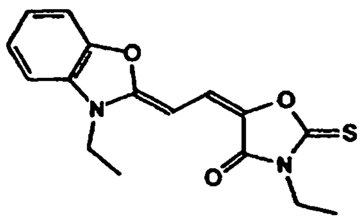

Chemical formula 168

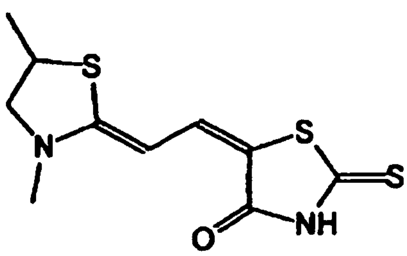

Chemical formula 169

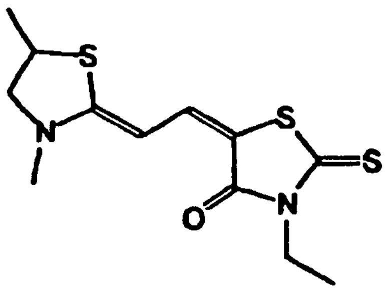

[Chem. 33]

Chemical formula 170

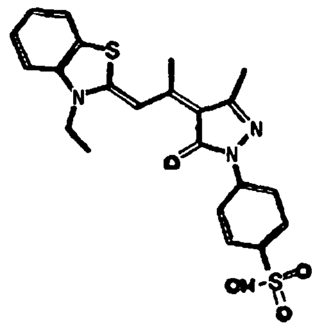

Chemical formula 171

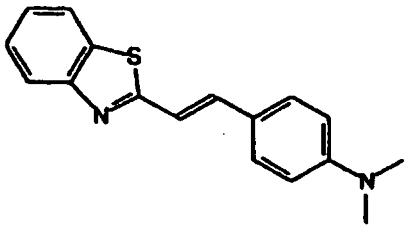

Chemical formula 172

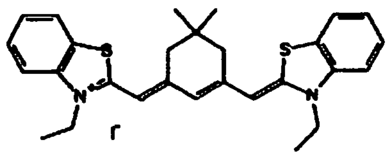

Chemical formula 173

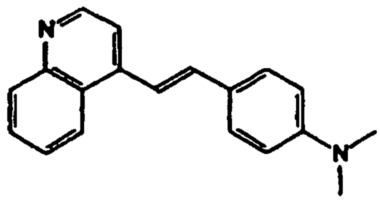

Chemical formula 174

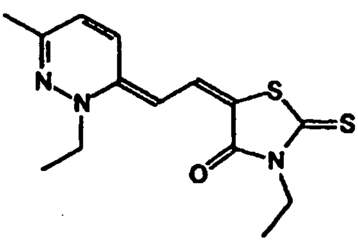

Chemical formula 175

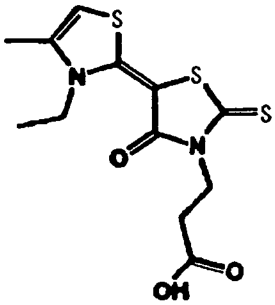

Chemical formula 176

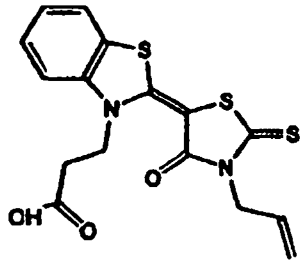

Chemical formula 177

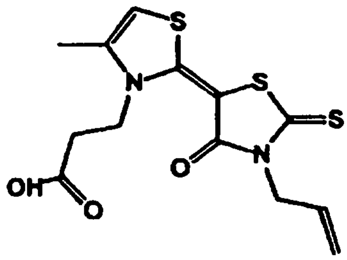

Chemical formula 178

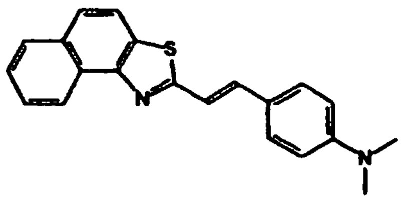

Chemical formula 179

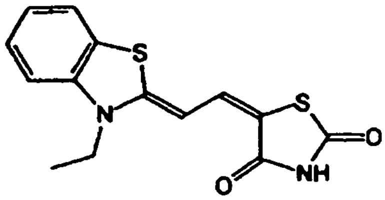

[Chem. 34]

Chemical formula 180

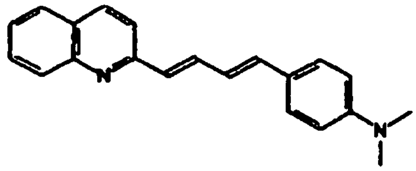

Chemical formula 181

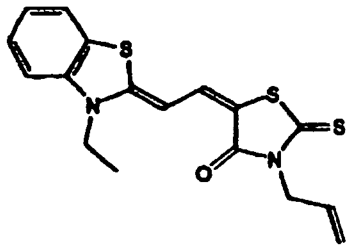

Chemical formula 182

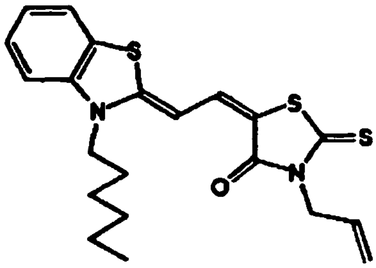

Chemical formula 183

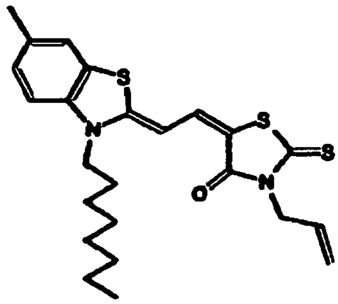

Chemical formula 184

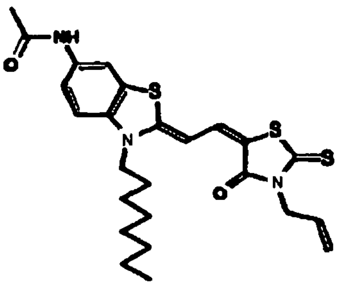

Chemical formula 185

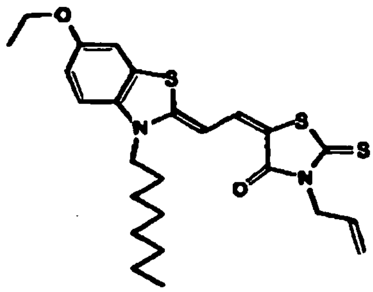

Chemical formula 186

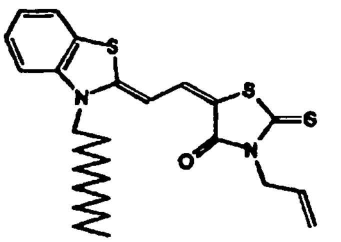

Chemical formula 187

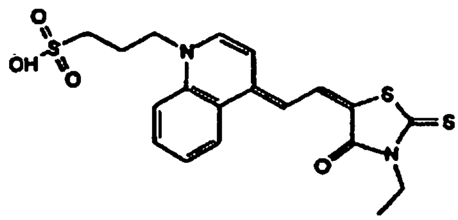

Chemical formula 188

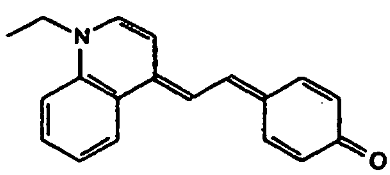

Chemical formula 189

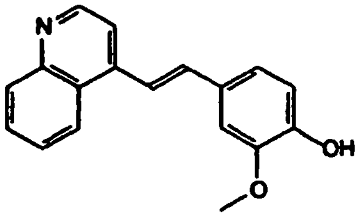

[Chem. 35]

Chemical formula 190

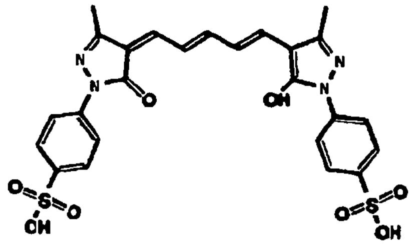

Chemical formula 191

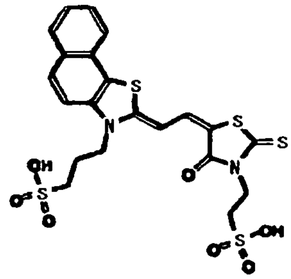

Chemical formula 192

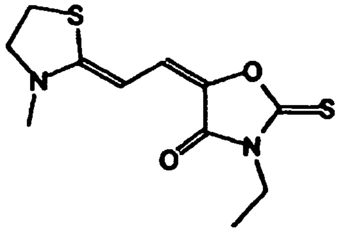

Chemical formula 193

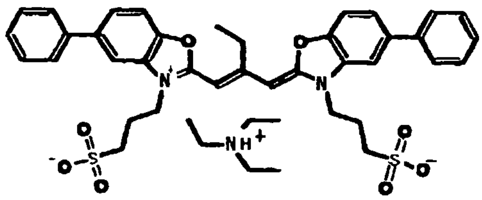

Chemical formula 194

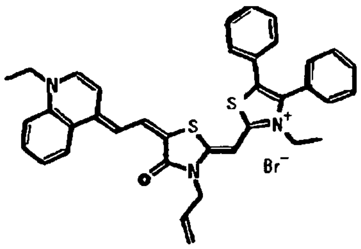

Chemical formula 195

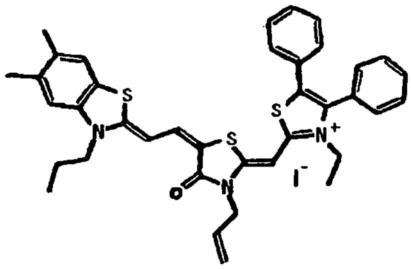

Chemical formula 196

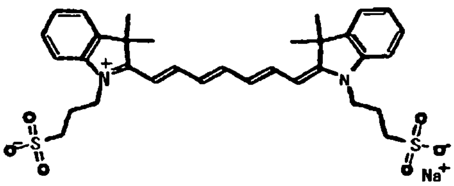

Chemical formula 197

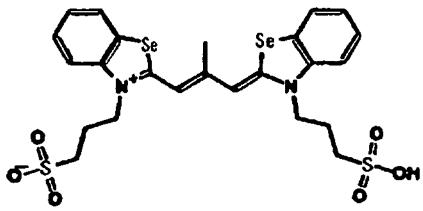

Chemical formula 198

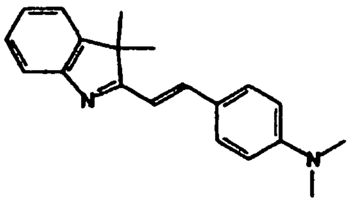

Chemical formula 199

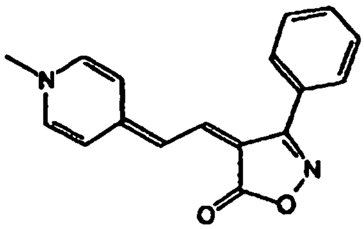

[Chem. 36]

Chemical formula 200

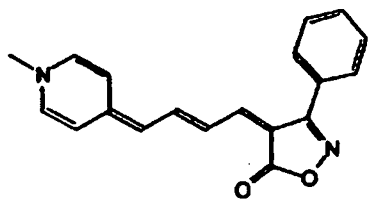

Chemical formula 201

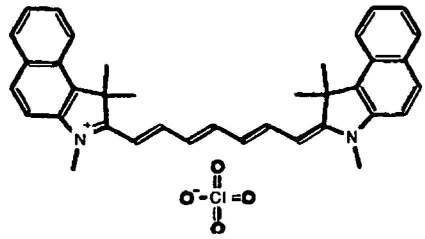

Chemical formula 202

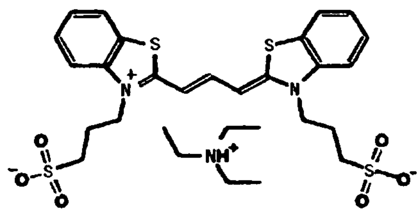

Chemical formula 203

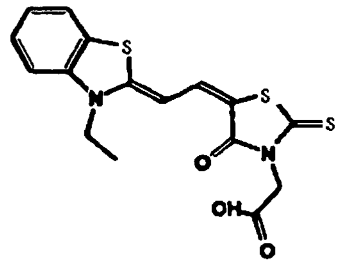

Chemical formula 204

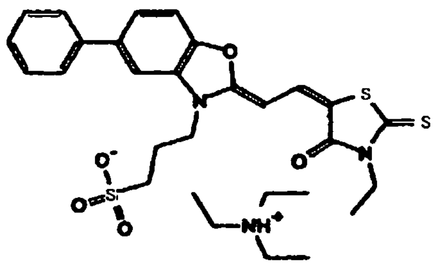

Chemical formula 205

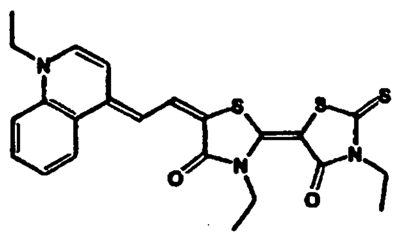

Chemical formula 206

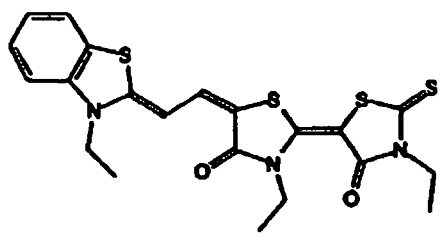

Chemical formula 207

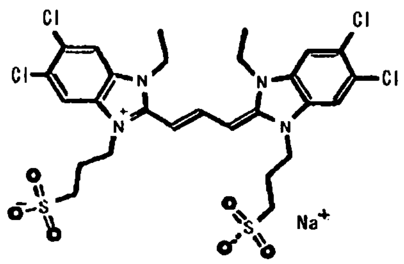

Chemical formula 208

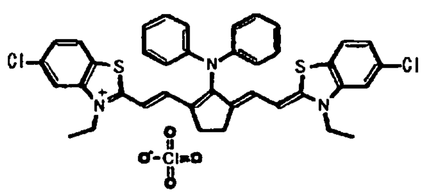

Chemical formula 209

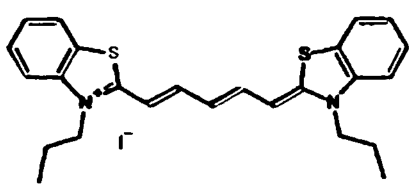

[Chem. 37]

Chemical formula 210

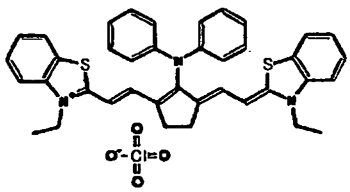

Chemical formula 211

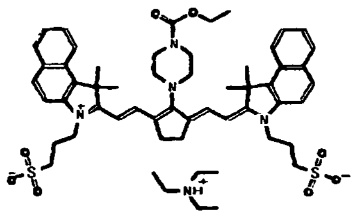

Chemical formula 212

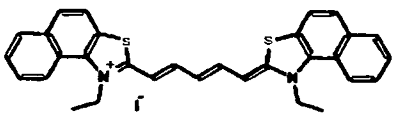

Chemical formula 213

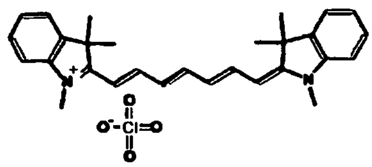

Chemical formula 214

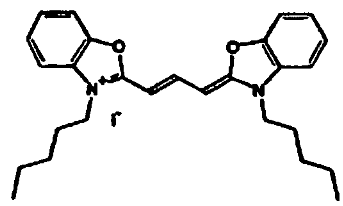

Chemical formula 215

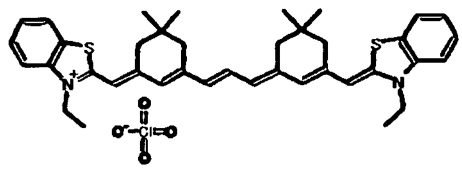

Chemical formula 216

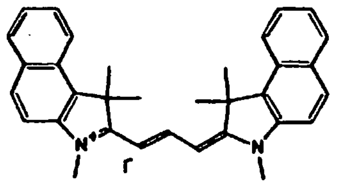

Chemical formula 217

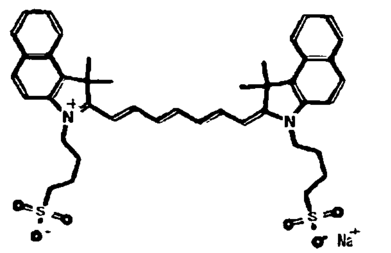

Chemical formula 218

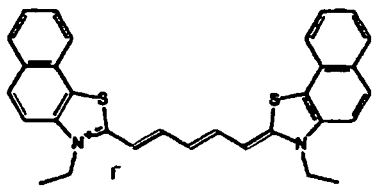

Chemical formula 219

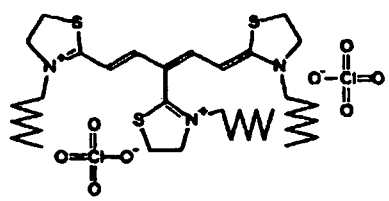

Chemical formula 220

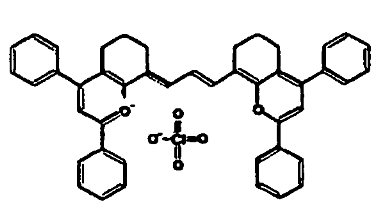

Chemical formula 221

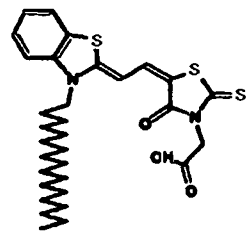

[Chem. 38]

Chemical formula 222

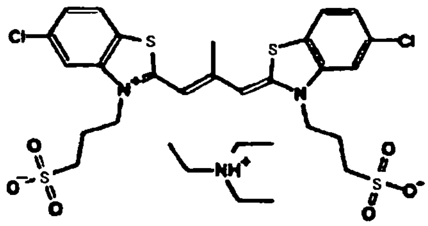

Chemical formula 223

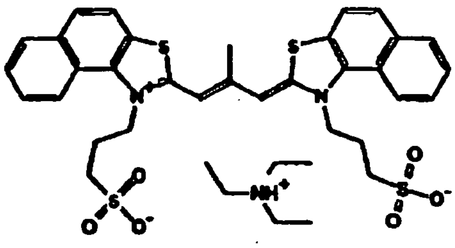

Chemical formula 224

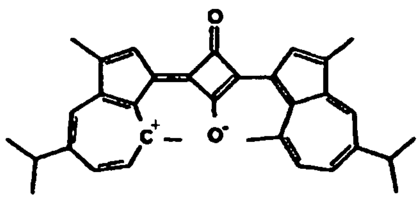

Chemical formula 225

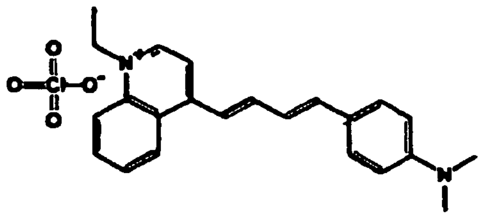

Chemical formula 226

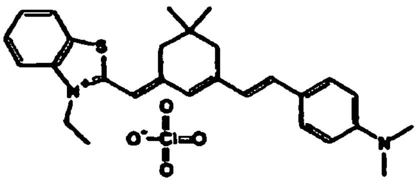

Chemical formula 227

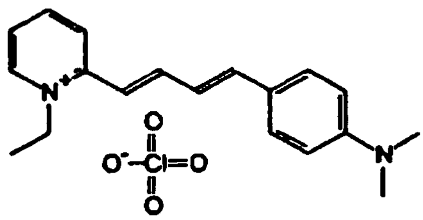

Chemical formula 228

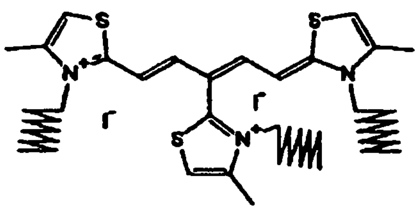

Chemical formula 229

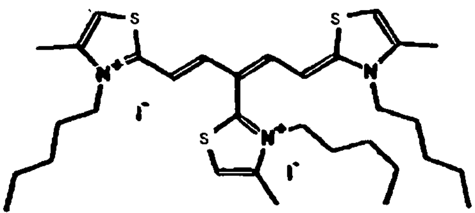

Chemical formula 230

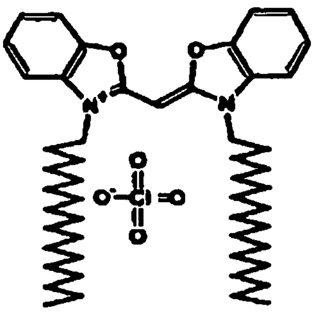

Chemical formula 231

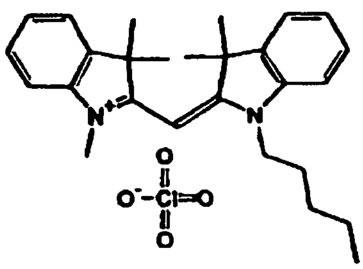

[Chem. 39]

Chemical formula 232

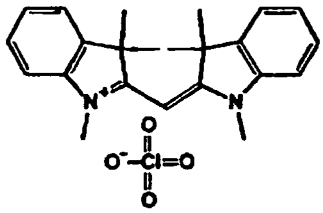

Chemical formula 233

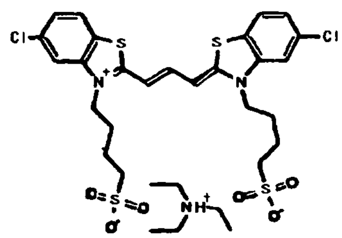

Chemical formula 234

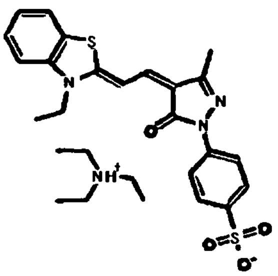

Chemical formula 235

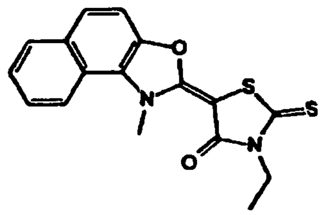

Chemical formula 236

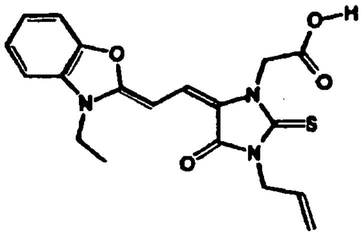

Chemical formula 237

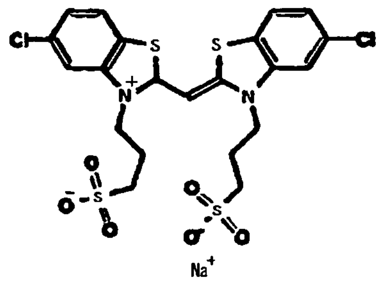

Chemical formula 238

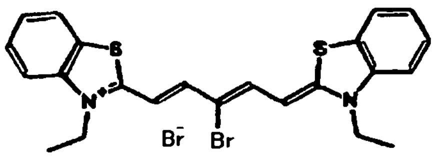

Chemical formula 239

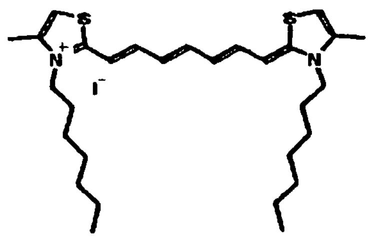

Chemical formula 240

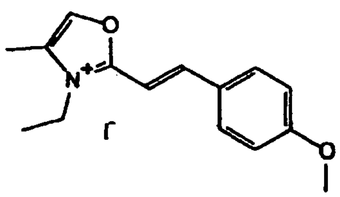

Chemical formula 241

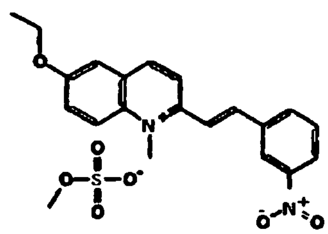

<Test sample>

Since most of the 239 types of compounds in Table 6 are substantially insoluble in water, they were dissolved in DMSO, Catalog No. "D8418", commercialized by Sigma-Aldrich Co., MO, USA, to give a concentration of 5 mg/ml;

filtrated using a DMSO-resistant membrane filter, "MILLEX-LG", Product No. "LLG025SS", commercialized by Millipore, MA, USA; and preserved at 25°C under light-shielded conditions. Just before use, each stock solution was diluted with Dulbecco's Modified Eagle Medium (D-MEM, commercialized by Nissui, Tokyo, Japan) supplemented with 10% by volume of fetal bovine serum (FBS) by 200-folds or more to make into a test solution for use in tests. These compounds were synthesized in Hayashibara Biochemical Laboratories Inc., Okayama, Japan.

Evaluation A: Method of evaluating neurocyte-growth-promoting action

According to the same method as in Experiment 1, PC12-HS cells were diluted with D-MEM supplemented with 10% by volume of FBS, and 100 μl/well of the diluted cell suspension was poured into a 96-well microplate pre-coated with collagen to give a cell density of 5 x $10^3$ cells/well. After culturing the cells for 24 hours, 100μl of each test sample solution, which was prepared by diluting the stock solution with D-MEM supplemented with 10% by volume of FBS to give a concentration of 100 ng/ml, was added to each well and then the cells were cultured further at 37°C for three days in a 5% (v/v) $CO_2$ incubator. After culturing for three days, the culture supernatant of each well was removed and then the cells were admixed with 20 μl/well of 10% (w/w) "ALAMAR BLUE", commercialized by Trek Diagnostic Systems, Cleveland, USA, prepared with D-MEM supplemented with 10% by volume of FBS, and further cultured at 37°C for six hours in a 5% (v/v) $CO_2$ incubator. After the cultivation, the fluorescent intensity of each well at a wavelength of 544 to 590 nm was measured using a fluorescence plate reader, commercialized by Molecular Device Japan, Tokyo, Japan. The action of cell growth promotion in each test sample was evaluated by a relative value of its fluorescent intensity to that of the control, regarded as 100%, while the control was prepared by adding D-MEM supplemented with 10% by volume of FBS without any test sample. Then, the effects of test samples were judged as follows:

The relative value of 140 to 199: Equivalent effect to that of NK-4, symbol (o);
The relative value of 200 or higher: Stronger effect than that of NK-4, symbol (•).

The results are in Table 6.

Evaluation B: Method of evaluating neurite-outgrowth action

As in the cases of measuring cell-growth-promoting action, PC12-HS cells were diluted with D-MEM supplemented with 10% by volume of FBS, and 100 μl/well of a diluted cell suspension was poured into a well of 96-well microplate pre-coated with collagen to give a cell density of 5 x $10^3$ cells/well. After culturing for 24 hours, 50 μl/well of each test sample solution, prepared by diluting the stock solution with D-MEM supplemented with 10% by volume of FBS to give a concentration of 400 ng/ml, and 50 μl/well of D-MEM supplemented with 10% by volume of FBS, containing 20 ng/ml of mouse NGF commercialized by Chemicon International, Inc., CA, USA, (at a final concentration of 5 ng/ml) were added to each well, and then further cultured for three days. On the $3^{rd}$ day of culturing, the cells were immobilized by treating with 10% by volume of glutaraldehyde at ambient temperature for 20 minutes. As a control, PC12-HS cells, cultured for thee days with D-MEM supplemented with 10% by volume of FBS, were immobilized with glutaraldehyde similarly asabove. The resulting immobilized cells were microscopically observed and the neurite outgrowth was evaluated. The test sample with a neurite outgrowth rate of 30% or higher was judged to be equivalent or more (o) compared with NK-4. The neurite-outgrowth rate (%) was determined by observing the cells using a microscope by adjusting the scale factor to give about 100 cells in a perspective, counting cells showing neurite outgrowth by 2-folds or higher than the cell size, dividing the resulting count by the total cell count in the perspective, and multiplying the resulting numeral by 100. When NGF was solely added to the experimental system (5 ng/ml), the neurite outgrowth rate of cells was about 5%. The results were also in Table 6.

Evaluation C: Method of evaluating the protective action against cytotoxicity by amyloid β fragment

Protective actions against cytotoxicity by amyloid β fragment of the test samples, which had showed cell-growth-promoting action in Evaluation A, were investigated whether they have protective action against cytotoxicity by amyloid β fragment by the same method as in Experiment 1. A test sample which showed significant inhibition of cytotoxicity by amyloid β fragment was judged to be "protective" and expressed with a symbol, (o). The results are also in Table 6.

In Table 6 and in the following Experiments, NK-4, NK-19 and NK-53 are exemplary compounds for use according to the present invention.

Table 6

| NK No. | Chemical formula No. | Evaluation A | Evaluation B | Evaluation C | NK No. | Chemical formula No. | Evaluation A | Evaluation B | Evaluation C |
|---|---|---|---|---|---|---|---|---|---|
| 2 | 10 | | | - | 96 | 25 | | ○ | - |
| 4 | 2 | ○ | ○ | ○ | 97 | 26 | | | - |
| 13 | 11 | | | - | 100 | 6 | ○ | ○ | ○ |
| 19 | 4 | ○ | ○ | ○ | 120 | 27 | ● | | ○ |
| 24 | 12 | | | - | 125 | 28 | | ○ | - |
| 32 | 13 | | | - | 136 | 29 | | ○ | - |
| 53 | 5 | ○ | ○ | ○ | 141 | 30 | | | - |
| 56 | 14 | | | - | 143 | 31 | | ○ | - |
| 67 | 15 | | ○ | - | 231 | 32 | | ○ | - |
| 76 | 16 | | | - | 233 | 33 | | | - |
| 77 | 17 | | | - | 266 | 34 | | ○ | - |
| 79 | 18 | | | - | 276 | 35 | | ○ | - |
| 85 | 19 | | ○ | - | 279 | 36 | | ○ | - |
| 86 | 20 | | ○ | - | 282 | 37 | | ○ | - |
| 87 | 21 | | ○ | - | 321 | 38 | | | - |
| 88 | 22 | | | - | 342 | 39 | | | - |

| | | | | | | | | | |
|---|---|---|---|---|---|---|---|---|---|
| 91 | 23 | | | - | 343 | 40 | ○ | | ○ |
| 92 | 24 | | ○ | - | 355 | 41 | ○ | | ○ |

Table 6 (Continued)

| NK No. | Chemical formula No. | Evaluation A | Evaluation B | Evaluation C | NK No. | Chemical formula No. | Evaluation A | Evaluation B | Evaluation C |
|---|---|---|---|---|---|---|---|---|---|
| 375 | 42 | | ○ | - | 659 | 58 | ○ | | ○ |
| 376 | 43 | | | - | 660 | 59 | ○ | | ○ |
| 382 | 44 | | ○ | - | 716 | 60 | | | - |
| 383 | 45 | | ○ | - | 719 | 61 | | ○ | - |
| 392 | 46 | | | - | 721 | 62 | ○ | | ○ |
| 399 | 47 | | ○ | - | 723 | 63 | | ○ | - |
| 462 | 48 | | | - | 736 | 64 | | | - |
| 467 | 49 | | | - | 737 | 65 | | | - |
| 490 | 50 | | ○ | - | 741 | 66 | | ○ | - |
| 526 | 51 | | ○ | - | 750 | 67 | | ○ | - |
| 528 | 7 | ○ | ○ | ○ | 863 | 68 | | ○ | - |
| 529 | 52 | | | - | 913 | 69 | | | - |
| 557 | 8 | ○ | ○ | ○ | 916 | 70 | | | - |
| 571 | 53 | | | - | 1045 | 71 | | | - |

| | | | | | | | | | |
|---|---|---|---|---|---|---|---|---|---|
| 594 | 54 | | ○ | - | 1046 | 72 | | ○ | - |
| 616 | 55 | | | - | 1049 | 73 | | ○ | - |
| 618 | 56 | | | - | 1050 | 74 | ● | | ○ |
| 629 | 57 | | ○ | - | 1055 | 75 | | ○ | - |

Table 6 (Continued)

| NK No. | Chemical formula No. | Evaluation A | Evaluation B | Evaluation C | NK No. | Chemical formula No. | Evaluation A | Evaluation B | Evaluation C |
|---|---|---|---|---|---|---|---|---|---|
| 1056 | 76 | | | - | 1223 | 94 | ○ | | ○ |
| 1067 | 77 | | | - | 1225 | 95 | | | - |
| 1075 | 78 | ○ | | ○ | 1228 | 96 | ● | | ○ |
| 1076 | 79 | ○ | | ○ | 1229 | 97 | ○ | | ○ |
| 1077 | 80 | ○ | | ○ | 1237 | 98 | ○ | | ○ |
| 1083 | 81 | | | - | 1247 | 99 | | | - |
| 1107 | 82 | ○ | | ○ | 1249 | 100 | ○ | | ○ |
| 1113 | 83 | ○ | | ○ | 1268 | 101 | ○ | | ○ |
| 1128 | 84 | ○ | | ○ | 1300 | 102 | | ○ | |
| 1141 | 85 | ○ | | ○ | 1315 | 103 | | | |
| 1150 | 86 | | | - | 1318 | 104 | | | |
| 1157 | 87 | ○ | | ○ | 1319 | 105 | | | |

| | | | | | | | | | |
|---|---|---|---|---|---|---|---|---|---|
| 1204 | 88 | | | - | 1320 | 106 | | | |
| 1218 | 89 | ○ | | ○ | 1321 | 107 | | | |
| 1219 | 90 | ○ | | ○ | 1322 | 108 | | | |
| 1220 | 91 | ○ | | ○ | 1323 | 109 | | | |
| 1221 | 92 | ○ | | ○ | 1324 | 110 | | | |
| 1222 | 93 | ● | | ○ | 1326 | 111 | ○ | | ○ |

Table 6 (Continued)

| NK No. | Chemical formula No. | Evaluation A | Evaluation B | Evaluation C | NK No. | Chemical formula No. | Evaluation A | Evaluation B | Evaluation C |
|---|---|---|---|---|---|---|---|---|---|
| 1328 | 112 | | | | 1427 | 130 | ● | | ○ |
| 1330 | 113 | | | | 1431 | 131 | ● | | ○ |
| 1331 | 114 | | | | 1438 | 132 | ● | | ○ |
| 1332 | 115 | | | | 1439 | 133 | ○ | | ○ |
| 1333 | 116 | ○ | | ○ | 1440 | 134 | ○ | | ○ |
| 1338 | 117 | | | | 1441 | 135 | ○ | | ○ |
| 1341 | 118 | | | | 1442 | 136 | ○ | | ○ |
| 1342 | 119 | | | | 1447 | 137 | ○ | | ○ |
| 1343 | 120 | | | | 1451 | 138 | ○ | | ○ |
| 1344 | 121 | | | | 1460 | 139 | | | - |

| | | | | | | | | | |
|---|---|---|---|---|---|---|---|---|---|
| 1345 | 122 | ○ | | ○ | 1461 | 140 | ○ | | ○ |
| 1346 | 123 | | | | 1462 | 141 | | | - |
| 1347 | 124 | ○ | | ○ | 1473 | 142 | ○ | | ○ |
| 1413 | 125 | | ○ | - | 1474 | 143 | ○ | | ○ |
| 1414 | 126 | | ○ | - | 1487 | 144 | | ○ | - |
| 1424 | 127 | | | - | 1516 | 9 | ○ | ○ | ○ |
| 1425 | 128 | ○ | | ○ | 1531 | 145 | ○ | | ○ |
| 1426 | 129 | ● | | ○ | 1538 | 146 | | | - |

Table 6 (Continued)

| NK No. | Chemical formula No. | Evaluation A | Evaluation B | Evaluation C | NK No. | Chemical formula No. | Evaluation A | Evaluation B | Evaluation C |
|---|---|---|---|---|---|---|---|---|---|
| 1551 | 147 | ○ | | ○ | 1773 | 165 | | | - |
| 1553 | 148 | ○ | | ○ | 1774 | 166 | | | - |
| 1560 | 149 | | ○ | - | 1776 | 167 | | | - |
| 1567 | 150 | | | - | 1777 | 168 | | | - |
| 1570 | 151 | | ○ | - | 1778 | 169 | | | - |
| 1580 | 152 | ○ | | ○ | 1812 | 170 | | | - |
| 1581 | 153 | ○ | | ○ | 1819 | 171 | | | - |
| 1584 | 154 | ○ | | ○ | 1836 | 172 | | | - |

| | | | | | | | | | |
|---|---|---|---|---|---|---|---|---|---|
| 1585 | 155 | ○ | | ○ | 1837 | 173 | | | - |
| 1590 | 156 | | | - | 1848 | 174 | | | - |
| 1603 | 157 | ○ | | ○ | 1868 | 175 | | | - |
| 1663 | 158 | | | - | 1875 | 176 | | | - |
| 1671 | 159 | | | - | 1878 | 177 | | | - |
| 1684 | 160 | | ○ | - | 1886 | 178 | | | - |
| 1741 | 161 | ○ | | ○ | 1896 | 179 | | | - |
| 1743 | 162 | ○ | | ○ | 1904 | 180 | ○ | | ○ |
| 1744 | 163 | ○ | | ○ | 1906 | 181 | | | - |
| 1745 | 164 | | | - | 1907 | 182 | ● | | ○ |

Table 6 (Continued)

| NK No. | Chemical formula No. | Evaluation A | Evaluation B | Evaluation C | NK No. | Chemical formula No. | Evaluation A | Evaluation B | Evaluation C |
|---|---|---|---|---|---|---|---|---|---|
| 1909 | 183 | | | - | 2014 | 201 | | | - |
| 1910 | 184 | ○ | | ○ | 2039 | 202 | | | - |
| 1911 | 185 | ○ | | ○ | 2045 | 203 | | | - |
| 1912 | 186 | | | - | 2071 | 204 | | | - |
| 1936 | 187 | | | - | 2096 | 205 | ○ | | ○ |
| 1937 | 188 | | | - | 2097 | 206 | | | - |

| | | | | | | | | | |
|---|---|---|---|---|---|---|---|---|---|
| 1938 | 189 | | | - | 2203 | 207 | | | - |
| 1939 | 190 | | | - | 2204 | 208 | | ○ | - |
| 1942 | 191 | | | - | 2251 | 209 | | ○ | - |
| 1951 | 192 | | | - | 2268 | 210 | | ○ | - |
| 1952 | 193 | | | - | 2282 | 211 | | | - |
| 1953 | 194 | | | - | 2409 | 212 | | ○ | - |
| 1954 | 195 | | ○ | - | 2421 | 213 | | | - |
| 1967 | 196 | | | - | 2453 | 214 | | | - |
| 1973 | 197 | | | - | 2545 | 215 | | ○ | - |
| 1977 | 198 | ○ | | ○ | 2610 | 216 | | | - |
| 1979 | 199 | | | - | 2612 | 217 | | | - |
| 1980 | 200 | | | - | 2627 | 218 | | | - |

Table 6 (Continued)

| NK No. | Chemical formula No. | Evaluation A | Evaluation B | Evaluation C | NK No. | Chemical formula No. | Evaluation A | Evaluation B | Evaluation C |
|---|---|---|---|---|---|---|---|---|---|
| 2671 | 219 | | | - | 3206 | 231 | | ○ | - |
| 2674 | 220 | | | - | 3212 | 232 | | | - |
| 2684 | 221 | | | - | 3796 | 233 | | | - |
| 2707 | 222 | | | - | 3972 | 234 | | | - |

| | | | | | | | | | |
|---|---|---|---|---|---|---|---|---|---|
| 2751 | 223 | | | - | 3983 | 235 | | | - |
| 2772 | 224 | | | - | 3986 | 236 | ○ | | ○ |
| 2807 | 225 | | ○ | - | 3989 | 237 | | | - |
| 2825 | 226 | | ○ | - | MH194 | 238 | | ○ | - |
| 2826 | 227 | | ○ | - | MH201 | 239 | | ○ | - |
| 2844 | 228 | | | - | MH1613 | 240 | | | - |
| 2850 | 229 | | | - | AF901 | 241 | | | - |
| 3050 | 230 | | | - | | | | | |

Evaluation A: Cell-growth-promoting action

Evaluation B: Neurite-outgrowth action

Evaluation C: Protective action against cytotoxicity by amyloid β fragment

Blank cell means a case with only lesser effect than the above respective criteria.

-: Not done

As shown in Table 6, it was revealed that the 239 types of compounds exhibit cell-growth-promoting action (Evaluation A) and neurite-outgrowth action (Evaluation B). Specifically, NK-19 (a compound represented by Chemical formula 4), NK-53 (a compound represented by Chemical formula 5), NK-100 (a compound represented by Chemical formula 6), NK-528 (a compound represented by Chemical formula 7), NK-557 (a compound represented by Chemical formula 8), and NK-1516 (a compound represented by Chemical formula 9) exhibited cell-growth-promoting action and neurite-outgrowth action equivalent to or more than those of NK-4. Further, the compounds, which had been evaluated to exhibit cell-growth-promoting action, were subjected to the test for protective action against cytotoxicity by amyloid β fragment (Evaluation C), revealing that they also exhibit the protective action against cytotoxicity by amyloid β fragment. From the above results and those in Experiments 1 and 2, it was revealed that the compounds, exhibiting cell-growth-promoting section and neurite-outgrowth action, shown in Table 6, can be used as anti-neurodegenerative disease agents for humans because they activate neurocytes. Among them, NK-19 (a compound represented by Chemical formula 4), NK-53 (a compound represented by Chemical formula 5), NK-100 (a compound represented by Chemical formula 6), NK-528 (a compound represented by Chemical formula 7), NK-557 (a compound represented by Chemical formula 8), and NK-1516 (a compound represented by Chemical formula 9) are specifically useful as anti-neurodegenerative disease agents for humans because they exhibit strong effects cell-growth-promoting action and neurite-outgrowth action. Furthermore, the compounds, NK-4, NK-19, NK-53, NK-100, NK-528, NK-557 and NK-1516, can be used as an agent for treating neurodegenerative diseases of humans, pathema accompanied by the diseases, and neurofunctional disorders. The compounds, exhibiting the protective action against cytotoxicity by amyloid β fragment, shown in Table 6, are useful as an agent for inhibiting apoptosis.

Experiment 4: Effect of dye concentration on cytotoxic response of cells by amyloid β fragment

In Experiment 3, it was confirmed that NK-19, NK-53, NK-100, NK-528, NK-557 and NK-1516 exhibit protective action against cytotoxicity by amyloid β fragment, similarly as in NK-4. Then, the effect of the above-compounds' concentrations on the cytotoxicity by amyloid β fragment was further investigated. The above 6-compounds and NK-4 were used as test samples, and the protective effects of these compounds against cytotoxicity by amyloid β fragment were evaluated by the same conditions as used in Evaluation C in Experiment 3, except for adding each compound to a well inoculated with PC12-HS cells to give a final concentration as shown in Table 7. The results were expressed by cell survival percentages (%) and shown in Table 7.

Table 7

| Compound | Cell survival percentage (%) | | | | |
|---|---|---|---|---|---|
| | Final concentration of compound (ng/ml) | | | | |
| | 0 | 12.5 | 50 | 200 | 800 |
| NK-4 | 32±1 | 34±1 | 69±7 | 122±32 | 89±4 |
| NK-19 | 27±4 | 71±8 | 102±27 | 58±9 | 38±3 |
| NK-53 | 32±1 | 115±16 | 111±9 | 82±1 | 62±6 |
| NK-100 | 27±4 | 47±2 | 88±12 | 60±5 | 44±4 |
| NK-528 | 33±4 | 40±9 | 36±4 | 50±10 | 111±7 |
| NK-557 | 25±3 | 36±3 | 119±9 | 94±11 | 73±1 |
| NK-1516 | 33±3 | 34±5 | 29±5 | 31±3 | 70±11 |
| Data measured : Average ± SEM | | | | | |

As evident from Table 7, NK-19, NK-53, NK-100 and NK-557 exhibited a higher protective effectagainstcytotoxicity by amyloid β fragment at lesser concentrations than those of NK-4. Among them, NK-53 exhibited higher effect in the lowest concentration and almost completely inhibited the cytotoxicity by amyloid β fragment at a concentration of 12.5 ng/ml (cell survival percentage of 115±16%). NK-19, NK-100 and NK-557 showed maximum cell survival percentage, i.e., 102±27%, 88±12%, and 114±9%, respectively, at a concentration of 50 ng/ml. These values were higher than the cell survival percentage of 69±7% obtained by adding NK-4. NK-53 showed a high protective effect as of a cell survival percentage of 111±9%, even at a concentration of 50 ng/ml. NK-4 showed a maximum cell survival percentage, 122±32%, at a concentration of 200 ng/ml. However, NK-19, NK-53, NK-100, and NK-557 showed decreased protective effects at a concentration of 200 ng/ml. It was confirmed that there exist optimum concentrations for protective effects

against cytotoxicity by these compounds, and it was revealed that the optimum concentrations of NK-19, NK-53, NK-100, and NK-557 are lower than that of NK-4. NK-528 and NK-1516 did not show higher protective effects against cytotoxicity than that of NK-4. From the above results, it was indicated that NK-19, NK-53, NK-100, and NK-557 possibly exhibit advantageous effects on neurodegenerative diseases such as Alzheimer's disease at a lower concentration than that of NK-4. NK-19 and NK-100, which had exhibited higher protective effects against cytotoxicity at lower concentrations than that of NK-4, have larger molecular weights than other compounds. It was judged that NK-19 and NK-53 exhibited higher protective effects against cytotoxicity because they have a relatively higher fat-solubility and cell-membrane-permeability due to their relatively long alkyl-side-chains with a carbon atom number of seven (the remaining compounds have a carbon atom number of two), which each bind to nitrogen atom in their thiazole rings

<u>Experiment 5: Effects of dye compounds on the agglutination of amyloid β peptide</u>

Among the compounds exhibiting the protective effect against the cytotoxicity by amyloid β peptide in Experiments 3 and 4, NK-4, NK-19, NK-53, NK-100, and NK-557, which had been confirmed to have neurite-outgrowth-promoting action, were selected as test samples, and the effects of the compounds on the agglutination of amyloid β peptide, used as a preferable model for developing agents for treating human Alzheimer's disease, were investigated as follows: Each test sample was dissolved in DMSO (Catalog No. "D8418"), commercialized by Sigma-Aldrich Co., MO, USA, to give a concentration of 5 mg/ml, and then filtrated with "MILLEX-LG" (product No. "LLG025SS"), a DMSO-resistant membrane commercialized by Millipore, MA, USA. The filtrate was made into a test sample solution by adjusting the concentration to 200 nM using Tris-HCl buffer.

<u>Method of measuring the agglutination of amyloid β peptide</u>

The agglutination of amyloid β peptide was measured by a method using thioflavine T (see Hilal A. Lashuel et al., Journal of Biological Chemistry, Vol. 277, No. 45, pp. 42881-42890 (2002)). Thioflavine T binds to β-sheet structure of agglutinated amyloid β peptide and produces fluorescence. The amount of the fluorescence was determined using a fluorometric plate reader and used as an index of the agglutination of amyloid β peptide. When the agglutination of amyloid β peptide is inhibited by the test sample, fluorescence of Thioflavine T is decreased. The effects of the test samples on the agglutination of amyloid β peptide were investigated by the method. A human amyloid β peptide, having the amino acid sequence of SEQ ID NO:2 consisting of 40 amino acids, commercialized by AnaSpec Inc., CA, USA, was dissolved in sterilized distilled water to give a concentration of 400 μ M for use. Test samples were diluted with Tris-HCl buffer. In a reaction vessel, 15 μl of 100 mM amyloid β peptide solution was admixed with 45 μl of a 200 nM test sample solution and allowed to react at 37°C for six days. After the reaction, 50 μl of the reaction mixture was withdrawn, admixed with 450 μ 1 of a 10 μM Thioflavine T solution, and after 30 minutes, the resulting fluorescence was measured using a fluorometer (excitation wavelength: 450 nm, adsorption wavelength: 482 nm). The fluorescent intensity of Thioflavine T only was regarded as 0%, and that of the solution, prepared by mixing 15 μl of a 100 mM amyloid β peptide solution and 45 μl of Tris-HCl buffer and allowing to react at 37°C for six days, was regarded as 100%. Inhibition rate (%) of the agglutination of amyloid β peptide was determined by the steps of measuring the relative fluorescence intensity in the case of using each test sample solution for the reaction, and subtracting the measured value from 100%. The results are in Table 8.

<u>Table 8</u>

| Compound | Inhibition rate (%) of agglutination of amyloid β peptide |
|---|---|
| Buffer | 0 |
| NK-4 | 73 |
| NK-19 | 87 |
| NK-53 | 97 |
| NK-100 | 99 |
| NK-557 | 95 |

As evident from Table 8, all the test sample, NK-4, NK-19, NK-53, NK-100, andNK-557wereinhibitedtheagglutination of amyloid β peptide, and NK-19, NK-53, NK-100, and NK-557 exhibited stronger inhibitory activities than that of NK-4. Among them, NK-53, NK-100, andNK-557 inhibited the agglutination of amyloid β peptide in a rate of 95% or higher, and NK-100 inhibited the agglutination almost completely in a rate of 99%. The results

indicated that compounds having protective effect against the cytotoxicity by amyloid β fragment, such as NK-4, NK-19, NK-53, NK-100, and NK-557 also have effects of inhibiting the agglutination of amyloid β peptide, and therefore, they are useful as an agent for preventing Alzheimer's disease as well as for treating the disease.

Experiment 6: Effects of concentrations of dye compounds on cell-growth-promoting action and on neurite-outgrowth action

Among the compounds exhibiting protective effect against cytotoxicity by amyloid β fragment in Experiment 4, NK-19, NK-53, NK-100, and NK-557 were selected and the concentration effects of these compounds on the cell-growth-promotion and the neurite outgrowth of PC12-HS cells were investigated by the same methods as used in Evaluations A and B in Experiment 3. As in the case of Evaluation A in Experiment 3, the effects of the compounds on cell-growth-promotion were evaluated by culturing the cells; admixing with NK-4, NK-19, NK-53, NK-100, or NK-557 to give concentrations, as final concentrations, shown in Table 9 in culturing cells; further culturing the cells; staining the cultured cells with "ALAMAR BLUE"; and measuring the fluorescent intensity of each culture at a wavelength of 544 to 590 nm on a fluorescent plate reader commercialized by Molecular Device Japan, Tokyo, Japan. The relative fluorescent intensity of each well was determined by using the fluorescent intensity of each well with cells, which had been cultured by admixing with D-MEM medium containing 10% by volume of FBS without any of the compounds, and the intensity of the resulting culture was regarded as 100%. The results are expressed as cell survival percentages (%) in Table 9. The effects of the compounds on neurite outgrowth were evaluated by culturing cells by the same method as used in Evaluation B in Experiment 3, admixing with NK-4, NK-19, NK-53, NK-100, or NK-557 to give concentrations, as final concentrations, in Table 10 in culturing cells; further culturing the cells; fixing the cells with glutaraldehyde; observing the fixed cells on a microscope by adjusting the scale factor to give a cell count of about 100 within a perspective; and determining the ratio (%) of cells, which showed neurite outgrowth, to the total cell count. The results are in Table 10.

Table 9

| Compound | Cell survival percentage (%) | | | | |
|---|---|---|---|---|---|
| | Final concentration of compound (ng/ml) | | | | |
| | 0 | 12.5 | 50 | 200 | 800 |
| NK-4 | 100 | 134±8 | 154±8 | 309±68 | 254±54 |
| NK-19 | 100 | 127±34 | 202±12 | 124±5 | 56±1 |
| NK-53 | 100 | 151±18 | 189±19 | 103±6 | 49±3 |
| NK-100 | 100 | 154±156 | 288±19 | 202±4 | 82±15 |
| NK-557 | 100 | 97±25 | 110±6 | 241±48 | 184±42 |
| Data measured : Average ± SD | | | | | |

Table 10

| Compound | Rate of cells exhibiting neurite outgrowth (%) | | | | |
|---|---|---|---|---|---|
| | Final concentration of compound (ng/ml) | | | | |
| | 0 | 200 | 400 | 800 | 1600 |
| NK-4 | 10> | 22±8 | 31±14 | 51±3 | 49±8 |
| NK-19 | 10> | 12±2 | 38±4 | 62±2 | 71±18 |
| NK-53 | 10> | 24±5 | 30±6 | 46±2 | 53±6 |
| NK-100 | 10> | 36±2 | 40±8 | 52±7 | 89±6 |
| NK-557 | 10> | 10> | 20±2 | 43±5 | 84±42 |
| Data measured : Average ± SD | | | | | |

As evident from Table 9, among the compounds added to the media, NK-4 and NK-100 showed a remarkably strong cell-growth-promoting action. Each compound showed an optimum concentration for exhibiting cell-growth-promoting action, and the optimum concentrations of NK-19, NK-53, and NK-100 were 50 ng/ml, while the optimum concentrations of NK-4 and NK-557 were 200 ng/ml. As evident from Table 10, the neurite outgrowth was promoted depending on the concentration of the compounds, and NK-100 showed the strongest cell-growth-promoting action.

Experiment 7: Effect of dye compounds on brain ischemia in animal model rat

From the results in Experiments 1 to 6, it was considered that NK-4, NK-19, NK-53, NK-100, and NK-557 exhibit a therapeutic effect for treating neurodegenerative diseases. Therefore, the effects of these compounds on brain ischemia in animal model rat, which has been used as a preferable model of human cerebral infarction, were investigated by evaluating their behavior and the size of cerebral infarct.

Brain ischemia rat

Male SD rats, seven- to eight-weeks of age and 280 to 330 g body weight, commercialized by Charles River Laboratories Japan Inc., Kanagawa, Japan, were allocated randomly into seven groups, consisting of five to seven rats ineachgroup. Among the seven groups, five groups were subjected to the following surgery as test groups: Rats in the test groups were subcutaneously administered with 0.3 mg/kg body weight of atropine, commercialized by Fuso Pharmaceutical industries, Osaka, Japan. Successively, each rat was anesthetized by administrating 600 mg/kg body weight of urethane, commercialized by Sigma-Aldrich Co. , MO, USA, and 60 mg/kg-weight of $\alpha$-sucralose, commercialized by Sigma-Aldrich Co., MO, USA, into the peritoneal cavity, and fixed to a fixator under voluntary breathing conditions. The right carotid artery bifurcation was exposed by median incision of the neck without damaging vagus nerve. The common carotid artery and external carotid artery around the right carotid artery bifurcation were exfoliated from surrounding connective tissue and wired respectively using "NESCOSUTURE", a 6-0 nylon string commercialized by Alfresa Pharma Corporation, Osaka, Japan. Then, the internal carotid artery was tied with the 6-0 nylon string for fixation after insertion of an obturator. Successively, the common carotid artery was incised and a silicon-coated obturator, prepared by a 4-0 nylon string, commercialized by Doccol Corp., CA, USA, was inserted from the common carotid artery into the internal carotid artery at a distance of about 16 mm, and clipped to the common carotid artery (see, for example, Koizumi et al., Cerebral stroke, Vol. 8, No. 1, pp. 1-8 (1986)). By the method, the silicon-coated tip of obturator was inserted through the right carotid artery bifurcation to frontal cerebral artery at a distance of about 2 mm and obstructs the inlet of the medial cerebral artery. After two hours occlusion of the medial cerebral artery while keeping the rat on an isothermal pad controlled at a temperature of 37°C, blood was allowed to reperfuse by withdrawing the obturator. Then, the internal carotid artery was tied near the carotid artery bifurcation for preventing the bleeding from the incised part of the common carotid artery. In the model, the blood was reperfused through the left carotid artery, vertebral artery, basilar artery, frontal and backward communication artery because the right carotid artery was clipped.

Administration of compounds

NK-4, NK-19, NK-53, NK-100, and NK-557, used in the test, were respectively dissolved in DMSO (product No. "D8418", commercialized by Sigma-Aldrich Co., MO, USA) to give a concentration of 5 mg/ml and filtrated using "MILLEX-LG SLLG025SS", a DMSO-resistant membrane filter commercialized by Millipore, MA, USA. Stock solutions of the compounds were respectively diluted with PBS to give a concentration of 25 ng/ml before use, and then 4 ml/kg body weight of any of the resulting diluted solutions was administered (a dose of 100 $\mu$g/kg body weight of any one of the compounds) to five test groups (test groups 1 to 5) consisting of five or seven rats, through their caudal veins at both after one hour from the occlusion of middle cerebral artery and immediately after initiating reperfusion. After 24 hours from the reperfusion of blood, behavioral and histological evaluations of rats were carried out according to the following procedures: As a control group 1, five rats in one group out of the remaining two groups were subjected to the same surgery as the rats in the test groups 1 to 5, and administered with 4 ml/kg body weight of PBS free of any of the compounds through their caudal veins at both after one hour of the medial cerebral artery occlusion and immediately after the start of reperfusion. As the control group 2, six rats in the remaining one group were subjected to a sham surgery, in which common, external, and internal carotid arteries were clipped and then reperfused their blood without inserting the obturator to each medial cerebral artery. Four ml/kg body weight of PBS, not containing any compound, was also administered to the rats through their caudal veins at both one hour after clipping the common, external, and internal carotid arteries, and immediately after initiating the reperfusion. The rats in the control groups 1 and 2 were also subjected to the behavioral and histological evaluations as in the cases of those in the test groups 1 to 5.

Method for evaluating the effects

Behavioral and histological evaluations

<Behavioral evaluation>

Based on the criteria shown in Table 11, behavioral scores were determined by the steps of evaluating the degree of symptom in each evaluation item and summing the scores for each subject (maximum score: 6) (see, Petullo D. et al., Life Sciences, Vol. 64, No. 13, pp. 1099-1108 (1999)). The results are in Table 12.

<Histological evaluation>

After the behavioral test, each rat was anesthetized with ether and killed by cutting postcava with running physiological saline from left ventricle to remove their blood. Within three minutes after death, their brains were removed, sliced to coronal direction by 2 mm thick using a slicer. Then, each section was incubated in PBS containing 2% (w/v) 2,3,5-triphenyltetrazolium chloride (TTC), which specifically stains a cerebral infarction region, at 37°C for 30 minutes and fixed with 10% by volume of formalin solution for one hour (see, Benderson J. B. et al., Stroke, Vol. 17, pp. 1304-1308 (1986)). The area of cerebral infarction region, stained with TTC, was analyzed using "SCION IMAGE", a free software for image analysis, commercialized by Scion Software, USA, and calculated the volumes of each subject's cerebral infarct and the whole brain thereof. Ratio (%) of each subject's cerebral infarct in the whole brain thereof was calculated by dividing the volume of the cerebral infarct by that of the whole brain and multiplying by 100. Further, the size of cerebral infarct (%) was determined as a relative value calculated by dividing the ratio of the volume of cerebral infarct to the volume of the whole brain in the rats in the test groups 1 to 5 by the ratio of the size of cerebral infarct to the total size of the whole brain of the rats in the control group 1, which is defined as 100%.

Table 11

| Evaluation item | Score | Degree of symptom | Evaluation method |
|---|---|---|---|
| Fore-limb flexion | 0.0 | No flexion | When a rat was held by the tail and fixed in a suspended manner, the degree of palsy was evaluated by the flexion degree of the left fore-limb. |
| | 0.5 | Slight | |
| | 1.0 | Moderate to severe | |
| Torso twisting | 0.0 | No twisting | When a rat was held by the tail and fixed on a flat surface, the torso twisting was evaluated. |
| | 0.5 | Slight | |
| | 1.0 | Moderate to severe | |
| Lateral push | 0.0 | Equal resistance | When a rat was pushed to lateral direction on a |
| | 0.5 | Slight resistance | flat surface, the degree of resistance to the force from the left side was evaluated. |
| | 1.0 | No resistance | |
| Hind-limb Placement | 0.0 | Replaced immediately | When a rat was placed on the edge of a flat surface and allowed its left hind-limb to fall down from the edge, the rapidity for returning to replace the leg on the original surface was evaluated. |
| | 0.5 | Replaced with delay | |
| | 1.0 | No replacement | |
| Motility | 0.0 | Normal | When a rat was placed in a cage, the degree of voluntary movement was evaluated. |
| | 0.5 | Decreased voluntary movement | |
| | 1.0 | No walking without stimulation | |
| | 2.0 | Abasia | |

Table 12

| Test group | Compound | Rats (heads) | Treatment | Behavior al score | Size of cerebral infarcttion region (%) |
|---|---|---|---|---|---|
| Control group 1 | PBS | 5 | Embolus | 5.8±0.1 | 100 |
| Control group 2 | PBS | 6 | Ligature of artery | 0.0 | 0.0 |
| Test group 1 | NK-4 | 7 | Embolus | 2.9±0.2** | 49.9±5.1** |
| Test group 2 | NK-19 | 5 | Embolus | 2.1±0.2** | 30.0±6.0** |
| Test group 3 | NK-53 | 5 | Embolus | 2.5±0.2** | 58.6±6.6* |
| Test group 4 | NK-100 | 5 | Embolus | 2.4±0.2** | 74.5±11.4 |
| Test group 5 | NK-557 | 5 | Embolus | 2.9±0.3** | 61.1±14.2 |
| *, **: There exists a significant difference compared with control group 1 (*: P<0.05, **: P<0.01) | | | | | |

As evident from Table 12, the brains of the rats in the control group 2, received with sham surgery, showed no cerebral infarct, and their behaviors were all normal. Behavioral sore of ischemic rats in the control group 1 were evaluated as 5.8±0.1, and the rats almost lost their mobility at any subtest. On the contrary, in any cases of the rats in the test groups 1 to 5, administered with NK-4, NK-19, NK-53, NK-100, or NK-557, the loss of mobility was significantly inhibited in comparison with the case of the rats in the control group 1. From the viewpoint of the size of cerebral infarct, in any cases of the rats in the test groups 1 to 5, administered with NK-4, NK-19, NK-53, NK-100, or NK-557, the sizes of cerebral infarction region of the rats were smaller than those of the ischemic rats in the control group 1, specifically, the size of cerebral infarct of the rats administered with NK-4, NK-19, and NK-53, in the test groups 1 to 3 were significantly small. Among the compounds, NK-19 exhibited the strongest effect to improve behavioral score and to inhibit the spread of cerebral infarction. The above results indicate that NK-4, NK-19, NK-53, NK-100, and NK-557 have actions of treating neurodegeneration and accompanying impaired nervous functions, caused by ischemia and blood reperfusion.

Experiment 8: Effects of dye compounds on the activity of acetylcholine esterase (AchE)

AchE inhibitors such as donepezil are clinically applied to Alzheimer's dementia. It was reported that AchE inhibitors activate central cholinergic nervous system and improve cognitive function in ischemic cognitive symptoms. Accordingly, the AchE-inhibiting actions of NK-4, NK-19, NK-53, NK-100, and NK-557, which had been confirmed to have improving effect on cerebral infarction of cerebral ischemic and its accompanying impaired nervous function in rats, were investigated. Each stock solution, prepared by dissolving NK-4, NK-19, NK-53, NK-100, or NK-557 into DMSO, was diluted with phosphate buffer and made into a solution containing any of the compounds with a concentration of 10-folds higher than that in Table 13 for use as a test sample solution. PC12-HS Cells, which had been cultured by the same method as in Experiment 1, were harvested and admixed with 5-fold volumes of 10 mM Tris-HCl buffer containing 1 M NaCl, 50 mM $MgCl_2$, and 1% Triton X-100 (pH 7.2). Then, the resulting mixture was homogenized in conventional manner and centrifuged (10,000 x g) at 4°C for 30 minutes. The resulting supernatant was collected as an acetylcholine esterase (AchE) solution. To a well of "μ TEST PLATE for cell culture, flat bottom", a 96-well plate commercialized by Sumitomo Bakelite Co. Ltd., Tokyo, Japan, 30 μl of 50 mM phosphate buffer (pH 8.0), 10 μl of a test sample solution and 10 μl of AchE solution were added, and then 50 μl of a phosphate buffer containing 0.5mMacetyl-thiocholine, commercialized by Wako Pure Chemical Industries, Osaka, Japan, and 1 mM 2-nitro-benzoic acid was further added to the mixture for substrate solutions. Enzyme reaction was carried out in an incubator controlled at 37°C for 30 minutes, and the absorbance at a wavelength of 405 nm ($A_R$) was measured on a plate reader. The absorbance ($A_U$) was measured by the same method as described above except for using 10 μ l of phosphate buffer as a substitute for AchE solution. As a control, the absorbance ($B_R$) was measured by the same method of conducting an enzymatic reaction in an incubator controlled at 37°C for 30 minutes as described above except for using 10 μl of phosphate buffer as a substitute for test

sample solution. The absorbance ($B_U$) was measured by the same method as described above except for using 10 μl of phosphate buffer as substitutes for AchE solution and test sample solution. The residual ratio of AchE activity was determined by the following formula, and the concentration of a compound inhibiting the AchE activity of 50% ($IC_{50}$) was determined:

Formula:

$$\text{Residual ratio of AchE activity (\%) (= [(}A_R - A_U\text{)/(}B_R - B_U\text{)] X 100)}$$

The results are in Table 13.

Table 13

| Compound | Residual ratio of AchE activity (%) | | | | | | |
|---|---|---|---|---|---|---|---|
| | Compound concentration (μg/ml) | | | | | | |
| | 0 | 0.78 | 1.56 | 3.13 | 6.25 | 12.5 | 25 |
| NK-4 | 100 | 84±6 | 66±7 | 42±4* | 37±10** | 38±2** | 30±8** |
| NK-19 | 100 | 96±14 | 94±4 | 97±7 | 79±22 | 80±12 | 70±18* |
| NK-53 | 100 | 114±12 | 85±26 | 81±4 | 77±24 | 85±13 | 65±3** |
| NK-100 | 100 | 77±18* | 68±16** | 67±3** | 60±3** | 49±5* | 43±3** |
| NK-557 | 100 | 110±1 | 107±2 | 97±5 | 97±3 | 97±4 | 88±9 |
| *, ** : There exists a significant difference compared to that with a compound concentration of "O" (*: P<0.05, ** : P<0.01). Data measured : Average ± SD | | | | | | | |

As evident from Table 13, NK-100 showed the strongest AchE-inhibiting activity in the range of lower concentrations, and NK-100 showed a significant AchE-inhibiting activity at a concentration of 0.78 μg/ml or higher. Further, NK-4, having an analogous structure with NK-100, showed a significant inhibitory activity at a concentration of 3.13 μg/ml or higher. The $IC_{50}$ of the NK-4 and NK-100 were respectively 3.3 and 11.8 μg/ml. While, NK-19 and NK-53 showed weak AchE-inhibitory activities, and showed significant inhibitory activities at only a concentration of 25 μg/ml. NK-557 showed almost no AchE-inhibitory activity at a concentration of 25 μg/ml or lower. The above results indicated that the 4 types of compounds, NK-4, NK-19, NK-53, and NK-100, have possibilities of activating cholinergic nervous system and improving Alzheimer's dementia and ischemic cognitive symptom by their AchE-inhibitory activities.

Galanthamine, which has been clinically applied as a therapeutic agent for Alzheimer's disease, shows the same degree of AchE-inhibitory activity at a lower concentration than that of NK-4, and the $IC_{50}$ of Galanthamine is 442 μg/ml. It was recently reported that the compound inhibits apoptosis via PI3K-Akt cascade similarly as in NK-4. However, NK-4, NK-19, NK-53, NK-100, and NK-557 exhibit the inhibitory effects in a concentration of several hundreds ng/ml, but in the cases of Donepezil, Galanthamine, and Tacrine, the order of the concentrations of compounds required for exhibiting the equivalent effects is different. The similar phenomenon is also detected in the case of cell protective effect against in vitro cytotoxicity model by amyloid β fragment. Considering these results, it is indicated that NK-4, NK-19, NK-53, NK-100, and NK-557 can be used as anti-neurodegenerative disease agents having a different action mechanism from the existing therapeutic agents for Alzheimer's disease.

Experiment 9: Effect of dye compounds on free radicals

It has been said that free radicals strongly involve in neuropathy caused by reperfusion of blood after ischemia. Accordingly, radical-scavenging activities of NK-4, NK-19, NK-53, NK-100, and NK-557, which had been confirmed to have an action of improving ischemic neuropathy, were investigated by measuring their scavenging activities for hydroxyl radicals generated from diethylenetriamine-N,N,N',N",N"-penta-acetate (DTPA) by Fenton reaction by using electric spin resonance (hereinafter, abbreviated as "ESR"). Peroxyl radicals are one of in vivo lipid peroxides generated by a reaction of unsaturated fatty acids and hydroxyl radicals. Since the brain enriched in lipids is strongly influenced by peroxyl radicals, peroxyl radicals generated by heating 2,2'-azobis (2-amidinopropane) di-hydrochloride (AAPH) are used as a model of in vivo radicals for investigating the scavenging activities of antioxidative compounds. Therefore, the peroxyl radical-scavenging activities of NK-4, NK-19, NK-53, NK-100, and NK-557 were investigated by measuring the scavenging activities for peroxyl radicals generated from AAPH by ESR.

<Measurement of hydroxyl radical-scavenging activity>

Stock solutions, prepared by dissolving NK-4, NK-19, NK-53, NK-100, and NK-557 in DMSO, were respectively diluted with refined water to give concentrations of 3-folds higher than those in Table 14 and used as test sample solutions. As a positive control, a neuroprotectant, "edaravone", commercialized as "RADICUT®" containing 3-methyl-1-phenyl-2-pyrazoline-5-one as an effective ingredient by Mitsubishi Tanabe Pharma Corporation, Osaka, Japan, was diluted with refined water to give a concentration of 3-folds higher than that in Table 14 for use. To 50 μl of refined water, 50 μl of 89 mM DMPO (5,5-dimethyl-1-proline-oxide) commercialized by Dojindo Laboratories, Kumamoto, Japan, 50 μl of the test sample solution, and 50 μl of an aqueous solution containing 1 mM hydrogen peroxide, 1 mM $FeSO_4$, and 1 mM DTPA, commercialized by Wako Pure Chemical Industries, Osaka, Japan, were added, mixed for 10 seconds using a vortex mixer and allowed to react in an incubator controlled at 37° for 40 seconds. After 30 seconds from completion of the reaction, the resulting reaction mixture was subjected to ESR measurement. A control solution was prepared by the same method as above except for using 50 μl of a diluted edaravone solution as a substitute for the test sample solution, and subjected to ESR measurement.

<Measurement of peroxyl radical-scavenging activity>

Stock solutions, prepared by dissolving NK-4, NK-19, NK-53, NK-100, and NK-557 in DMSO, were respectively diluted with 0.1 M phosphate buffer to give concentrations of 3-folds higher than those in Table 15 and used as test sample solutions. To 50 μl of 0.1 M phosphate buffer (pH 7.4), 50 μl of 180 mM DMPO (5,5-dimethyl-1-proline-oxide) commercialized by Dojindo Laboratories, Kumamoto, Japan, 50 μl of any one of the test sample solutions, and 50 μl of 100 mM AAPH, commercialized by Wako Pure Chemical Industries, Osaka, Japan, were added, mixed for 10 seconds using a vortex mixer and allowed to react in an incubator controlled at 37° for two minutes and 50 seconds. After 30 seconds from completion of the reaction, the resulting reaction mixture was subjected to ESR measurement.

<Measurement of ESR>

The reaction mixture for measuring hydroxyl or peroxyl radicals was poured into a flat quartz cell for ESR, set to "FREE RADICAL MONITOR JES-FR30", an ESR apparatus commercialized by JOEL Ltd., Tokyo, Japan, and ESR was measured according to manual manner. The residual ratio of peroxyl radical (%) was determined as a relative value by defining the ESR value, obtained by using 0.1M phosphate buffer as a substitute of the test sample solution, as 100. The results are in Tables 14 and 15. Further, based on the results, the $IC_{50}$ of radical-scavenging activity of each compound was determined and also shown in Tables 14 and 15. In the case using edaravone as a positive control for measuring hydroxyl radical-scavenging activity, it showed no radical-scavenging activity at a concentration of 25 μg/ml or lower. Therefore, the $IC_{50}$ of edaravone was determined by another test using its higher concentration, and the result is also shown in Table 14. The conditions of the ESR measurement were set as follows:

<Measuring conditions>

| | |
|---|---|
| Power | : 4 mW |
| Magnetic field | : 335.5 mT |
| Sweep time | : 2 minutes |
| Modulation width | : 0.079 mT |
| Amplitude | : 79 (for hydroxyl radicals) |
| | : 125 (for peroxyl radicals) |
| Time constant | : 0.1 second |
| Accum | : 1 |

Table 14

| Compound | Residual ratio of hydroxyl radical (%) | | | | | $IC_{50}$ ($\mu$g/ml) |
|---|---|---|---|---|---|---|
| | Concentration of compound ($\mu$g/ml) | | | | | |
| | 0 | 3.13 | 6.25 | 12.5 | 2.5 | |
| NK-4 | 100 | 67±1** | 49±2** | 26±1** | 15±1** | 6.7 |
| NK-19 | 100 | 68±2** | 49±1** | 27±1** | 8±1** | 6.8 |
| NK-53 | 100 | 73±7** | 49±7** | 30±2** | 7±0** | 6.2 |
| NK-100 | 100 | 68±1** | 51±1** | 32±1** | 8±0** | 6.6 |
| NK-557 | 100 | 71±1** | 50±1** | 30±1** | 15±6** | 6.2 |
| Edaravone (Positive control) | 100 | 100 | 100 | 100 | 100 | 148.0 |
| *, ** : Significantly different compared to that with a compound concentration of zero (*: P<0.05), **: P<0.01)<br>Data measured: Average ± SD | | | | | | |

Table 15

| Compound | Residual ratio of peroxyl radical (%) | | | | $IC_{50}$ ($\mu$g/ml) |
|---|---|---|---|---|---|
| | Compound concentration ($\mu$g/ml) | | | | |
| | 0 | 5 | 25 | 50 | |
| NK-4 | 100 | 46±9** | 17±1** | 16±0** | 4.7 |
| NK-19 | 100 | 62±7** | 54±6** | 33±2** | 26.4 |
| NK-53 | 100 | 81±9* | 58±0** | 33±7** | 33.2 |
| NK-100 | 100 | 86±15 | 86±15 | 76±7** | 50< |
| NK-557 | 100 | 90±9 | 58±5** | 66±8** | 50< |
| *, ** : Significantly different compared to that with a compound concentration of zero (*: P<0.05), **: P<0.01)<br>Data measured: Average ± SD | | | | | |

As evident from Table 14, the residual ratios of hydroxyl radical were lowered depending on the concentration of each compound in the test sample solution, and it was confirmed that all the compounds exhibited a hydroxyl radical-scavenging activity. The $IC_{50}$ of hydroxyl radical-scavenging activity of any of NK-4, NK-19, NK-53, NK-100, and NK-557 was about 6 μ g/ml, while the $IC_{50}$ of edaravone, as a commercially available neuroprotectant with the action mechanism of scavenging free radicals, was 148 μg/ml. It was revealed that NK-4, NK-19, NK-53, NK-100, and NK-557 have advantageous hydroxyl radical-scavenging activities than the commercially available free radical-scavenging agent for neuroprotection. The $IC_{50}$ of hydroxyl radical-scavenging activity of NK-9694 and NK-150 were respectively 3.4 4 μg/ml and 1.8 μg/ml. As evident from Table 15, the residual ratios of peroxyl radical were lowered depending on the concentration of compounds in the test sample solution, and it was confirmed that all the compounds exhibited a peroxyl radical-scavenging activity. Specifically, NK-4 exhibited a relatively strong radical-scavenging activity being almost equivalent to that of sodium ascorbate (AsA-Na) at the same concentration (data not shown). Further, NK-19 and NK-53 exhibited a relatively strong activity and showed a significant radical-scavenging activity at a higher concentration of 5 μ g/ml or higher. NK-557 and NK-100 significantly scavenged peroxyl radicals at concentrations of 25 μg/ml or higher and 50 μg/ml or higher, respectively. From the above results, it was revealed that the 5-types of compounds used in the test exhibit a strong hydroxyl radical-scavenging activity and the 3-types of compounds, NK-4, NK-19, and NK-53 exhibit a peroxyl radical-scavenging activity at relatively lower concentrations. Therefore, it was considered that the hydroxyl radical-scavenging activities and the peroxyl radical-scavenging activities of these compounds are involved in one of the major action mechanisms of the effect of reducing infarction of brain infraction model rats. Actually, in Experiment 7, relatively strong effects for reducing infarction were detected in the groups administered with NK-4, NK-19, and NK-53 than those administered with NK-557 and NK-100. Therefore, these compounds exhibit the effect of inhibiting the death of neurocytes by inhibiting the oxidative stress by hydroxyl radicals and/or peroxyl radicals, generated by reperfusion of blood in ischemic diseases, and they can be advantageously used as anti-neurodegenerative disease agent, neuroprotectant, radical scavenger, inhibitor for oxidative stress, agent for inhibiting the production of lipidperoxide, agent for inhibiting oxidative disorder of brain, etc.

Edaravone, used as a positive control, has a free radical-scavenging activity (free radical scavenger) and inhibits the hydroxyl radical formation after the reperfusion of blood in the brain of ischemia model rat. Further, it is known that edaravone exhibits the effects of inhibiting the spread of cerebral infarction, the decrease of blood flow in the region around cerebral infarction, the cerebral edema, and the delayed neurocyte death (see, for example, Nihon-Yakurigaku-Zasshi, Vol. 119, pp. 301-308 (2002)). Correspondingly, it was revealed that the anti-neurodegenerative disease agent of the present invention has a stronger hydroxyl radical-scavenging activity than that of edaravone, and exhibits an action of inhibiting the spread of cerebral infarction and the neurocyte death. Therefore, the anti-neurodegenerative disease agent of the present invention can be advantageously used as a neuroprotectant, inhibitor for ischemic cerebral disorder, inhibitor for spreading cerebral infarction, inhibitor for cerebral edema, and inhibitor for the delayed neurocyte death, equivalently with edaravone or more advantageously. It is known that 3-methyl-1-phenyl-2-pyrazoline-5-one and its analogues, effective ingredients of edaravone, can be used as the following agents by applying the free radical-scavenging activity and lipid peroxide formation-inhibiting activity: brain function normalizing agent (Japanese Patent Kokoku No. 31523/93); inhibitor for the formation of lipid peroxide (Japanese Patent Kokoku No. 35128/93); anti-ulcer agent (Japanese Patent No. 2906512); inhibitor for hyperglycemia (Japanese Patent No. 2906513); agent for preventing or treating eye diseases such as cataract and corneous disorder (Japanese Patent Kokai No. 25765/95); agent for preserving transplanted organs (Japanese Patent Kokai No. 52801/97, WO 03/67979) ; necrosis-inhibitor for transplanted tissues (including skin) and organs (Japanese Patent Kokai No. 79991/99); agent for preventing or treating disorders of various organs such as kidney disorders caused by acuterenalfailure, skin tissue disorder, lungdisorder, hepatic disorder caused by liver fibrosis, chemical substances, endotoxin, ischemia; skin tissue disorder caused by hot water, cord injury, vascular disorder of blood vessel in the brain and artery, muscular disorder of cardiac muscle, tubulointestinal disease, and their accompanying functional disorders (Japanese Patent Kokai No. 52831/97, Japanese Patent Kokai No. 2004-99560, Japanese Patent Kokai No. 279480/98, Japanese Patent Kokai No. 2004-131402, Japanese Patent Kokai No. 2006-96664, Japanese Patent Kokai No. 2004-123716, Japanese Patent Kokai No. 2004-2381, JapanesePatentKokaiNo. 2004-115508, WO 03/105909, WO 04/13107, Japanese Patent Kokai No. 2004-67585, Japanese Patent Kokai No. 2004-115505, Japanese Patent Kokai No. 2008-509879, WO 03/66051, WO 03/80583, Japanese Patent Kokai No. 2006-182677); agent for preventing or treating radiation damage (Japanese Patent Kokai No. 2003-335674); anti-tumor agent and tumor metastasis-inhibitory agent (Japanese Patent Kokai Nos. 2004-277315 and 2005-29573); inhibitor for cell injury markers (Japanese Patent Kokai No. 2004-137252); agent for preventing or treating inflammatory diseases of various tissues and organs such as myocarditis, pancreas inflammation, inflammation of the intestine, arthritis, and allergy (Japanese Patent Kokai Nos. 2004-137253 and 2004-143149, WO 04/22543, WO 05/12255); inhibitor for disorders of sensory cells, sensory nerve and sensory organs such as visual cell disorder, optic nerve disorder, retinal disease, acoustic cell disorder, and auditory nerve disorder (WO 02/260, Japanese Patent Kokai Nos. 2003-252760, 2004-123713, and 2004-137256); agent for preventing or treating chemical addiction such as paraquat poisoning (Japanese Patent Kokai No. 2004-161720); inhibitor for Na-Ca exchange system (JapanesePatentKokaiNo. 2004-115511); inhibitor for oxidative stress (WO 03/24446); agent for treating pain and pruritus (Japanese Patent Kokai Nos. 2004-331653 and 2008-37753); Protein-kinase-stimulating agent (Japanese Patent Kokai No. 2004-339214); agent for preventing or treating mitochondrial encephalomyopathy (Japanese Patent Kokai No. 2005-89456); agent for preventing or treating occlusion and arctation of arteria (Japanese Patent Kokai No. 2005-162749); inhibitor for the failure of blood-brain barrier (WO 04/63167) ; agent for treating drug dependency; (Japanese Patent Kokai No. 2008-247813); and apoptosis inhibitor (Japanese Patent Kokai No. 2003-300880). Therefore, the anti-neurodegenerative disease agent of the present invention can be advantageously used as an agent for preventing or treating the above-identified diseases, since the agent exhibits an effect equivalent to or more advantageous than edaravone.

Experiment 10: Neurotrophic factor-like activity of NK-19 analogues

Since NK-19 was proved to have a strong neurodegenerative inhibitory effect in the above experiments, an experiment to verify whether NK-19 analogues have a similar effect was conducted. Twelve types of compounds represented by the following General formula 3, having a side-chain alkyl group ($R_7$ to $R_9$) with a carbon atom number of 1 to 12 and $I^-$ or $Cl^-$ as a counter anion, were synthesized (synthesized by Hayashibara Biochemical Laboratories, Inc., Okayama, Japan), and their intensities of enhancing effects on cell growth and neurite outgrowth of PC12-HS cells were determined by the same method as in Experiment 3. Twelve types of compounds, NK-19 analogues including NK-19 shown in Table 16, were respectively dissolved in DMSO at a concentration of 5 mg/ml. Each solution was diluted with D-MEM medium supplemented with 10 (v/v) % of FBS to give test solutions at a concentration of 100 ng/ml or 2 μg/ml.

For NK-24 and NK-19, the compounds, whose counter anions $I^-$ were replaced with $Cl^-$ (NK-56 and NK-53), were respectively dissolved in DMSO at a concentration of 5 mg/ml. Each solution was diluted with D-MEM medium supplemented with 10 (v/v) % of FBS to give test solutions with a concentration of 100 ng/ml or 2 μg/ml of any of the compounds.

Evaluation of the action of enhancing cell growth

Similarly as the evaluation method A as in Experiment 3, the cells were diluted with D-MEM medium supplemented with 10 v/v % FBS to give a cell density of 5 x $10^3$/well and the resulting dilute was inoculated to 96-well plates coated with collagen in an amount of 100 μl/well. After 24 hours, the cell cultures were diluted with D-MEM medium supplemented with 10 v/v % FBS, admixed with any of the above test solutions at a concentration of 100 ng/ml of each compound in an amount of 100 μl/well, and then cultured at 37°C for three days in a 5 v/v % $CO_2$ incubator. After three days of culturing, the supernatant was removed, 10% by weight of ALAMAR BLUE (Trek Diagnostic)/D-MEM medium supplemented with 10 v/v % FBS was added in an amount of 200 μl/well, cultured at 37°C for six hours in a 5 v/v % $CO_2$ incubator, followed by measuring the fluorescence intensity at a wavelength of 544 to 590 nm was measured on a fluorescent plate reader (Molecular Device Corporation Japan). The action of enhancing cell growth when admixed with each test solution was represented by a relative intensity to the intensity of control, which had been admixed with D-MEM medium supplemented with 10 v/v % FBS by 100 μl/well, being regarded as 100. The results were shown in Table 16. The test was conducted twice for each test sample in a triplicate manner and the data were averaged.

<Evaluation of the action of enhancing neurite outgrowth>

Similarly as the evaluation method B in Experiment 3, PC12-HS cells were diluted with D-MEM medium supplemented with 10 v/v % FBS to give a cell density of 5 x $10^3$/well, and the resulting dilute was inoculated to 96-well plates coated with collagen in an amount of 100 μl/well. After 24 hours, the cell cultures were admixed with any of the test solutions with a concentration of 2 μg/ml of each compound by 50 μl/well and NGF (a final concentration of 5 ng/ml) by 50 μl/well, cultured for three days, and then fixed with 10 v/v % of glutaraldehyde at ambient temperature for 20 minutes. As a control, cells were cultured with D-MEM medium supplemented with 10 v/v % FBS alone for three days and fixed with glutaraldehyde. The action of each compound on the enhancement of neurite outgrowth of the cells was evaluated by microscopically observing the fixed cells by the same method as in Experiment 3. The results were shown in Table 16. The data were obtained by averaging those from twice tests conducted in a triplet manner for each test solution, except for those of NK-56 and NK-53. The neurite outgrowth percentage was about five percent when NGF was added alone (5 ng/ml) in the above experiment.

General formula 3:

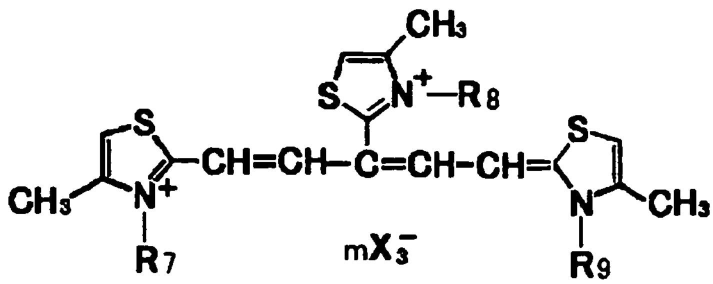

In General formula 3, $R_7$ to $R_9$ are the same or different aliphatic hydrocarbon groups. $X_3^-$ is an adequate anion, and m is 1 or 2 to give a balanced charge with cation.

Table 16

| Compound | Carbon atom number of alkyl chain ($R_7$ to $R_9$) | Counter anion | Action of enhancing cell growth (%) | Percentage (%) of cells with neurite outgrowth |
|---|---|---|---|---|
| NK-2 | 1 | $I^-$ | 126 | 4 |
| NK-13 | 2 | $I^-$ | 125 | 5 |

(continued)

| Compound | Carbon atom number of alkyl chain ($R_7$ to $R_9$) | Counter anion | Action of enhancing cell growth (%) | Percentage (%) of cells with neurite outgrowth |
|---|---|---|---|---|
| NK-237 | 3 | $I^-$ | 212 | 16 |
| NK-24 | 4 | $I^-$ | 581 | 33 |
| NK-56* | 4 | $Cl^-$ | - | 28 |
| NK-2850 | 5 | $I^-$ | 553 | 56 |
| NK-392 | 6 | $I^-$ | 560 | 44 |
| NK-19 | 7 | $I^-$ | 554 | 64 |
| NK-53** | 7 | $Cl^-$ | - | 70 |
| NK-150 | 8 | $I^-$ | 601 | 78 |
| NK-393 | 9 | $I^-$ | 550 | 68 |
| NK-2844 | 10 | $I^-$ | 399 | 54 |
| NK-9640 | 11 | $I^-$ | 191 | 13 |
| NK-32 | 12 | $I^-$ | 199 | 16 |
| *: Exchanging $I^-$, as a counter anion of NK-24, for $Cl^-$<br>**: Exchanging $I^-$, as a counter anion of NK-19, for $Cl^-$<br>-: Not tested | | | | |

As evident from Table 16, the actions of enhancing cell growth and neurite outgrowth were observed, when the carbon number of alkyl group in side chain is 3 to 12, and the actions were enhanced when the carbon atom number is 3 to 10. The action of enhancing cell growth is the highest when the carbon atom number is 4 to 9, and that of enhancing neurite outgrowth is the highest when the carbon atom number is 5 to 10. Since there is no difference in neurite outgrowth between NK-24 and NK-56 or between NK-19 and NK-53, the neurotrophic factor-like activities of NK-19 analogues are not varied independently of their counter anions.

The above results indicate that, since the compounds represented by General formula 3 including NK-19, NK-53 and NK-150 with carbon atom number of 3 to 10 of alkyl group in side chain, particularly, the compounds represented by General formula 6 with carbon atom number of 3 to 10 of alkyl group in side chain have physiological activities such as neurotrophic factor-like activity and neurodegeneration suppressing activity, these compounds are usable as an anti-neurodegenerative disease agent for Alzheimer's disease or cerebella ataxia.

Experiment 11: Neurotrophic factor-like activity of NK-4 analogues

Similarly as in Experiment 10, to verify whether NK-4 analogues, i.e., seven compounds represented by NK-4 analogues have a similar action and effect, the following compounds represented by General formula 2, in which the carbon atom number of the alkyl group ($R_4$ to $R_6$) of the side chain is 2 to 8 and which has $I^-$ as a counter anion, were synthesized and examined their actions of enhancing cell growth and neurite outgrowth of PC12-HS cells by the same method as in Experiment 3. Seven compounds as in Table 17, NK-234, NK-26, NK-9815, NK-9694, NK-28 and NK-147 as well as NK-4, were each dissolved in DMSO to give a concentration of 5 mg/ml. Each solution was diluted with D-MEM medium supplemented with 10 v/v % FBS to give test solutions having the concentrations shown in Table 17 or 18. NK-13, NK-392, NK-19 and NK-150, as NK-19 analogues, were each dissolved in DMSO to give a concentration of 5 mg/ml, and diluted with D-MEM medium supplemented with 10 v/v % FBS to give test solutions having the concentrations shown in Tables 17 or 18. The test was conducted twice for each test sample in a triplicate manner and the data were averaged. The results of the action of enhancing cell growth are shown in Table 17, and those of the action of enhancing neurite outgrowth are shown in Table 18.

General formula 2:

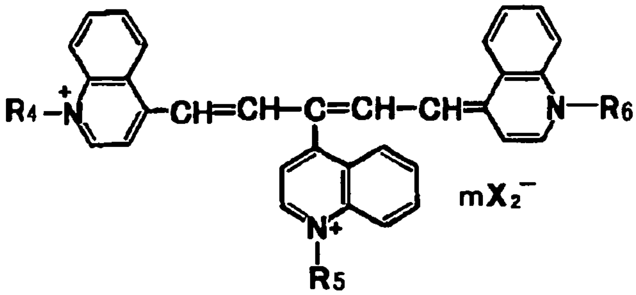

In General formula 2, $R_4$ to $R_6$ are the same or different aliphatic hydrocarbon groups. $X_2^-$ is an adequate anion, and m is 1 or 2 to give a balanced charge with the cation part.

Table 17

| Compound | | Carbon atom number of alkyl chain (R) | Action of enhancing cell growth (%) | | | | |
|---|---|---|---|---|---|---|---|
| | | | Compound concentration (ng/ml) | | | | |
| | | | 0 | 25 | 50 | 100 | 200 |
| NK-4 Analogue | NK-4 | 2 | 100 | 149±20 | 181 ± 79 | 301 ± 65 | 438 ± 48 |
| | NK-234 | 3 | 100 | 168±45 | - | - | - |
| | NK-26 | 4 | 100 | 275±19 | 357 ± 35 | 210 ± 12 | 210 ± 12 |
| | NK-9815 | 5 | 100 | 304±26 | - | - | - |
| | NK-9694 | 6 | 100 | 269±17 | 123 ± 88 | 402 ±111 | 320 ± 36 |
| | NK-28 | 7 | 100 | 146±10 | 111 ± 28 | 252 ± 65 | 470 ± 17 |
| | NK-147 | 8 | 100 | 122±14 | 136 ± 30 | 16 3± 60 | 404 ± 76 |
| NK-19 Analogue | NK-13 | 2 | 100 | 126±12 | 140 ± 7 | 231 ± 84 | 209 ± 24 |
| | NK-392 | 6 | 100 | 349±63 | 601 ± 93 | 607 ± 71 | 460 ± 41 |
| | NK-19 | 7 | 100 | 245±61 | 550 ± 118 | 597 ±147 | 551 ± 143 |
| | NK-150 | 8 | 100 | 153±28 | 263 ± 48 | 534 ± 52 | 606 ± 79 |
| Data measured : Average ± SD<br>-: Not measured | | | | | | | |

Table 18

| Compound | | Carbon atom number of alkyl chain (R) | Action on neurite outgrowth (%) | | | | |
|---|---|---|---|---|---|---|---|
| | | | Compound concentration of(ng/ml) | | | | |
| | | | 0 | 80 | 400 | 2000 | 10000 |
| NK-4 Analogue | NK-4 | 2 | 10< | 25 ± 9 | 35 ± 9 | 52 ± 4 | 14 ± 6 |
| | NK-234 | 3 | 10< | 24 ± 7 | 57 ± 7 | 61 ± 3 | 66 ± 20 |
| | NK-26 | 4 | 10< | 72 ± 4 | 71 ± 7 | 25 ± 7 | 10< |
| | NK-9815 | 5 | 10< | 68 ± 12 | 62 ± 12 | 56 ± 2 | 11 ± 2 |
| | NK-9694 | 6 | 10< | 45 ± 8 | 55 ± 14 | 38 ± 12 | 10< |
| | NK-28 | 7 | 10< | 38 ± 1 | 64 ± 10 | 70 ± 11 | 46 ± 20 |
| | NK-147 | 8 | 10< | 32 ± 7 | 50 ± 5 | 70 ± 11 | 35 ± 14 |
| NK-19 Analogue | NK-13 | 2 | 10< | 10< | 10< | 10< | 10< |
| | NK-392 | 6 | 10< | 44 ± 12 | 48 ± 2 | 76 ± 8 | 81 ± 10 |
| | NK-19 | 7 | 10< | 32 ± 2 | 61 ± 8 | 81 ± 16 | 69 ± 15 |
| | NK-150 | 8 | 10< | 49 ± 9 | 68 ± 19 | 87 ± 7 | 39 ± 11 |
| Data measured : Average ± SD<br>-: Not measured | | | | | | | |

As shown in Tables 17 and 18, among the NK-4 analogues represented by General formula 2, the compounds, having carbon atom number of three to eight for alkyl group of side chain, had relatively high actions of enhancing cell growth and neurite outgrowth comparable to those of NK-4. When compared at a concentration of 80 ng/ml, the compounds, having carbon atom number of four to six for alkyl group of side chain, had relatively high actions of enhancing cell growth; and the compounds, having carbon atom number of four to five for alkyl group of side chain, had relatively high actions of enhancing neurite outgrowth. NK-19 analogues represented by General Formula 3, having carbon atom number of two to eight, particularly, six to eight for alkyl group of side chain, had relatively high actions of enhancing cell growth and neurite outgrowth.

Experiment 12: Effect of NK-4 analogues and NK-19 analogues on cytotoxicity by 6-hydroxy dopa

Since it was revealed that NK-4 analogues and NK-19 analogues have relatively high actions of enhancing cell growth and neurite outgrowth, the influence of these compounds on cell damage was investigated in this Experiment. 6-Hydroxy dopa was used as a cytotoxic agent. Similarly as in Experiment 1, PC12-HS cells, which had been cultured in D-MEM medium supplemented with 10 v/v % FBS, were inoculated to 96-well microplates by $2 \times 10^4$/100 μl/well. After 24 hours, the resultant cell suspension was admixed with 50 μl of NK-4, NK-9694, NK-19 or NK-150, which had been diluted with D-MEM medium supplemented with 10 v/v % FBS to give a 2-fold concentration of a final concentration, and 50 μl of 400 μM of 6-hydroxy dopa; and cultured in a 5 v/v % $CO_2$ incubator at 37°C for 24 hours. Thereafter, the cells were fixed with 10% glutaraldehyde and the absorbance at 650 nm was measured by conventional dye-uptake method using methylene blue. Using the count of cells (absorbance), as a control, which had been cultured for 24 hours in D-MEM medium supplemented with 10 v/v % FBS, the cell survival percentage (%) was obtained as a relative cell count (absorbance) for each well when the cell count (absorbance) of control is regarded as 100%. The results are in Table 19.

Table 19

| Compound | | Carbon atom number of alkyl chain ($R_4$ to $R_6$) | Survival-cell percentage (%) Concentration of compound (ng/ml) | | | | | | |
|---|---|---|---|---|---|---|---|---|---|
| | | | 0 | 0.83 | 3.3 | 13 | 50 | 200 | 800 |
| NK-4 analogue | NK-4 | 2 | 55 ± 9 | 59 ± 3 | 65 ± 4 | 69 ± 6* | 70 ± 6* | 76 ± 3* | 92 ± 4* |
| | NK-9694 | 6 | 55 ± 9 | 62 ± 1 | 68 ± 2* | 67 ± 0* | 62 ± 3 | 47 ± 1 | 42 ± 1 |
| NK-19 analogue | NK-19 | 7 | 55 ± 9 | 70±10* | 74 ± 5* | 68 ± 4* | 68 ± 3* | 71 ± 2* | 57 ± 1 |
| | NK-150 | 8 | 54 ± 4 | 54 ± 1 | 58 ± 4 | 68 ± 9 | 76 ± 19 | 84 ± 23 | 88 ± 19* |
| *, **: Significantly different from APP Tg mouse administered with physiological saline (*: $P < 0.05$, **: $P < 0.01$) Data measured : Average ± SD | | | | | | | | | |

As evident from Table 19, NK-4, NK-9694, NK-19 and NK-150 significantly suppressed cytotoxicity by 6 -hydroxy dopa. Since 6-hydroxy dopa is a catecholaminergic neurocyte selective neurotoxin used as an in vivo or in vitro model of Parkinson's disease, these results indicate that NK-4 analogues and NK-19 analogues can be used as a therapeutic of Parkinson's disease. Since under the experimental conditions, these compounds exhibit a suppressing action at a concentration of 1 μg/ml or lower, at which commercially available edaravone or donepezil can not suppress the cytotoxicity, andthisconcludedthatNK-4, NK-9694, NK-19 and NK-150 more strongly suppress the cytotoxicity of 6-hydroxy dopa than edaravone or donepezil.

Though concrete data is not shown, using primary neuron (neuron, astrocyte and microglia) prepared from brain cortex of a rat fetus in place of PC12-HS cells, the influence of NK-4, NK-26, NK-234, NK-19 and NK-150 on amyloid β fragment disorder and hydrogen peroxide disorder was investigated. As a result, it was found that these compounds have actions of suppressing amyloid β fragment disorder and hydrogen peroxide disorder. It was also found that these compounds have actions of suppressing NO production from microglia in the presence of LPS.

Experiment 13: Effect of NK-4 analogues and NK-19 analogues on cerebral-infarction-model rat

Effect of NK-4, NK-26 and NK-15, which had been confirmed to have actions of enhancing cell growth and neurite outgrowth, on cerebral infarction were investigated using a human cerebral-infarction-model rat. According to Experiment 7, SD rats (male, eight-weeks of age, body weight of 280 to 330 g, commercialized by Charles Liver Laboratories Japan Inc., Kanagawa, Japan), were embolized their cerebral arteries. NK-150, NK-26 and NK-4 were each dissolved in DMSO at a concentration of 5 mg/ml, filtrated with a 0.45 μm-pore sized membrane filter, diluted with DMSO to give a concentration of 2.5 to 0.05 mg/ml, and stored under light shielded conditions. The solutions of the compounds were diluted with physiological saline 250-folds just before use, and administered to the embolized rat at one hour after the embolus and at the disobliteration, through caudal vein (volume: 5ml/kg body weight). NK-4 was prepared into a solution with a concentration of 10mg/ml, diluted by 250 to 167-folds, and administered through caudal vein (volume: 5 ml/kg body weight). Saline was administered according to the schedule of NK-4 as a negative control, and conventional edaravone ("RADICUT" commercialized by Mitsubishi Tanabe Pharma Corporation, Tokyo, Japan) was diluted with DMSO and intravenously administered to the rat as a positive control. By the same method as in Experiment 7, the nervous symptoms were diagnosed by behavioral score. The volume ($mm^3$) of cerebral infarction region was calculated by dividing actual infarction volume by tumefaction ratio, which was preliminary calculated by dividing ischemic hemicerebrum volume by healthy hemicerebrum volume, to circumvent the influence of edema brought by infarction. The results and the groups are in Table 20.

Table 20

| Administered compound | Dose (per kg body weight) | Rat (head) | Treatment | Behaviora 1 score | Volume of cerebral infarction region ($mm^3$) |
|---|---|---|---|---|---|
| Physiologica 1 saline | - | 8 | Embolus | 5.1 ± 0.3 | 205 ± 14 |

(continued)

| Administered compound | Dose (per kg body weight) | Rat (head) | Treatment | Behaviora 1 score | Volume of cerebral infarction region (mm$^3$) |
|---|---|---|---|---|---|
| Edaravone | 0.3 mg | 8 | Embolus | 4.7 ± 0.4 | 179 ± 27 |
| | 1 mg | 8 | Embolus | 3.7 ± 0.5* | 152 ± 28 |
| | 3 mg | 8 | Embolus | 4.8 ± 0.2 | 183 ± 16 |
| NK-4 | 25 μg | 5 | Embolus | 4.5 ± 0.6 | 186 ± 21 |
| | 50 μg | 5 | Embolus | 3.3 ± 0.4** | 107 ± 21* |
| | 100 μg | 5 | Embolus | 2.6 ± 0.2** | 88 ± 18** |
| | 200 μg | 5 | Embolus | 3.7 ± 0.4 | 143 ± 26 |
| | 300 μg | 5 | Embolus | 4.2 ± 0.5 | 176 ± 31 |
| NK-26 | 1 μg | 8 | Embolus | 4.4 ± 0.4 | 194 ± 22 |
| | 10 μg | 8 | Embolus | 3.44 ± 0.6** | 121 ± 22* |
| | 100 μg | 8 | Embolus | 4.3 ± 0.4 | 169 ± 23 |
| NK-150 | 10 μg | 8 | Embolus | 3.7 ± 0.4 | 179 ± 21 |
| | 100 μg | 8 | Embolus | 3.7 ± 0.4* | 111 ± 15** |
| *, ** : Significantly different from rat administered with physiological saline (* : P < 0 . 05, ** : P < 0.01)<br>Data measured : Average ± SD | | | | | |

As evident from Table 20, in the rats administered with NK-4, NK-26 and NK-150, the behavioral scores were significantly improved and the increments of the volume of cerebral infarction region were significantly suppressed, although their optimum doses were different. When edaravone was administered, the behavioral score was significantly improved but the increment of the volume of cerebral infarction region was not significantly suppressed. These results indicate that NK-4, NK-26 and NK-150 have a strong action of improving ataxia by suppressing the increment of the volume of cerebral infarction region. It was found that, in this Experiment system, NK-4, NK-26 and NK-150 have a stronger action of improving ataxia accompanied by cerebral infarction than commercialized edaravone.

Experiment 14: Comparison of inhibitory action of NK-4 analogues on AchE activity

As described above, AchE-activity inhibitory agents are used as a therapeutic agent for Alzheimer's disease. For applying to Alzheimer's disease, the inhibitory actions of NK-4 analogues on AchE activity were compared. Using the 4-types of NK-4 analogues used in Experiment 11, represented by General formula 2 having a carbon atom number of two to five of the alky group in the side chain, the residual ratios (%) of AchE activity were measured by the same method as in Experiment 8. Using NK-150 as an NK-19 analogue, the residual ratios (%) of AchE activity were measured. The values of $IC_{50}$ (corresponding to a concentration of each compound used in this experiment that suppresses AchE activity by 50%), calculated based on the results, and the residual ratios of their activity are also shown in Table 21.

Table 21

| Compoun d | Residual ratio of AchE activity (%) | | | | | | | | ICso (μg/ml) |
|---|---|---|---|---|---|---|---|---|---|
| | Concentration of compound (μg/ml) | | | | | | | | |
| | 0 | 0.78 | 1.56 | 3.13 | 6.25 | 12.5 | 25 | 50 | |
| NK-4 | 100 | 91 ± 4 | 47 ± 4** | 41 ± 9** | 38 ± 7** | 24 ± 2** | 25 ± 3** | 22 ± 2** | 1.5 |
| NK-234 | 100 | 77 ± 21 | 77 ± 13 | 47 ± 11* | 45 ± 9** | 30 ± 12** | 26 ± 6** | 12 ± 6** | 3.0 |
| NK-26 | 100 | 94 ± 11 | 84 ± 9 | 88 ± 8 | 88 ± 7 | 69 ± 5** | 48 ± 4** | 36 ± 9** | 23.3 |
| NK-9815 | 100 | 82 ± 2 | 98 ± 24 | 93 ± 36 | 94 ± 16 | 56 ± 7** | 41 ± 14** | 24 ± 4** | 17.3 |

(continued)

| Compoun d | Residual ratio of AchE activity (%) | | | | | | | | ICso (μg/ml) |
|---|---|---|---|---|---|---|---|---|---|
| | Concentration of compound (μg/ml) | | | | | | | | |
| | 0 | 0.78 | 1.56 | 3.13 | 6.25 | 12.5 | 25 | 50 | |
| NK-150 | 100 | 95 ± 1 | 106 ± 11 | 124 ± 10 | 101 ± 14 | 99 ± 11 | 104 ± 5 | 78 ± 4** | 50< |
| * **: Significantly different from the case of "0% compound concentration" (*: P < 0.05, **: P < 0.01)<br>Data measured: Average ± SD | | | | | | | | | |

As evident from Table 21, the higher the residual ratio of AchE activity, the larger the carbon atom number of NK-4 analogues is. NK-4 and NK-234, particularly NK-4, strongly inhibited AchE activity. NK-150, a NK-19 analogue, was revealed to have a lesser action of inhibiting AchE activity than NK-4 analogues.

These results indicate that NK-4 and NK-234, particularly NK-4, are usable as a therapeutic agent for Alzheimer's disease. Using donepezil hydrochloride ("ARICEPT"), which is used for treating Alzheimer's disease as an inhibitory agent for AchE activity, the residual ratio (%) of AchE activity was measured by the same experiment system, resulting in an $IC_{50}$ of 0.9 μg/ml. The results indicate that NK-4 has almost comparable activity of inhibiting AchE activity to that of donepezil hydrochloride.

Experiment 15: Effect of NK-4 analogues and NK-19 analogues on human Alzheimer's dementia-model mouse

Since the above experiments suggested that NK-4 can be used as a therapeutic agent for Alzheimer's disease, this experiment was conducted to examine the influences of NK-4 analogues and NK-19 analogues on human Alzheimer's dementia-model mouse.

<Test sample>

As test samples, NK-4, NK-234, NK-26, NK-19 and NK-150 were used. As control 1, physiological saline was administered at a dose of 200 μl/mouse. As control 2, donepezil hydrochloride was used. Each sample was dissolved in DMSO to give a concentration of 5 mg/ml, diluted with physiological saline, and then administered.

<Experimental method>

One hundred of ICR mice (male, five weeks of age, body weight of 25 to 30 g, commercialized by Charles Liver Laboratories Japan Inc., Kanagawa, Japan) were randomly divided into 10 groups, each consisting 10 heads, and raised separately until the experiment was terminated. Mice, which had been anesthetized with chloral hydrate (administered into the abdominal cavity in an amount of 350 mg/kg body weight, commercialized by SIGMA Corporation, Tokyo, Japan), were fixed at their dorsal decubitis parts, incised along their medial heads, and after confirming born suture, 9 nmol/6 μl/mouse of a solution of amyloid fragment (β-$Amyloid_{25-35}$), represented by amino acid sequence of SEQ ID No.: 1, was injected into their brain ventricles by puncturing at 1.0-mm left side and 0.5-mm backside from bregmas with a depth of 3 mm (the administration method is referred to "Brain Research", Vol. 706, pp. 181-193 (1996)). A micro syringe with an 8-gauge stainless needle (3 mm length) was used for injection. The injection site was determined by confirming the staining at right-and-left lateral ventricles of the frontal section, dorsal third ventricle, and ventral third ventricle by injecting an Evans' Blue solution (0.3 μg/0.3 μl) in place of the solution of amyloid β fragment. The scalps were sutured after injection, any one of the compounds was administered intraperitoneally once a day over 13 days, and the behavioral score was determined by the method described below. The results and the groups are shown in Table 22.

Table 22

| Test group | | | Mouse administered with amyloid β peptide | |
|---|---|---|---|---|
| Compound | Dose (μg/kg body weight) | Administration of amyloid β fragment | Index for object discrimination | Passive avoidance reaction (sec) |
| Control 1 (physiologic al saline) | 0 | No | 0.42 ± 0.11 | 174 ± 6 |
| - | 0 | Yes | -0.06 ± 0.06 | 77 ± 26 |

(continued)

| Test group | | | Mouse administered with amyloid β peptide | |
|---|---|---|---|---|
| Compound | Dose (μg/kg body weight) | Administration of amyloid β fragment | Index for object discrimination | Passive avoidance reaction (sec) |
| Control 2 | 200 | Yes | 0.15 ± 0.05 | 145 ± 22* |
| Control 2 | 1000 | Yes | 0.26 ± 0.08** | 142 ± 20* |
| NK-4 | 50 | Yes | 0.15 ± 0.07 | 135 ± 24* |
| NK-4 | 500 | Yes | 0.41 ± 0.08** | 164 ± 16** |
| NK-234 | 500 | Yes | 0.29 ± 0.07** | 166 ± 8** |
| NK-26 | 5 | Yes | 0.05 ± 0.08 | 84 ± 25 |
| NK-26 | 50 | Yes | 0.19 ± 0.07* | 160 ± 14** |
| NK-26 | 500 | Yes | 0.11 ± 0.08 | 144 ± 13* |
| NK-19 | 500 | Yes | 0.34 ± 0.11** | 107 ± 25 |
| NK-150 | 500 | Yes | 0.36 ± 0.08** | 80 ± 23 |
| *, **: Significantly different from mouse administered with amyloid β fragment alone (*: P < 0.05, **: P < 0.01)<br>Data measured : Average ± SD | | | | |

<Evaluation method>

<Novelty object recognition test>

Novelty object recognition test is a test using a characteristic mouse's preference for novelty, differing from other evaluation methods of learning in that the test does not use any artificial reinforced factor. The test, which consists of three parts of acclimation, practicing trial, and holding trial, was operated at six to eight days after administering amyloid β fragment into the subjects' ventricles. The experiment apparatus (40 cm in depth, 30 cm in width and 30 cm in height), in which the floor was paved with wood tips, was placed in a noiseless place under an illumination of about 1, 000 lux. On the $6^{th}$ day after the administration, a mouse was placed in the center of the apparatus without seeking object and allowed to seek freely for 10 minutes (acclimation). After 24 hours (on the $7^{th}$ day after the administration), two objects (objects A and B) were placed in the apparatus at a position 10-cm apart from the lateral side, and the mouse was placed in the center of the apparatus and allowed to seek freely for 10 minutes (practicing trial). After 24 hours (on $8^{th}$ day after the administration), object A (memorized object) sought by the mouse on the previous day was placed at the same position of object A as placed on the previous day, and object C (novel object) different from object B used on the previous day was placed at the same position was placed at the same position of object B as placed on the previous day, and then the mouse was placed in the center of the apparatus and allowed to seek freely for 10 minutes (holding trial). When the mouse directed its muffle close to the object within 2 cm apart from the object or allowed its muffle to contact with the object, the mouse was judged to be seeking, and the seeking time thereof was measured with a stopwatch. The object discrimination index [= {(seeking time for novel object) - (seeking time for memorized object)} / {(seeking time for novel object) + (seeking time for memorized object)}] was calculated. The object discrimination index means the ratio of increase in seeking time for novel object to the total seeking time, which becomes longer when the memory of the once sought object is more strongly held and becomes shorter when the memory is more weakly held.

<Passive avoidance test>

The captioned test is the one for evaluating animals' avoidance behavior to the once experienced aversive stimulus (electric stimulus) as an index of memory. In this experiment, step-through test was selected as a passive avoidance test, which uses the nature of mouse who favors dark room. Using an apparatus having a bright room and a dark room connected via a door, a mouse was placed in the bright room, and measured a time required for moving into the dark room as an index of memory. The passive avoidance test was performed on 9 to 12 days after the injection of amyloid β fragment into the ventricle. On the $9^{th}$ day after the administration, the mouse was placed in the bright room (1, 000 lux, 30-cm in depth, 30-cm in width and 15-cm in height) for a minute, and then placed in the dark room (30-cm in depth, 30-cm in width and 15-cm in height) for two minutes to be acclimatized. On the $10^{th}$ day after the administration,

the mouse was further acclimatized similarly as above. On the 11th day after the administration, the mouse was placed in the center of the bright room, and when the mouse moved into the dark room, the door between the rooms was immediately closed and the electric stimulus (0.8 mA, for a second) was given to the mouse. After 24 hours (on the 12th day after the administration), the mouse was placed in the center of the bright room as had been placed in the previous day, and the time required for moving into the dark room was measured as a passive avoidance reaction. When the mouse memorized the aversive electric stimulus, the passive avoidance reaction was elongated.

As evident from Table 22, the mice administered with 500 μg/kg body weight of NK-4, 500 μg/kg body weight of NK-234, 50 μg/kg body weight of NK-26, 500 μg/kg body weight of NK-19, or 500 μg/kg body weight of NK-150 marked a significant improvement in the novelty object recognition test compared to the mouse administered with amyloid β fragment alone. The mice administered with 50 μg/kg body weight of NK-4, 500 μg/kg body weight of NK-4, 500 μg/kg body weight of NK-234, 50 μg/kg body weight of NK-26, 500 μg/kg body weight of NK-26, or 500 μg/kg body weight of NK-19 marked a significant improvement in the passive avoidance test compared to the mouse administered with amyloid β fragment alone. Particularly, the mouse administered with 500 μg/kg body weight of NK-4 marked a more distinct improvement in both of the novelty object recognition test and the passive avoidance test than that of the mouse administered with 1, 000 μg/kg body weight of donepezil (Control 2). No adverse effect due to NK-4, NK-234, NK-26, NK-19 or NK-150 was observed. The results indicate that NK-234, NK-26, NK-19 and NK-150 has an effect of improving the dementia due to amyloid β peptide, and NK-4 has the highest effect of improving the dementia.

Experiment 16: Effects of NK-4 on APP transgenic mouse

NK-4, which showed the highest effect of improving dementia of mouse administered with amyloid β fragment in Experiment 14, the effect of NK-4 on a commercialized APP transgenic mouse (APP Tg mouse) introduced with a causative gene mutation of Swedish Alzheimer's disease. Forty five APP Tg mice (female, 10-weeks of age, bodyweight of 15 to 23 g, commercialized by Taconic Farms, Inc., NY, USA) was preliminary raised for 10 days, divided into four groups to give an equivalent total body weight. Each mouse was raised alone and administered with an intraperitoneal administration of NK-4 five times a week for 12 weeks. As Control 1, 10 mice with no introduction of gene mutation (wild type, female, 10-weeks of age, body weight of 15 to 23 g) were preliminary raised for 10 days, and further raised alone with an intraperitoneal administration of physiological saline five times a week for 12 weeks. As Control 2, APP Tg mouse was administered with physiological saline five times a week for 12 weeks. As Control 3, APP Tg mouse was administered with donepezil hydrochloride five times a week for 12 weeks. On the 12th weeks after the administration of either NK-4, physiological saline or donepezil hydrochloride, the novelty object recognition test was firstly performed, and then the passive avoidance test was performed by the same method as in Experiment 15. The following water maze test was successively performed. The results are in Table 23.

Table 23

| Administered compound | Dose (μg/kg body weight) | Type of mouse | Mouse (heads) | Index for object discrimination | Passive avoidance reaction (sec) | Water maze test | | | |
|---|---|---|---|---|---|---|---|---|---|
| | | | | | | 1st day | 2nd day | 3rd day | 4th day |
| Physiological saline (Control 1) | - | Wild type | 10 | 0.49 ± 0.10 | 284 ± 15 | 95 ± 10 | 63 ± 10 | 43 ± 6 | 25 ± 6 |
| Physiological saline (Control 2) | - | APP Tg | 10 | -0.02 ± 0.08 | 187 ± 36 | 118 ± 2 | 110 ± 6 | 99 ± 11 | 56 ± 11 |
| Donepezil HCl (Control 3) | 200 | APP Tg | 5 | 0.31 ± 0.16 | 200 ± 61 | 120 ± 0 | 77 ± 19* | 53±13* | 62 ± 16 |
| NK-4 | 100 | APP Tg | 10 | 0.20 ± 0.06 * | 280 ± 20* | 101 ± 38* | 73 ± 13* | 59 ± 10** | 34 ± 10 |

(continued)

| Administered compound | Dose (μg/kg body weight) | Type of mouse | Mouse (heads) | Index for object discrimination | Passive avoidance reaction (sec) | Water maze test | | | |
|---|---|---|---|---|---|---|---|---|---|
| | | | | | | 1st day | 2nd day | 3rd day | 4th day |
| NK-4 | 500 | APP Tg | 10 | 0.30 ± 0.05** | 243 ± 25 | 116 ± 3 | 63 ± 12* | 40 ± 7** | 27 ± 7* |
| *, **: Significantly different from APP Tg mouse administered with physiological saline (*: P < 0.05, **: P < 0.01) Data measured : Average ± SD | | | | | | | | | |

<Water maze test>

In a circle pool with a diameter of 130 cm, water dyed with a white ink was filled up to give a 20-cm in depth and was regulated at a temperature of 23±1°C with an aquarium heater. The pool was divided into four parts and a refuge platform was installed at the center of one part, at 10-cm apart from the sidewall of the pool and 2-cm under the water surface. The position of the platform was not changed throughout the test. From the next day of the end of the passive avoidance test, a mouse was placed in the pool toward the sidewall of the pool, and the time required for arriving the platform hidden under the water surface was measured. The start point was 10-cm apart from the sidewall of the pool and at the center of any one of the four divided parts, and it was randomly altered at each test. The mouse was allowed to seek the platform freely. When the mouse was not able to reach the platform within two minutes, it was led to the platform, allowed to stay therein for 30 seconds, and then transferred to a cage paved with a paper towel. The same test (a second test) was performed again at one minute after the end of the first test. These tests were successively performed over four days, and the data for each day was obtained by averaging the data from twice tests.

As evident from Table 23, NK-4 improved the dementia of APP Tg mouse administered with NK-4 in any of the object discrimination test, passive avoidance test, and water maze test. The improvement effect was significantly higher than that of donepezil hydrochloride, a commercialized therapeutic agent for Alzheimer's disease. These results indicate that NK-4 can be widely used as a therapeutic agent for Alzheimer's dementia. No adverse effect of NK-4 was observed throughout the experiment.

Experiment 16: Effects of NK-4 on mouse in cerebrovascular dementia

Since the above experiments revealed that NK-4 effectively improves both ataxia induced by cerebral infarction and Alzheimer's dementia, the effect of NK-4 on mice with cerebrovascular dementia was investigated in this experiment. Thirty-one C57BL/6J mice (male, 12-weeks of age, commercialized by CLEA Japan Inc., Tokyo, Japan) were preliminarily raised for a week. After twenty-one heads of the mice were administered with atropine (0.3 mg/kg, subcutaneous administration), they were intraperitoneally administered with sodium pentobarbitone (50 mg/kg), anesthetized, and then operated for permanent ligation of the right common carotid artery (see, Japanese Patent Kokai No. 2008-193941 about the operation method). After the ligation operation, all the mice were respectively raised, without any restriction of feed and water. Ten out of the 21 mice with the operation were raised (ligation group), and the remaining other 11 mice were administered with NK-4 (administration group). The 10 mice without the operation were used as a control (no-operation group). From the 2nd day after the operation, the no-operation group and the ligation operation group were administered with physiological saline, and the NK-4 administration group was intraperitoneally administered with NK-4 (100 μg/kg) every day (five days a week). On the 3rd week and the 4th week after the operation, the novelty object recognition test was performed by the same method as in Experiment 15. The results are in Table 24.

Table 24

| Test group | Mouse (head) | Object discrimination index | |
|---|---|---|---|
| | | On the 3rd week after operation | On the 4th week after operation |
| No-operation group | 10 | 0.45 ± 0.22 | 0.32 ± 0.16 |
| Ligation group | 10 | 0.16 ± 0.19 | 0.08 ± 0.20 |

(continued)

| Test group | Mouse (head) | Object discrimination index | |
|---|---|---|---|
| | | On the 3rd week after operation | On the 4th week after operation |
| Group administered with NK-4 | 11 | 0.51 ± 0.22** | 0.31 ± 0.20* |
| *, ** : Signif icantlydifferent from ligation group (*: P < 0.05, **: P < 0.01)<br>Data measured : Average ± SD | | | |

As evident from Table 24, the object discrimination index of the NK-4 administered group was not different from that with no-operation group, resulting in almost entire recovering from cerebrovascular dementia due to ligation of the right common carotid artery. These results strongly indicate that NK-4 can be used as a therapeutic agent for vascular dementia. No adverse effect of NK-4 was observed throughout the experiment.

The above experimental results indicate that the compounds represented by General formula 2, having an alkyl group of carbon atom number of 2 to 8 in the side chain, have effects of suppressing neurodegeneration; and the compounds represented by General formula 3, having an alkyl group of carbon atom number of 3 to 10 in the side chain, such as NK-19, NK-53, and NK-150. Any of the above-identified compounds can be used as an anti-neurodegenerative disease agent for the treatment of dementia due to cerebral infarction, ataxia, Alzheimer's dementia, vasculardementia, and cerebellum. Particularly, the compounds represented by General formula 2, having an alkyl group of carbon atom number of 2 to 4 in the side chain, such as NK-4, NK-234 and NK-26, have a higher effect of suppressing neurodegeneration, and more particularly, NK-4 has an excellent effect of improving symptoms caused by dementia due to cerebral infarction, ataxia, Alzheimer's dementia, vascular dementia, or Parkinson's disease.

The following examples explain the anti-neurodegenerative disease agent of the present invention, but they do never restrict the present invention.

<u>Example 1</u>

<u>Liquid agent for injection</u>

A solution obtained by dissolving 60 g of a purified maltose for injection (produced by Hayashibara Co., Ltd., Okayama, Japan) in 370 g of refined water for injection, and a solution obtained by dissolving, as an effective ingredient, 12 mg of any one of NK-4 (the compound represented by Chemical formula 2), NK-26 (the compound represented by Chemical formula 1), NK-28 (the compound represented by General formula 2, having an alkyl group (R) of carbon atom number of 7 in the side chain), NK-147 (the compound represented by General formula 2, having an alkyl group (R) of carbon atom number of 8 in the side chain), NK-19 (the compound represented by Chemical formula 4), NK-53 (the compound represented by Chemical formula 5), NK-150 (thecompound represented by Chemical formula 3), NK-393 (the compound represented by General formula 3, having an alkyl group (R) of carbon number of 8 in the side chain), NK-100 (the compound represented by Chemical formula 6), NK-528 (the compound represented by Chemical formula 7), NK-557 (the compound represented by Chemical formula 8), and NK-1516 (the compound represented by Chemical formula 9), all of which are produced by Hayashibara Biochemical Laboratories, Inc., Okayama, Japan, as an effective ingredient, the following compounds, in 170 g of refined water for injection were mixed, sterilized by filtration, and bubbled with nitrogen gas to decrease the dissolved oxygen down to about 0.1 ppm, and divided by 1 ml in brown amples, followed by sealing the amples under a nitrogen-gas stream. The products can be used as a pyrogen-free anti-neurodegenerative disease agent. They can be also used as an agent for suppressing neurodegeneration, protecting neurocytes, and promoting neurite outgrowth, as well as therapeutic agents for diseases caused by neurodegeneration or neurological dysfunction. The product can be also used as an agent for preserving brain, suppressing oxidative cerebral disorder, suppressing ischemic cerebral disorder, suppressing cerebral infarction development, suppressing cerebral edema, suppressing delayed neural death, normalizing brain function, and suppressing oxidative stress, as well as an agent for anti-ulcer, suppressing blood sugar elevation, treating and preventing eye diseases, preserving organs for transplant, preventing necrosis of organs for transplant, treating and preventing organs and tissues, treating and preventing radiation damages, anti-tumors, suppressing tumor-metastasis, suppressing markers of cell disorders, treating and preventing inflammation disorders and tissue disorders caused thereby, suppressing disorders of sensory cells/sensory neurons/sensory organs, treating and preventing chemical addiction, inhibiting Na-Ca exchange system, treating and preventing pain and pruritus, stimulating protein kinase, treating and preventing mitochondrial encephalomyopathy, treating and preventing artery obstruction and stenosis, suppressing breakthrough of blood-brain barrier, treating drug dependence, suppressing apoptosis, suppressing the production of lipid peroxide, scavenging radicals, inhibiting aggregation of amyloid β peptide, suppressing damages induced by amyloid β peptide, inhibiting acetylcholine esterase

(AchE) activity, activating serine/threonine kinase(Akt), activating phosphatidylinositol (3,4,5) 3-phosphate kinase (PI3K) - serine/threonine kinase (Akt) cascade, enhancing elevation of cyclic AMP concentration, or suppressing SAPK/JNK phosphorylation. The anti-neurodegenerative disease agent of the present invention can be used as a therapeutic or prophylactic agent for animals, including pets as well as humans, with neurodegenerative diseases.

Using the above-identified agents, their therapeutic effects on motor coordination reduction of hamsters with cerebellar ataxia and on mice administered with amyloid β fragment (Alzheimer' disease model) were investigated.

Effect on motor coordination reduction of hamster with cerebellar ataxia

Similarly as in Experiment 2, 130 hamsters with cerebellar ataxia, born within the same week and 3-weeks of age, were randomly divided into 13 groups, consisting of 10 hamsters, respectively. As shown in Table 19, 12 groups (10 hamsters in each group), 3- to 10-weeks of age, were intraperitoneally administered with one of the agents, prepared in Example 1 and containing any one of the 12 compounds, in an amount of 0.5 ml/hamster (test groups 1 to 12) once a day for 56 days. The hamsters (10 heads) in the remaining one group were intraperitoneally administered from their age of 3- to 10-weeks with a sterilized 10% maltose solution (pyrogen-free) in an amount of 0.5 ml/hamster (control) once a day for 56 days At the next day after the end of the administration, body weight of each hamster was weighed, followed by performing rotarod test, slope endurance test, and counting of falling down frequency. The types of compounds applied to each group and the results are in Table 25. Since the average body weight of the control hamsters was 35.4 g at 3-weeks of age and 122.9 g at 10-weeks of age, which were not significantly different from any one of the test groups 1 to 12 administered with any one of the agents prepared in Example 1. Accordingly, only the results of rotarod test, slope endurance test, and falling down frequency are shown in Table 25.

<Effecton mouse administered with amyloid(3fragment(Alzheimer' disease model)>

One hundred and thirty ICR mice (commercialized by Charles Liver Laboratories Japan Inc., Kanagawa, Japan) were randomly divided into 13 groups, consisting of 10 mice in each group. Amyloid β fragment, represented by an amino acid sequence of SEQ ID NO: 1, used in Experiment 3 was aged at 37°C for four days, and administered to the lateral ventricle of each mouse in an amount of 9 nmol/6 μl/mouse (the administration method is referred to "Brain Research", Vol. 706, pp. 181-193 (1996)). From the 1$^{st}$ day after the administration, as shown in Table 20, 12 groups (consisting of 10 mice in each group) were intraperitoneally administered with one of the agents, prepared in Example 1 and containing any one of the 12 compounds, once a day in an amount of 0.3 ml/head (test groups 13 to 24) up to the 8$^{th}$ day. The mice (10 heads) in the remaining one group were intraperitoneally administered with a sterilized 10% maltose solution (pyrogen-free) once a day up to the 8$^{th}$ day in an amount of 0.3 ml/mouse (control). On the 8$^{th}$ day after the administration, novel object recognition test (see, Japanese Patent Kokai No. 2008-193941) was performed and the average discrimination index (the ratio of prolonged seeking time for novel object to the total seeking time) as a cognitive function index were shown in Table 26. On the 9$^{th}$ day after the administration, the brain of the mice was anatomically isolated, prepared into tissue samples in conventional manner. Depositions of aggregation of amyloid β fragment were observed by staining with Congo red or thioflavin T, and degeneration or defect of hippocampal pyramidal cells concerned with cognitive function. The degeneration or the defect of hippocampal pyramidal cells was evaluated by 4-point grade score, by regarding the condition of the control without administration of amyloid β fragment as "none (0)", "slight (1)", "moderate (2)", and "sever (3)", and the average scores of 10 mice are shown in Table 20.

<Novelty object recognition test>

An experiment apparatus (a glass box of 30 cm in depth, 45 cm in width, and 30 cm in height) and two kinds of objects to be memorized by mice were provided. On the day before the test, the mice were allowed to seek freely in the experiment apparatus with no seeking object for 10 minutes for acclimation. On the test day, the following test was performed twice at an interval of 60 minutes. In the first trial, the same two objects were placed in the apparatus at its both ends, and the mouse was allowed to seek freely for 10 minutes. On the second trial, one of the objects used in the first test was replaced with a different kind of object, and then the mouse was allowed to seek freely for five minutes. In each experiment, when the mouse brought its muffle close to the object within 1-cm apart from the object, or made its muffle or whiskers to contact with the object, the mouse was judged to have a seeking behavior, and the seeking time thereof was measured. The object discrimination index [= {(seeking time for novel object) - (seeking time for memorized object) } / {(seeking time of novel object) + (seeking time of memorized object)}] was calculated. The object discrimination index means the ratio of an increased seeking time for novel object to the total seeking time, and the ratio becomes larger when the memory of the once sought object was more strongly held, and it becomes smaller when the memory was more weakly held.

Table 25

| Test group | Effective ingredient | Hamster with cerebellar ataxia | | |
|---|---|---|---|---|
| | | Time of falling from rotarod (sec) | Endurable-slant-slope -angle (°) | Falling down frequency (count/min) |
| Group 1 | NK-4 | 88. 4 ± 3.2** | 55.8 ± 2.5* | 1.1 ± 0.8** |
| Group 2 | NK-26 | 90.0 ± 7.1** | 53.5 ± 1.3* | 1.0 ± 0.7** |
| Group 3 | NK-28 | 69.0 ± 28** | 51.4 ± 2.2* | 1.3 ± 0.9** |
| Group 4 | NK-147 | 56.5 ± 9.8** | 49.3 ± 2.1* | 1.5 ± 0.8** |
| Group 5 | NK-19 | 75.6 ± 6.1** | 50.3 ± 1.7* | 1.1 ± 0.4** |
| Group 6 | NK-53 | 74.6 ± 9.4** | 50.6 ± 2.4* | 1.2 ± 0.5** |
| Group 7 | NK-150 | 94.0 ± 9.2** | 54.1 ± 1.2* | 0.7 ± 0.6** |
| Group 8 | NK-393 | 86.5 ± 1.8** | 53.1 ± 1.5* | 0.9 ± 0.8** |
| Group 9 | NK-100 | 61.9 ± 9.4** | 50.3 ± 1.9* | 1.3 ± 0.7** |
| Group 10 | NK-528 | 37.5 ± 11.2** | 43.9 ± 3.0* | 5.2 ± 1.4* |
| Group 11 | NK-557 | 39.9 ± 5.1** | 45.1 ± 3.7* | 6.5 ± 1.1* |
| Group 12 | NK-1516 | 42.4 ± 6.3** | 46.5 ± 2.9* | 7.1 ± 0.8* |
| Control | Maltose | 0 ± 0 | 35.8 ± 5.1 | 12.8 ± 0.5 |
| * **: Significantly different from control (*: P < 0.05, **: P < 0.01) | | | | |

Table 26

| Test group | Effective ingredient | Mouse administered with amyloid β peptide | |
|---|---|---|---|
| | | Object discrimination index | Degeneration/defluxion of hippocampal pyramidal cell |
| Test group 13 | NK-4 | 0.1 | 1.1 |
| Test group 14 | NK-26 | 0.1 | 1.1 |
| Test group 15 | NK-28 | 0.2 | 1.4 |
| Test group 16 | NK-147 | 0.2 | 1.5 |
| Test group 17 | NK-19 | 0.2 | 1.1 |
| Test group 18 | NK-53 | 0.2 | 1.1 |
| Test group 19 | NK-150 | 0.2 | 1.1 |
| Test group 20 | NK-393 | 0.2 | 1.1 |
| Test group 21 | NK-100 | 0.1 | 0.9 |
| Test group 22 | NK-528 | 0.3 | 2.2 |

(continued)

| Test group | Effective ingredient | Mouse administered with amyloid β peptide | |
|---|---|---|---|
| | | Object discrimination index | Degeneration/defluxion of hippocampal pyramidal cell |
| Test group 23 | NK-557 | 0.4 | 2.1 |
| Test group 24 | NK-1516 | 0.4 | 1.9 |
| Control | Maltose | 0.5 | 3.1 |

As evident from Table 25, all the 12 agents, prepared in Example 1, distinctly improved the hamsters with motor coordination and cerebellar ataxia from shortening in their falling times from the rotarod, lowering in their endurable-slant-slope-angles, and increasing in their falling down frequencies. Comparing the intensities of the effects of the administered 12 agents, the pentamethine cyanine dye compounds (NK-4, NK-26, NK-28, NK-147, NK-19, NK-53, NK-150, NK-393, NK-100, NK-528, NK-557 and NK-1516) had a higher effect of improving motor coordination (test groups 1 to 9) than those with a preparation containing any one of the dimethine styryl dye compounds (NK-523, NK-557 and NK-1516) in every test. Comparing the pentamethine cyanine dye compounds, NK-4, NK-26, NK-150 and NK-393, had a particularly high improving effect on motor coordination. The results indicate that all the prepared agents can be used as a therapeutic agent for neurodegenerative diseases. Since the body weights of the hamsters were not significantly different from those of the control group even after their administrations of over 56 days, the agents were considered to be harmless. As evident from Table 26, the 12 agents, prepared in Example 1, suppressed the reduction of cognitive function and the degeneration or the defection of hippocampal pyramidal cells in the mouse administered with amyloid β fragment. Comparing the effects of the 12 agents, the pentamethine cyanine dye compounds (NK-4, NK-26, NK-28, NK-147, NK-19, NK-53, NK-150, NK-393, NK-100, NK-528, NK-557 and NK-1516) had a relatively higher effect on the degeneration of hippocampal pyramidal cells or the dysfunction of cognitive function(test groups 13 to 21) than the dimethine styryl dye compounds (NK-523, NK-557 and NK-1516) in every test.

Example 2

Liquid agent for injection

A solution obtained by dissolving 60 g of a purified maltose for injection (produced by HayashibaraCo., Ltd., Okayama, Japan) in 370 g of refined water for injection, a solution obtained by dissolving in 170 g of refined water for injection 2 g of lecithin and, as an effective ingredient, 120 mg of any one of NK-4 (the compound represented by Chemical formula 2), NK-234 (the compound represented by General formula 2, having an alkyl group (R) of carbon atom number of three in the side chain), NK-26 (the compound represented by Chemical formula 1), NK-28 (the compound represented by General formula 2, having an alkyl group (R) of carbon atom number of 7 in the side chain), NK-147 (the compound represented by General formula 2, having an alkyl group (R) of carbon atom number of eight in the side chain), NK-19 (the compound represented by Chemical formula 4), NK-53 (the compound represented by Chemical formula 5), NK-150 (the compound represented by Chemical formula 3), NK-393 (thecompound represented by General formula 3, having an alkyl group (R) of carbon atom number of eight in the side chain), NK-100 (the compound represented by Chemical formula 6), NK-528 (thecompound represented by Chemical formula 7), NK-557 (the compound represented by Chemical formula 8) and NK-1516 (the compound represented by Chemical formula 9), all of which were produced by Hayashibara Biochemical Laboratories, Inc., Okayama, Japan, were mixed, sterilized by filtration, bubbled with nitrogen gas to decrease the dissolved oxygen to about 0.1 ppm, and divided into brown amples by 1 ml, followed by sealing the amples under a nitrogen gas stream. The products can be used as a pyrogen-free anti-neurodegenerative disease agent. They can be also used as an agent for suppressing neurodegeneration, protecting neurocytes, and promoting neurite outgrowth, as well as therapeutic agents for diseases caused by neurodegeneration or neurological dysfunction. The product can be also used as an agent for preserving brain, suppressing oxidative cerebral disorder, suppressing ischemic cerebral disorder, suppressing cerebral infarction development, suppressing cerebral edema, suppressing delayed neural death, normalizing brain function, and suppressing oxidative stress, as well as an agent for anti-ulcer, suppressing blood sugar elevation, treating and preventing eye diseases, preserving organs for transplant, preventing necrosis of organs for transplant, treating and preventing organs and tissues, treating and preventing radiation damages, anti-tumors, suppressing tumor-metastasis, suppressing markers of cell disorders, treating and preventing inflammation disorders and tissue disorders caused thereby, suppressing disorders of sensory cells/sensory neurons/sensory organs, treating and preventing chemical addiction, inhibiting Na (sodium)-Ca(calcium) exchange system, treating and preventing pain and pruritus, stimulating protein kinase, treating and preventing mitochondrial encephalomyopathy, treating and preventing artery obstruction and stenosis, suppressing breakthrough of blood-brain barrier, treating drug dependence, suppressing apoptosis, suppressing the production of lipid peroxide, scavenging radicals, inhibiting aggregation of amyloid β peptide, suppressing damages induced by amyloid β peptide, inhibiting acetylcholine esterase (AchE) activity, activating serine/threonine kinase (Akt), activating phosphatidylinositol (3,4,5) 3-phosphate kinase (PI3K) - serine/threonine kinase (Akt) cascade, enhancing elevation of cyclic AMP concentration, or suppressing SAPK/JNK phosphorylation. The anti- neurodegenerative disease agent of the present invention can be used as a therapeutic or prophylactic agent for animals, including pets as well as humans, with neurodegenerative diseases.

The 13 types of anti-neurodegenerative agents prepared in Example 2 were respectively, intraperitoneally administered once to 10 ddy mice (average body weight of 25.6 g) at a dose of 0.5 ml/head, and observed their conditions while measuring their body weight every day for one week after the administration. As a result, they gave no significant difference in their body weight and other appearance compared with control mice, consisting of 10 ddy mice with an average body weight of 26.3 g, which had been intraperitoneally administered with a 10% maltose solution containing 0.2% lecithin. The result indicates that these preparations are safely administrable to humans based on the fact that the $LD_{50}$ of any of the 13 compounds, incorporated into the preparations as effective ingredients, is 3.9 mg/kg body weight or more.

Example 3

Liquid agent for injection

A solution obtained by dissolving 60 g of a purified maltose for injection (produced by Hayashibara Co., Ltd., Okayama, Japan) in 370 g of refinedwater for injection, a solution obtained by dissolving in 170 g of refined water 3 g of Polysorbate 80 (commercialized by NOF Corporation, Tokyo, Japan) and, as an effective ingredient, 60 mg of any one of NK-4 (the compound represented by Chemical formula 2), NK-234 (the compound represented by General formula 2 having an alkyl group (R) of carbon atom number of three in the side chain), NK-26 (the compound represented by Chemical formula 1), NK-28 (the compound represented by General formula 2 having an alkyl group (R) of carbon atom number of 7 in the side chain), NK-147 (the compound represented by General formula 2 having an alkyl group (R) of carbon atom number of eight in the side chain), NK-19 (the compound represented by Chemical formula 4), NK-53 (the compound represented by Chemical formula 5), NK-150 (the compound represented by Chemical formula 3), NK-393 (the compound represented by General formula 3 having an alkyl group (R) of carbon atom number of eight in the side chain), NK-100 (the compound represented by Chemical formula 6), NK-528 (thecompound represented by Chemical formula 7), NK-557 (the compound represented by Chemical formula 8), and NK-1516 (the compound represented by Chemical formula 9), all of which are produced by Hayashibara Biochemical Laboratories, Inc., Okayama, Japan, were mixed, sterilized by filtration, divided in brown amples by 10 ml, and lyophilized in usual manner, followed by sealing the amples under a nitrogen gas stream. The products are pyrogen-free and dissolved in 2 to 10 ml of refined water or physiological saline for injection when needed and used by instillation, subcutaneous administration, intraperitoneal injection, etc., and they can be used as an anti-neurodegenerative disease agent. The products can be used as an anti-neurodegenerative disease agent. They can be also used as an agent for suppressing neurodegeneration, protecting neurocytes, and promoting neurite outgrowth, as well as therapeutic agents for diseases caused by neurodegeneration or neurological dysfunction. The products can be also used as an agent for preserving brain, suppressing oxidative cerebral disorder, suppressing ischemic cerebral disorder, suppressing cerebral infarction development, suppressing cerebral edema, suppressing delayed neural death, normalizing brain function, and suppressing oxidative stress, as well as an agent for anti-ulcer, suppressing blood sugar elevation, treating and preventing eye diseases, preserving organs for transplant, preventing necrosis of organs for transplant, treating and preventing organs and tissues, treating and preventing radiation damages, anti-tumors, suppressing tumor-metastasis, suppressing markers of cell disorders, treating and preventing inflammation disorders and tissue disorders caused thereby, suppressing disorders of sensory cells/sensory neurons/sensory organs, treating and preventing chemical addiction, inhibiting calcium-sodium exchange system, treating and preventing pain and pruritus, stimulating protein kinase, treating and preventing mitochondrial encephalomyopathy, treating and preventing artery obstruction and stenosis, suppressing breakthrough of blood-brain barrier, treating drug dependence, suppressing apoptosis, suppressing the production of lipid peroxide, scavenging radicals, inhibiting aggregation of amyloid β peptide, suppressing damages induced by amyloid β peptide, inhibiting acetylcholine esterase (AchE) activity, activating serine/threonine kinase (Akt), activating phosphatidylinositol (3,4,5) 3-phosphate kinase (PI3K) - serine/threonine kinase (Akt) cascade, enhancing elevation of cyclic AMP concentration, or suppressing SAPK/JNK phosphorylation. The anti- neurodegenerative disease agent of the present invention can be used as a therapeutic or prophylactic agent for animals, including pets as well as humans, with neurodegenerative diseases.

Industrial Applicability

The anti-neurodegenerative disease agent of the present invention is useful for preventing, treating and/or suppressing the development of Parkinson's disease, parkinsonism, Alzheimer's disease, dementia, and cerebral stroke due to neurodegeneration, as well as for protecting neurocytes from factors which cause neurodegeneration. It can be also used for improving various types of symptoms or impaired neurofunction (for example, tremor, rigidity, akinesis..., akinesia, bradykinesia, stellreflexedysfunction, dysautonomia, lateropulsion, gait disorder, depression, disorder of memory, amyotrophia, muscle weakness, upper-limb function disorder, dysarthria, dysphagia, breathing disorder, numbness and paralysis). Since the anti-neurodegenerative disease agent of the present invention does not cause adverse effect even when administered for a relatively long period of time, it can be used harmlessly without anxiety. The present invention with such an outstanding function and effect is a significant invention that will greatly contribute to this art.

## Claims

**1.** A compound represented by General formula 2 or 3 for use in preventing or treating a neuro-degenerative disease, wherein the prevention or treatment of the neuro-degenerative disease comprises promoting Neurocyte-growth or Neurite-outgrowth:

General formula 2:

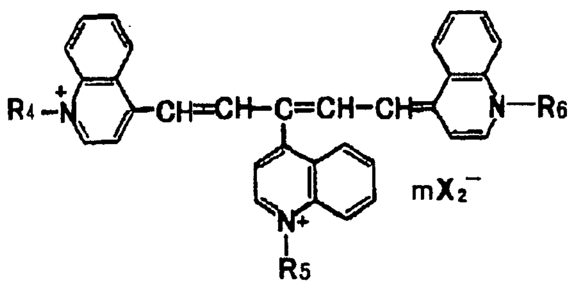

wherein in General formula 2, $R_4$ through $R_6$ each independently represent the same alkyl group having a carbon number of 2 to 8; $X_2^-$ represents a counter anion selected from the group consisting of fluorine ion, chlorine ion, bromine ion, iodine ion, prechloric acid ion, periodic acid ion, hexafluorophosphoric ion, hexafluoroantimonate ion, hexafluorostanate ion, phosphoric acid ion, fluoroboric ion, tetrafluoroborate ion, thiocyanic acid ion, benzene sulfonic acid ion, naphthalenesulfonic acid ion, naphthalenedisulfonic acid ion, p-toluenesulfonic acid ion, alkyl sulfonic acid ion, benzenecarboxylic acid ion, alkylcarboxylic acid ion, trihaloalkylcarboxylic acid ion, alkylsulfonate ion, trihaloalkylsulfonate ion, nicotinic acid ion and aspartic acid ion; and m represents an integer of 1 or 2 that forms an electric charge for balancing with the electric charge of a cationic part;

General formula 3:

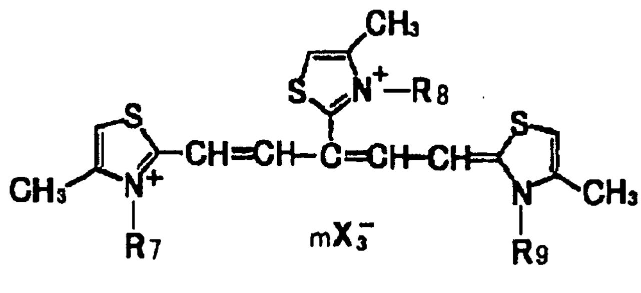

wherein in General formula 3, $R_7$ through $R_9$ each independently represent the same alkyl group having a carbon number of 3 to 12; $X_3^-$ represents a counter anion selected from the group consisting of fluorine ion, chlorine ion, bromine ion, iodine ion, prechloric acid ion, periodic acid ion, hexafluorophosphoric ion, hexafluoroantimonate ion,

hexafluorostanate ion, phosphoric acid ion, fluoroboric ion, tetrafluoroborate ion, thiocyanic acid ion, benzene sulfonic acid ion, naphthalenesulfonic acid ion, naphthalenedisulfonic acid ion, p-toluenesulfonic acid ion, alkyl sulfonic acid ion, benzenecarboxylic acid ion, alkylcarboxylic acid ion, trihaloalkylcarboxylic acid ion, alkylsulfonate ion, trihaloalkylsulfonate ion, nicotinic acid ion and aspartic acid ion; and m represents an integer of 1 or 2 that forms an electric charge for balancing with the electric charge of a cationic part.

2. A compound for use according to claim 1, wherein the compound represented by General formula 2 is the compound represented by Chemical formula 1 or 2, wherein the compound represented by General formula 3 is the compound represented by Chemical formula 3, 4 or 5

Chemical formula 1:

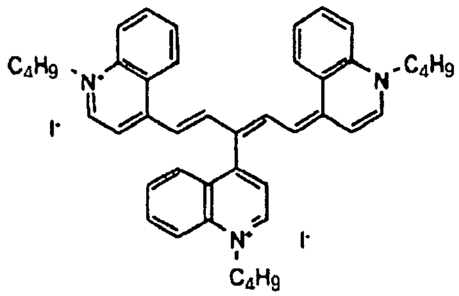

Chemical formula 2:

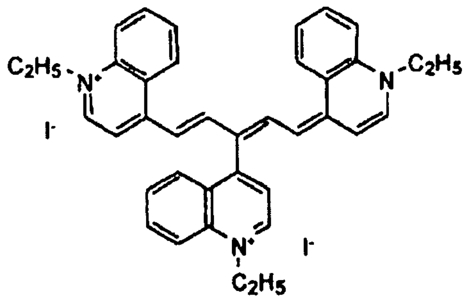

Chemical formula 3:

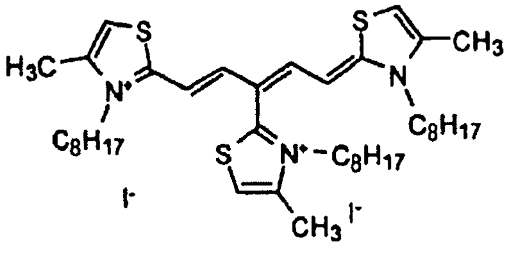

Chemical formula 4:

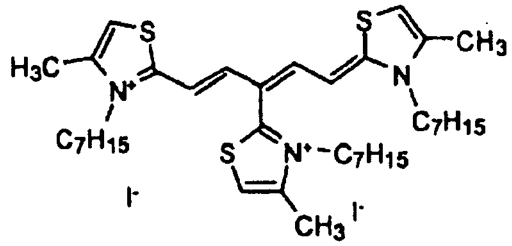

Chemical formula 5:

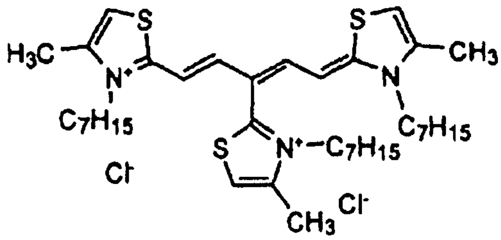

3. A composition comprising the compound of claim 1 or 2 for use in preventing or treating a neuro-degenerative disease, wherein the prevention or treatment of the neuro-degenerative disease comprises promoting Neurocyte-growth or Neurite-outgrowth, which further contains one or more pharmaceutically acceptable ingredients.

4. The composition for use according to claim 3, wherein said pharmaceutically acceptable ingredients are aqueous media.

5. The composition for use according to claim 4, wherein the use comprises inhibiting a neurocyte degeneration, protecting a neurocyte, or improving motorius degenerative diseases accompanied by neurocyte degeneration.

6. The composition for use according to claim 5, wherein said neurocyte is Purkinje's cell of cerebellum.

7. The composition for use according to claim 3 or 4, wherein said neurodegenerative diseases are Parkinson's disease, dementia, spinocerebellar degeneration, Alzheimer's disease, cerebral infarction, or ataxia.

**Patentansprüche**

1. Eine durch Allgemeine Formel 2 oder 3 dargestellte Verbindung, die zum Verhindern oder Behandeln einer neuro-degenerativen Erkrankung benutzt wird, wobei die Verhinderung oder Behandlung der neuro-degenerativen Erkrankung die Förderung von Neurozytenwachstum oder Neuritenauswuchs umfasst:

Allgemeine Formel 2:

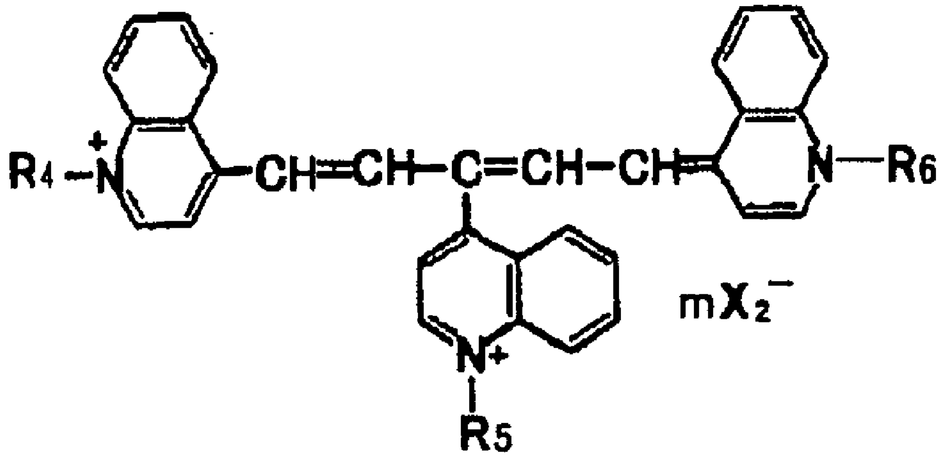

wobei $R_4$ bis $R_6$ in der Allgemeinen Formel 2 jeweils unabhängig voneinander die gleiche Alkylgruppe mit einer Kohlenstoffzahl von 2 bis 8 darstellen; wobei $X_2^-$ ein Gegenanion darstellt, das von der Gruppe ausgewählt ist, die aus Ionen von Fluor, Chlor, Brom, Jod, Perchlorsäure, Perjodsäure, Hexafluorphosphor, Hexafluorantimon, Hexafluorstannat, Phosphorsäure, Fluorobor, Tetrafluorborat, Thiocyansäure, Benzolsulfonsäure, Naphthalensulfonsäure, Naphthalendisulfonsäure, p-Toluolsulfonsäure, Alkylsulfonsäure, Benzolcarboxylsäure, Alkylcarboxylsäure, Trihaloalkylcarboxylsäure, Alkylsulfonat, Trihaloalkylsulfonat, Nikotinsäure und Asparaginsäure besteht; und wobei m eine ganze Zahl von 1 oder 2 darstellt, die eine elektrische Ladung zum Ausgleich mit der elektrischen Ladung eines kationischen Teils bildet;

Allgemeine Formel 3:

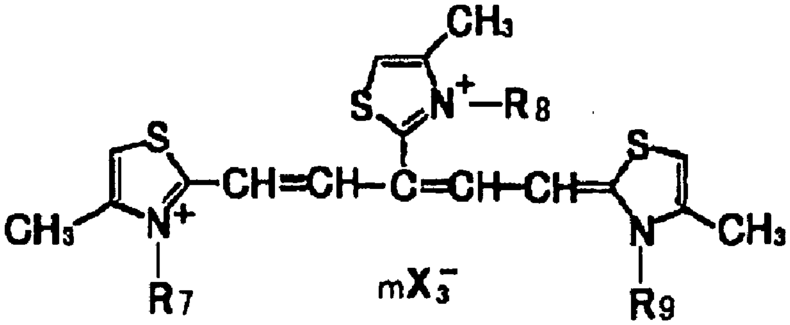

wobei $R_7$ bis $R_9$ in der Allgemeinen Formel 3 jeweils unabhängig voneinander die gleiche Alkylgruppe mit einer Kohlenstoffzahl von 3 bis 12 darstellen; wobei $X_3^-$ ein Gegenanion darstellt, das von der Gruppe ausgewählt ist, die aus Ionen von Fluor, Chlor, Brom, Jod, Perchlorsäure, Perjodsäure, Hexafluorphosphor, Hexafluorantimon, Hexafluorstannat, Phosphorsäure, Fluorobor, Tetrafluorborat, Thiocyansäure, Benzolsulfonsäure, Naphthalensulfonsäure, Naphthalendisulfonsäure, p-Toluolsulfonsäure, Alkylsulfonsäure, Benzolcarboxylsäure, Alkylcarboxylsäure, Trihaloalkylcarboxylsäure, Alkylsulfonat, Trihaloalkylsulfonat, Nikotinsäure und Asparaginsäure besteht; und wobei m eine ganze Zahl von 1 oder 2 darstellt, die eine elektrische Ladung zum Ausgleich mit der elektrischen Ladung eines kationischen Teils bildet.

**2.** Eine Anspruch 1 entsprechend zu benutzende Verbindung, wobei die durch Allgemeine Formel 2 dargestellte Verbindung die Verbindung ist, die durch Chemische Formel 1 oder 2 dargestellt ist, wobei die durch Allgemeine Formel 3 dargestellte Verbindung die Verbindung ist, die durch Chemische Formel 3, 4 oder 5 dargestellt ist.

Chemische Formel 1:

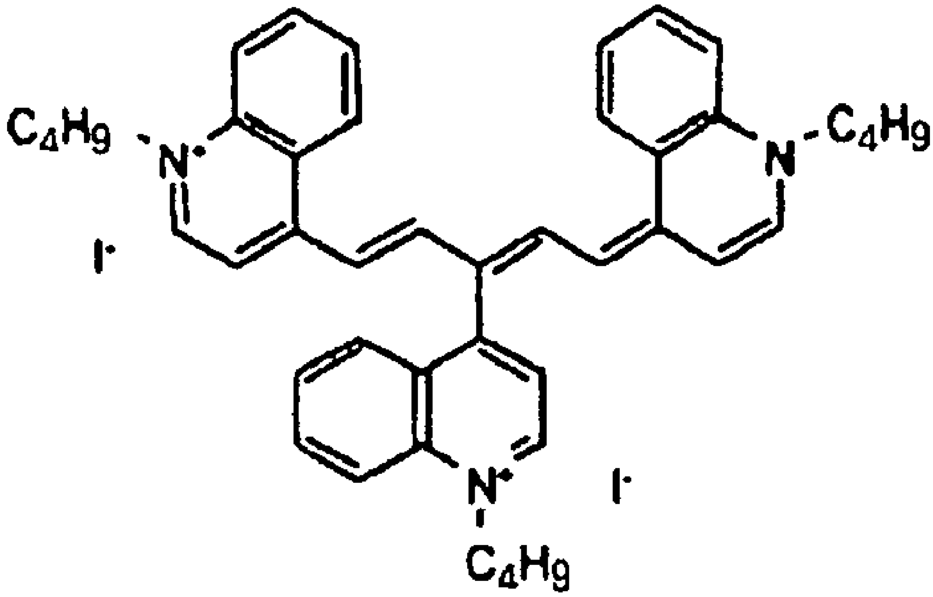

Chemische Formel 2:

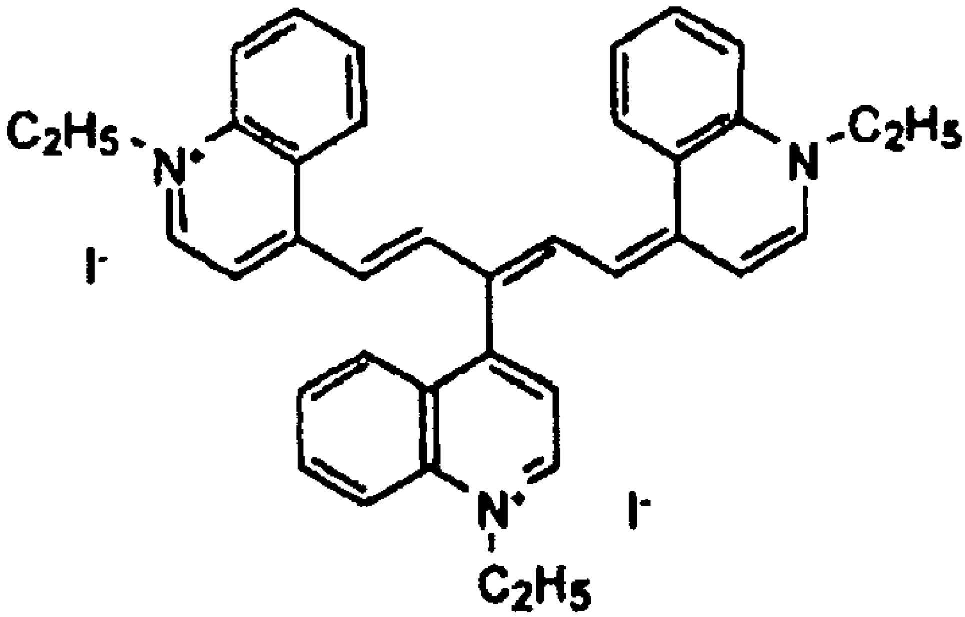

Chemische Formel 3:

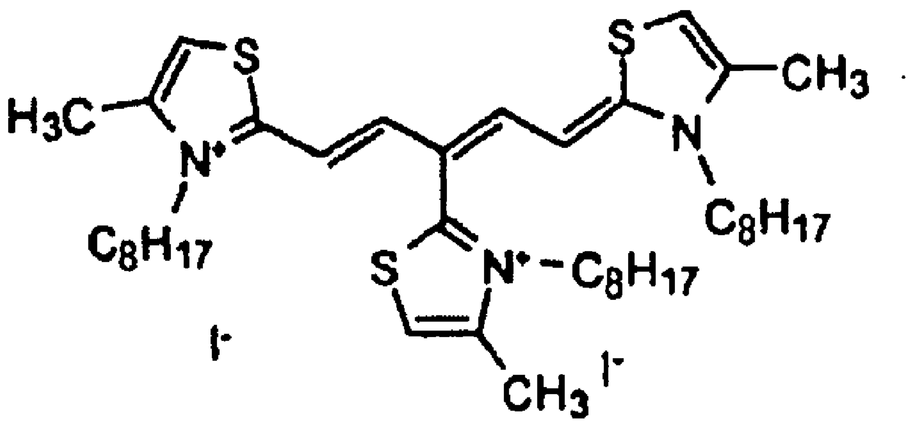

Chemische Formel 4:

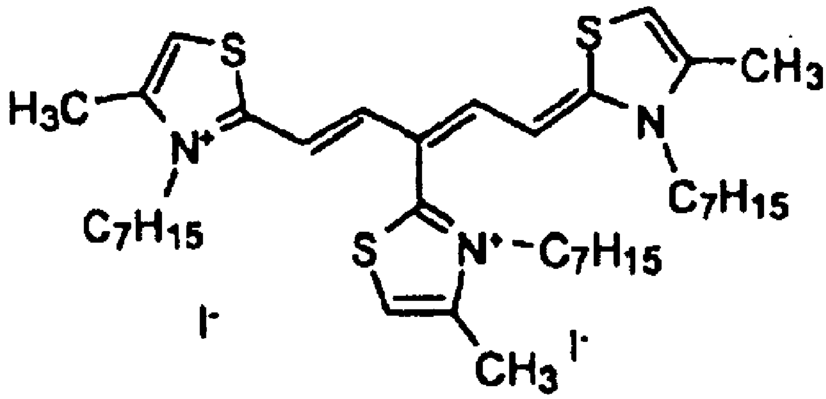

Chemische Formel 5:

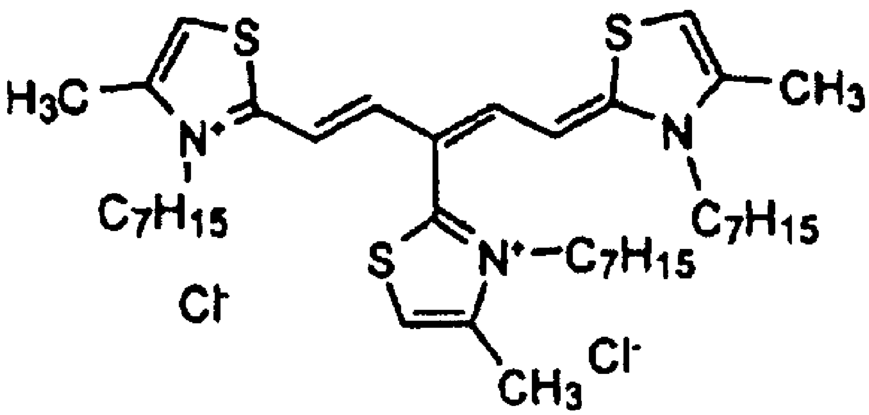

3. Eine Zusammensetzung, die die Anspruch 1 oder 2 entsprechende Verbindung zum Verhindern oder Behandeln einer neuro-degenerativen Erkrankung umfasst, wobei die Verhinderung oder Behandlung der neuro-degenerativen Erkrankung die Förderung von Neurozytenwachstum oder Neuritenauswuchs umfasst und die Zusammensetzung ferner einen pharmazeutisch annehmbaren Bestandteil oder mehrere enthält.

4. Die Anspruch 3 entsprechend zu benutzende Zusammensetzung, wobei es sich bei besagten pharmazeutisch annehmbaren Bestandteilen um wässrige Medien handelt.

5. Die Anspruch 4 entsprechend zu benutzende Zusammensetzung, wobei die Benutzung umfasst, eine Neurozytendegeneration zu hemmen, einen Neurozyten zu schützen oder motorische Degenerationskrankheiten mit einhergehender Neurozytendegeneration zu verbessern.

6. Die Anspruch 5 entsprechend zu benutzende Zusammensetzung, wobei es sich bei dem besagten Neurozyten um Purkinjes Zelle des Zerebellums handelt.

7. Die Anspruch 3 oder 4 entsprechend zu benutzende Zusammensetzung, wobei es sich bei den besagten neurodegenerativen Erkrankungen um Parkinsonkrankheit, Demenz, spinozerebelläre Degeneration, Alzheimer Krankheit, Hirninfarkt oder Ataxie handelt.

## Revendications

1. Un composé représenté par les formules générales 2 ou 3 pour une utilisation dans la prévention ou le traitement d'une maladie neurodégénérative, dans lequel la prévention ou le traitement de la maladie neurodégénérative comprend la promotion de la croissance de neurones ou l'excroissance de neurites :

Formule générale 2 :

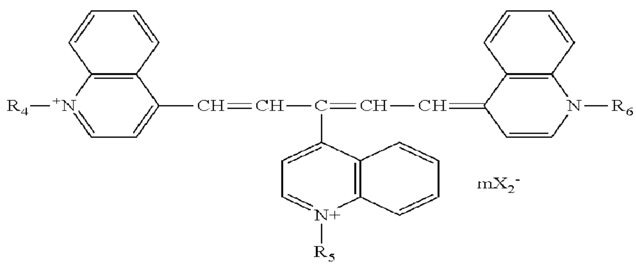

dans lequel dans la formule générale 2, $R_4$ jusqu'à $R_6$ représentent chacun indépendamment le même groupe alkyle ayant un nombre de carbones de 2 à 8 ; $X_2^-$ représente un contre-anion sélectionné du groupe consistant en ion de fluor, ion de chlore, ion de brome, ion d'iode, ion d'acide perchlorique, ion d'acide périodique, ion d'hexafluorophosphorique, ion d'hexafluoroantimonate, ion d'hexafluorostanate, ion d'acide phosphorique, ion de fluoroborique, ion de tétrafluoroborate, ion d'acide thiocyanique, ion d'acide benzène sulfonique, ion d'acide naphtalènesulfonique, ion d'acide naphtalènedisulfonique, ion d'acide p-toluènesulfonique, ion d'acide sulfonique alkyle, ion d'acide benzènecarboxylique, ion d'acide alkylcarboxylique, ion d'acide trihaloalkylcarboxylique, ion d'alkylsulfonate, ion de trihaloalkylsulfonate, ion d'acide nicotinique, et ion d'acide aspartique ; et m représente un entier de valeur 1 ou 2 qui forme une charge électrique pour un équilibre avec la charge électrique d'une partie cationique ;

Formule générale 3 :

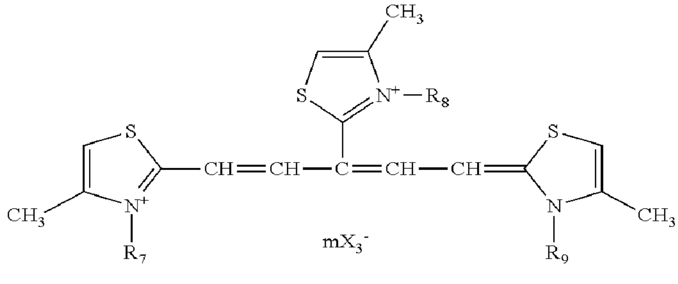

dans lequel dans la formule générale 3, $R_7$ jusqu'à $R_9$ représentent chacun indépendamment le même groupe alkyle ayant un nombre de carbones de 3 à 12 ; $X_3^-$ représente un contre-anion sélectionné du groupe consistant en ion

de fluor, ion de chlore, ion de brome, ion d'iode, ion d'acide perchlorique, ion d'acide périodique, ion d'hexafluorophosphorique, ion d'hexafluoroantimonate, ion d'hexafluorostanate, ion d'acide phosphorique, ion de fluoroborique, ion de tétrafluoroborate, ion d'acide thiocyanique, ion d'acide benzène sulfonique, ion d'acide naphtalènesulfonique, ion d'acide naphtalènedisulfonique, ion d'acide p-toluènesulfonique, ion d'acide sulfonique alkyle, ion d'acide benzènecarboxylique, ion d'acide alkylcarboxylique, ion d'acide trihaloalkylcarboxylique, ion d'alkylsulfonate, ion de trihaloalkylsulfonate, ion d'acide nicotinique, et ion d'acide aspartique ; et m représente un entier de valeur 1 ou 2 qui forme une charge électrique pour un équilibre avec la charge électrique d'une partie cationique.

**2.** Un composé pour une utilisation selon la revendication 1, dans lequel le composé représenté par la formule générale 2 est le composé représenté par les formules chimiques 1 ou 2, dans lequel le composé représenté par la formule générale 3 est le composé représenté par les formules chimiques 3, 4 ou 5.

Formule chimique 1:

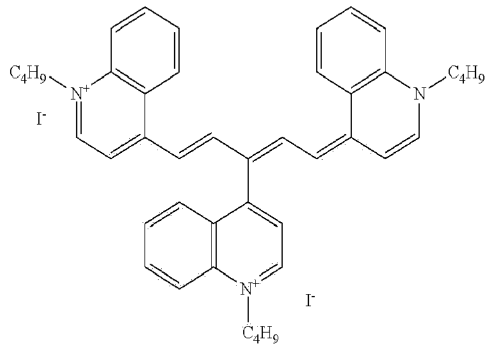

Formule chimique 2 :

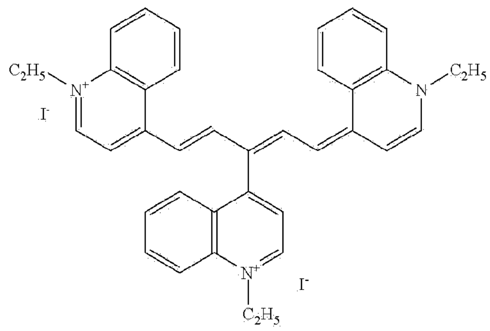

Formule chimique 3:

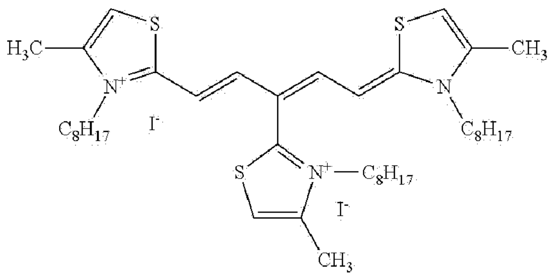

Formule chimique 4 :

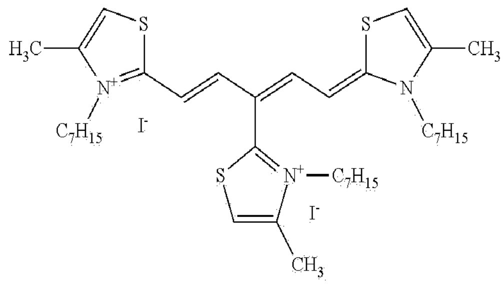

Formule chimique 5 :

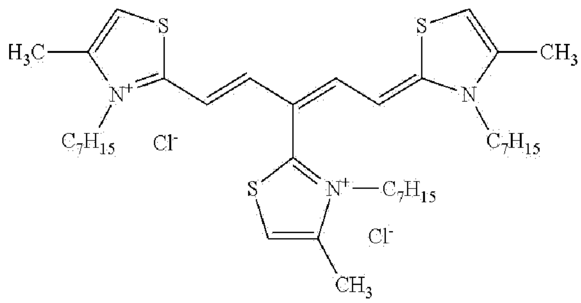

3. Une composition comprenant le composé des revendications 1 ou 2 pour une utilisation dans la prévention ou le traitement d'une maladie neurodégénérative, dans lequel la prévention ou le traitement de la maladie neurodégénérative comprend la promotion de la croissance de neurones ou l'excroissance de neurites, qui contient en outre un ou plusieurs ingrédients acceptables sur le plan pharmaceutique.

4. La composition pour une utilisation selon la revendication 3, dans laquelle lesdits ingrédients acceptables sur le plan pharmaceutique sont des milieux aqueux.

5. La composition pour une utilisation selon la revendication 4, dans laquelle l'utilisation comprend une inhibition d'une dégénérescence des neurones, une protection d'un neurone, ou une amélioration des maladies dégénératives des nerfs moteurs accompagnées de dégénérescence de neurones.

6. La composition pour une utilisation selon la revendication 5, dans laquelle lesdits neurones sont les cellules de Purkinje du cervelet.

7. La composition pour une utilisation selon les revendications 3 ou 4, dans laquelle lesdites maladies neurodégénératives sont la maladie de Parkinson, la démence, la dégénération spinocérébelleuse, la maladie d'Alzheimer, l'infarctus cérébral, ou l'ataxie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 97030703 A
- JP 11228417 A
- JP 2006143708 A
- JP 2006321737 A
- JP 2002234841 A
- JP 5031523 A
- JP 5035128 A
- JP 2906512 B
- JP 2906513 B
- JP 7025765 A
- JP 9052801 A
- WO 0367979 A
- JP 11079991 A
- JP 9052831 A
- JP 2004099560 A
- JP 10279480 A
- JP 2004131402 A
- JP 2006096664 A
- JP 2004123716 A
- JP 2004002381 A
- JP 2004115508 A
- WO 03105909 A
- WO 0413107 A
- JP 2004067585 A
- JP 2004115505 A
- JP 2008509879 A
- WO 0366051 A
- WO 0380583 A
- JP 2006182677 A
- JP 2003335674 A
- JP 2004277315 A
- JP 2005029573 A
- JP 2004137252 A
- JP 2004137253 A
- JP 2004143149 A
- WO 0422543 A
- WO 0512255 A
- WO 02260 A
- JP 2003252760 A
- JP 2004123713 A
- JP 2004137256 A
- JP 2004161720 A
- JP 2004115511 A
- WO 0324446 A
- JP 2004331653 A
- JP 2008037753 A
- JP 2004339214 A
- JP 2005089456 A
- JP 2005162749 A
- WO 0463167 A
- JP 2008247813 A
- JP 2003300880 A
- JP 2008193941 A


### Non-patent literature cited in the description


- Kankoso-Hyo. Kankoshikiso-Kenkyu-Sho, 1969
- *CHEMICAL ABSTRACT Index Guide (N-Z),* 1994, 1531G-1536G
- **KASE H. et al.** *Biochemical and Biophysical Research Communications,* 1987, vol. 144, 35-40
- **VLAHOS C. et al.** *Journal of Biological Chemistry,* 1994, vol. 269, 5241-5248
- **RENAE L. et al.** *Journal of Biological Chemistry,* 1999, vol. 274, 35499
- **WANLI W. et al.** *Journal of Biological Chemistry,* 2002, vol. 277, 17649-17656
- **AKITA K. et al.** *J. Neurogenetics,* 2007, vol. 21, 19-29
- **FERNANDEZ et al.** *Proc. Natl. Acad. Sci. USA,* 1998, vol. 95, 1253-1258
- **RIVLIN et al.** *J. Neurosurg.,* 1997, vol. 47, 577-581
- **HILAL A. LASHUEL et al.** *Journal of Biological Chemistry,* 2002, vol. 277 (45), 42881-42890
- **KOIZUMI et al.** *Cerebral stroke,* 1986, vol. 8 (1), 1-8
- **PETULLO D. et al.** *Life Sciences,* 1999, vol. 64 (13), 1099-1108
- **BENDERSON J. B. et al.** *Stroke,* 1986, vol. 17, 1304-1308
- *Nihon-Yakurigaku-Zasshi,* 2002, vol. 119, 301-308
- *Brain Research,* 1996, vol. 706, 181-193